# EUROPEAN PATENT SPECIFICATION

(11) **EP 4 448 486 B1**
(45) Date of publication and mention of the grant of the patent: **15.10.2025**
(21) Application number: 22847699.0
(22) Date of filing: 15.12.2022
(51) Int. Cl.: A61K 9/1272, A61K 9/51, C07C 233/47, C07C 237/06, C07D 295/13

(54) **FLUORINATED CATIONIC LIPIDS FOR USE IN LIPID NANOPARTICLES**
FLUORIERTE KATIONISCHE LIPIDE ZUR VERWENDUNG IN LIPIDNANOPARTIKELN
LIPIDES CATIONIQUES FLUORÉS DESTINÉS À ÊTRE UTILISÉS DANS DES NANOPARTICULES LIPIDIQUES

(30) Priority: 16.12.2021 US 202163290384 P
(43) Date of publication of application: 23.10.2024
(73) Proprietor: Acuitas Therapeutics, Inc., Vancouver, British Columbia V6T 1W5 (CA)
(72) Inventor: TAN, Jason Samuel, Vancouver, British Columbia V6T 1W5 (CA); ARNS, Steve, Vancouver, British Columbia V6T 1W5 (CA); GATENYO, Julia, Vancouver, British Columbia V6T 1W5 (CA)
(74) Representative: Grünecker Patent- und Rechtsanwälte PartG mbB
(86) International application number: PCT/US2022/081714
(87) International publication number: WO 2023/114944

(56) References cited:
- WO-A1-2013/086373
- WO-A1-2019/036000
- US-A1- 2016 376 224
- US-A1- 2020 093 936

## Description

### BACKGROUND

### Technical Field

The present disclosure generally relates to novel fluorinated cationic lipids that can be used in combination with other lipid components, such as neutral lipids, cholesterol and polymer conjugated lipids, to form lipid nanoparticles to facilitate the intracellular delivery of therapeutic agents, such as nucleic acids (*e*.*g*., oligonucleotides, messenger RNA), both *in vitro* and *in vivo.*

### Description of the Related Art

There are many challenges associated with the delivery of nucleic acids to affect a desired response in a biological system. Nucleic acid based therapeutics have enormous potential but there remains a need for more effective delivery of nucleic acids to appropriate sites within a cell or organism in order to realize this potential. Therapeutic nucleic acids include, *e*.*g*., messenger RNA (mRNA), antisense oligonucleotides, ribozymes, DNAzymes, plasmids, immune stimulating nucleic acids, antagomir, antimir, mimic, supermir, and aptamers. Some nucleic acids, such as mRNA or plasmids, can be used to effect expression of specific cellular products as would be useful in the treatment of, for example, diseases related to a deficiency of a protein or enzyme. The therapeutic applications of translatable nucleotide delivery are extremely broad as constructs can be synthesized to produce any chosen protein sequence, whether or not indigenous to the system. The expression products of the nucleic acid can augment existing levels of protein, replace missing or non-functional versions of a protein, or introduce new protein and associated functionality in a cell or organism.

Some nucleic acids, such as miRNA inhibitors, can be used to effect expression of specific cellular products that are regulated by miRNA as would be useful in the treatment of, for example, diseases related to deficiency of protein or enzyme. The therapeutic applications of miRNA inhibition are extremely broad as constructs can be synthesized to inhibit one or more miRNA that would in turn regulate the expression of mRNA products. The inhibition of endogenous miRNA can augment its downstream target endogenous protein expression and restore proper function in a cell or organism as a means to treat disease associated to a specific miRNA or a group of miRNA.

Other nucleic acids can down-regulate intracellular levels of specific mRNA and, as a result, down-regulate the synthesis of the corresponding proteins through processes such as RNA interference (RNAi) or complementary binding of antisense RNA. The therapeutic applications of antisense oligonucleotide and RNAi are also extremely broad, since oligonucleotide constructs can be synthesized with any nucleotide sequence directed against a target mRNA. Targets may include mRNAs from normal cells, mRNAs associated with disease-states, such as cancer, and mRNAs of infectious agents, such as viruses. To date, antisense oligonucleotide constructs have shown the ability to specifically down-regulate target proteins through degradation of the cognate mRNA in both *in vitro* and *in vivo* models. In addition, antisense oligonucleotide constructs are currently being evaluated in clinical studies.

However, two problems currently face the use of oligonucleotides in therapeutic contexts. First, free RNAs are susceptible to nuclease digestion in plasma. Second, free RNAs have limited ability to gain access to the intracellular compartment where the relevant translation machinery resides. Lipid nanoparticles formed from cationic lipids with other lipid components, such as neutral lipids, cholesterol, PEG, PEGylated lipids, and oligonucleotides have been used to block degradation of the RNAs in plasma and facilitate the cellular uptake of the oligonucleotides. US 2016/376224 discloses examples of such cationic lipids as well as their formulations and use.

There remains a need for improved cationic lipids and lipid nanoparticles for the delivery of oligonucleotides. Preferably, these lipid nanoparticles would provide optimal drug:lipid ratios, protect the nucleic acid from degradation and clearance in serum, be suitable for systemic or local delivery, and provide intracellular delivery of the nucleic acid. In addition, these lipid-nucleic acid particles should be well-tolerated and provide an adequate therapeutic index, such that patient treatment at an effective dose of the nucleic acid is not associated with unacceptable toxicity and/or risk to the patient. The present disclosure provides these and related advantages.

### BRIEF SUMMARY

In brief, the present disclosure provides lipid compounds, including stereoisomers, pharmaceutically acceptable salts or tautomers thereof, which can be used alone or in combination with other lipid components such as neutral lipids, charged lipids, steroids (including for example, all sterols) and/or their analogs, and/or polymer conjugated lipids to form lipid nanoparticles for the delivery of therapeutic agents. In some instances, the lipid nanoparticles are used to deliver nucleic acids such as antisense and/or messenger RNA. Lipid nanoparticles for use in the treatment or prevention (e.g., vaccination) of various diseases or conditions, such as those caused by infectious entities and/or insufficiency of a protein, are also provided.

In one embodiment, compounds having the following Formula (I) are provided: 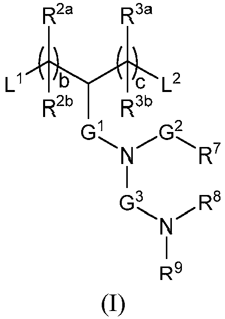 or a pharmaceutically acceptable salt, tautomer or stereoisomer thereof, wherein R^{2a}, R^{2b} , R^{3a} , R^{3b} , R⁷ , R⁸ , R⁹ , L¹, L² , G¹, G² , G³, b, and c are as defined herein.

Pharmaceutical compositions comprising one or more of the foregoing compounds of Formula (I) and a therapeutic agent are also provided. In some embodiments, the pharmaceutical compositions further comprise one or more components selected from neutral lipids, charged lipids, steroids and polymer conjugated lipids. Such compositions are useful for formation of lipid nanoparticles for the delivery of the therapeutic agent.

In other embodiments, the present invention provides a therapeutic agent for use in the treatment of a patient in need thereof, comprising preparing a composition of lipid nanoparticles comprising the compound of Formula (I) and a therapeutic agent and delivering the composition to the patient.

These and other aspects of the invention will be apparent upon reference to the following detailed description.

### DETAILED DESCRIPTION

In the following description, certain specific details are set forth in order to provide a thorough understanding of various embodiments of the disclosure. However, one skilled in the art will understand that the disclosure may be practiced without these details.

The present disclosure is based, in part, upon the discovery of novel cationic (amino) lipids that provide advantages when used in lipid nanoparticles for the *in vivo* delivery of an active or therapeutic agent such as a nucleic acid into a cell of a mammal. In particular, embodiments of the present disclosure provide nucleic acid-lipid nanoparticle compositions comprising one or more of the novel cationic lipids described herein that provide increased activity of the nucleic acid and improved tolerability of the compositions *in vivo,* resulting in a significant increase in the therapeutic index as compared to nucleic acid-lipid nanoparticle compositions previously described. In other embodiments, the disclosed lipids, and lipid nanoparticles comprising the same, have increased safety and/or tolerability when used for delivery of active agents, such as nucleic acids.

In particular embodiments, the present disclosure provides novel cationic lipids that enable the formulation of improved compositions for the *in vitro* and *in vivo* delivery of mRNA and/or other oligonucleotides. In some embodiments, these improved lipid nanoparticle compositions are useful for expression of protein encoded by mRNA. In other embodiments, these improved lipid nanoparticles compositions are useful for upregulation of endogenous protein expression by delivering miRNA inhibitors targeting one specific miRNA or a group of miRNA regulating one target mRNA or several mRNA. In other embodiments, these improved lipid nanoparticle compositions are useful for down-regulating (*e*.*g*., silencing) the protein levels and/or mRNA levels of target genes. In some other embodiments, the lipid nanoparticles are also useful for delivery of mRNA and plasmids for expression of transgenes. In yet other embodiments, the lipid nanoparticle compositions are useful for inducing a pharmacological effect resulting from expression of a protein, *e*.*g*., increased production of red blood cells through the delivery of a suitable erythropoietin mRNA, or protection against infection through delivery of mRNA encoding for a suitable antigen or antibody.

The lipid nanoparticles and compositions of embodiments of the present disclosure may be used for a variety of purposes, including the delivery of encapsulated or associated (*e.g.,* complexed) therapeutic agents such as nucleic acids to cells, both *in vitro* and *in vivo.* Accordingly, embodiments of the present disclosure provides a lipid nanoparticle that encapsulates or is associated with a suitable therapeutic agent, wherein the lipid nanoparticle comprises one or more of the novel cationic lipids described herein.

As described herein, embodiments of the lipid nanoparticles of the present disclosure are particularly useful for the delivery of nucleic acids, including, *e*.*g*., mRNA, antisense oligonucleotide, plasmid DNA, microRNA (miRNA), miRNA inhibitors (antagomirs/antimirs), messenger-RNA-interfering complementary RNA (micRNA), DNA, multivalent RNA, dicer substrate RNA, complementary DNA (cDNA), etc. Therefore, the lipid nanoparticles and compositions of certain embodiments of the present disclosure may be used to induce expression of a desired protein both *in vitro* and *in vivo* by contacting cells with a lipid nanoparticle comprising one or more novel cationic lipids described herein, wherein the lipid nanoparticle encapsulates or is associated with a nucleic acid that is expressed to produce the desired protein *(e.g.,* a messenger RNA or plasmid encoding the desired protein) or inhibit processes that terminate expression of mRNA (e.g., miRNA inhibitors). In certain embodiments, the protein expressed by the nucleic acid is an antigen, and the LNPs thus induce an immune response (e.g., vaccination). Alternatively, the lipid nanoparticles and compositions of embodiments of the present disclosure may be used to decrease the expression of target genes and proteins both *in vitro* and *in vivo* by contacting cells with a lipid nanoparticle comprising one or more novel cationic lipids described herein, wherein the lipid nanoparticle encapsulates or is associated with a nucleic acid that reduces target gene expression (*e*.*g*., an antisense oligonucleotide or small interfering RNA (siRNA)). The lipid nanoparticles and compositions of embodiments of the present disclosure may also be used for co-delivery of different nucleic acids (*e.g.* mRNA and plasmid DNA) separately or in combination, such as may be useful to provide an effect requiring co-localization of different nucleic acids (*e*.*g*. mRNA encoding for a suitable gene modifying enzyme and DNA segment(s) for incorporation into the host genome).

Nucleic acids for use with embodiments of this disclosure may be prepared according to any available technique. For mRNA, the primary methodology of preparation is, but not limited to, enzymatic synthesis (also termed *in vitro* transcription) which currently represents the most efficient method to produce long sequence-specific mRNA. *In vitro* transcription describes a process of template-directed synthesis of RNA molecules from an engineered DNA template comprised of an upstream bacteriophage promoter sequence (*e*.*g*., including but not limited to that from the T7, T3 and SP6 coliphage) linked to a downstream sequence encoding the gene of interest. Template DNA can be prepared for *in vitro* transcription from a number of sources with appropriate techniques which are well known in the art including, but not limited to, plasmid DNA and polymerase chain reaction amplification (see Linpinsel, J.L and Conn, G.L., General protocols for preparation of plasmid DNA template and Bowman, J.C., Azizi, B., Lenz, T.K., Ray, P., and Williams, L.D. in RNA in vitro transcription and RNA purification by denaturing PAGE in Recombinant and in vitro RNA syntheses Methods v. 941 Conn G.L. (ed), New York, N.Y. Humana Press, 2012).

Transcription of the RNA occurs *in vitro* using the linearized DNA template in the presence of the corresponding RNA polymerase and adenosine, guanosine, uridine, and cytidine ribonucleoside triphosphates (rNTPs) under conditions that support polymerase activity while minimizing potential degradation of the resultant mRNA transcripts. *In vitro* transcription can be performed using a variety of commercially available kits including, but not limited to RiboMax Large Scale RNA Production System (Promega), MegaScript Transcription kits (Life Technologies), as well as with commercially available reagents including RNA polymerases and rNTPs. The methodology for *in vitro* transcription of mRNA is well known in the art. (see, *e.g.* Losick, R., 1972, In vitro transcription, Ann Rev Biochem v.41 409-46; Kamakaka, R. T. and Kraus, W. L. 2001. In Vitro Transcription. Current Protocols in Cell Biology. 2:11.6:11.6.1-11.6.17; Beckert, B. And Masquida, B.,(2010) Synthesis of RNA by In Vitro Transcription in RNA in Methods in Molecular Biology v. 703 (Neilson, H. Ed), New York, N.Y. Humana Press, 2010; Brunelle, J.L. and Green, R., 2013, Chapter Five - In vitro transcription from plasmid or PCR-amplified DNA, Methods in Enzymology v. 530, 101-114).

The desired *in vitro* transcribed mRNA is then purified from the undesired components of the transcription or associated reactions (including unincorporated rNTPs, protein enzyme, salts, short RNA oligos, etc.). Techniques for the isolation of the mRNA transcripts are well known in the art. Well known procedures include phenol/chloroform extraction or precipitation with either alcohol (ethanol, isopropanol) in the presence of monovalent cations or lithium chloride. Additional, non-limiting examples of purification procedures which can be used include size exclusion chromatography (Lukavsky, P.J. and Puglisi, J.D., 2004, Large-scale preparation and purification of polyacrylamide-free RNA oligonucleotides, RNA v.10, 889-893), silica-based affinity chromatography and polyacrylamide gel electrophoresis (Bowman, J.C., Azizi, B., Lenz, T.K., Ray, P., and Williams, L.D. in RNA in vitro transcription and RNA purification by denaturing PAGE in Recombinant and in vitro RNA syntheses Methods v. 941 Conn G.L. (ed), New York, N.Y. Humana Press, 2012). Purification can be performed using a variety of commercially available kits including, but not limited to SV Total Isolation System (Promega) and In Vitro Transcription Cleanup and Concentration Kit (Norgen Biotek).

Furthermore, while reverse transcription can yield large quantities of mRNA, the products can contain a number of aberrant RNA impurities associated with undesired polymerase activity which may need to be removed from the full-length mRNA preparation. These include short RNAs that result from abortive transcription initiation as well as double-stranded RNA (dsRNA) generated by RNA-dependent RNA polymerase activity, RNA-primed transcription from RNA templates and self-complementary 3' extension. It has been demonstrated that these contaminants with dsRNA structures can lead to undesired immunostimulatory activity through interaction with various innate immune sensors in eukaryotic cells that function to recognize specific nucleic acid structures and induce potent immune responses. This in turn, can dramatically reduce mRNA translation since protein synthesis is reduced during the innate cellular immune response. Therefore, additional techniques to remove these dsRNA contaminants have been developed and are known in the art including but not limited to scaleable HPLC purification (see, *e*.*g*., Kariko, K., Muramatsu, H., Ludwig, J. And Weissman, D., 2011, Generating the optimal mRNA for therapy: HPLC purification eliminates immune activation and improves translation of nucleoside-modified, protein-encoding mRNA, Nucl Acid Res, v. 39 e142; Weissman, D., Pardi, N., Muramatsu, H., and Kariko, K., HPLC Purification of in vitro transcribed long RNA in Synthetic Messenger RNA and Cell Metabolism Modulation in Methods in Molecular Biology v.969 (Rabinovich, P.H. Ed), 2013). HPLC purified mRNA has been reported to be translated at much greater levels, particularly in primary cells and *in vivo.*

A significant variety of modifications have been described in the art which are used to alter specific properties of *in vitro* transcribed mRNA, and improve its utility. These include, but are not limited to modifications to the 5' and 3' termini of the mRNA. Endogenous eukaryotic mRNA typically contain a cap structure on the 5'-end of a mature molecule which plays an important role in mediating binding of the mRNA Cap Binding Protein (CBP), which is in turn responsible for enhancing mRNA stability in the cell and efficiency of mRNA translation. Therefore, highest levels of protein expression are achieved with capped mRNA transcripts. The 5'-cap contains a 5'-5'-triphosphate linkage between the 5'-most nucleotide and guanine nucleotide. The conjugated guanine nucleotide is methylated at the N7 position. Additional modifications include methylation of the ultimate and penultimate most 5'-nucleotides on the 2'-hydroxyl group.

Multiple distinct cap structures can be used to generate the 5'-cap of *in vitro* transcribed synthetic mRNA. 5'-capping of synthetic mRNA can be performed co-transcriptionally with chemical cap analogs (*i.e.,* capping during *in vitro* transcription). For example, the Anti-Reverse Cap Analog (ARCA) cap contains a 5'-5'-triphosphate guanine-guanine linkage where one guanine contains an N7 methyl group as well as a 3'-O-methyl group. However, up to 20% of transcripts remain uncapped during this co-transcriptional process and the synthetic cap analog is not identical to the 5'-cap structure of an authentic cellular mRNA, potentially reducing translatability and cellular stability. Alternatively, synthetic mRNA molecules may also be enzymatically capped post-transcriptionally. These may generate a more authentic 5'-cap structure that more closely mimics, either structurally or functionally, the endogenous 5'-cap which have enhanced binding of cap binding proteins, increased half-life and reduced susceptibility to 5' endonucleases and/or reduced 5' decapping. Numerous synthetic 5'-cap analogs have been developed and are known in the art to enhance mRNA stability and translatability (see, *e*.*g*., Grudzien-Nogalska, E., Kowalska, J., Su, W., Kuhn, A.N., Slepenkov, S.V., Darynkiewicz, E., Sahin, U., Jemielity, J., and Rhoads, R.E., Synthetic mRNAs with superior translation and stability properties in Synthetic Messenger RNA and Cell Metabolism Modulation in Methods in Molecular Biology v.969 (Rabinovich, P.H. Ed), 2013).

On the 3'-terminus, a long chain of adenine nucleotides (poly-A tail) is normally added to mRNA molecules during RNA processing. Immediately after transcription, the 3' end of the transcript is cleaved to free a 3' hydroxyl to which poly-A polymerase adds a chain of adenine nucleotides to the RNA in a process called polyadenylation. The poly-A tail has been extensively shown to enhance both translational efficiency and stability of mRNA (see Bernstein, P. and Ross, J., 1989, Poly (A), poly (A) binding protein and the regulation of mRNA stability, Trends Bio Sci v. 14 373-377; Guhaniyogi, J. And Brewer, G., 2001, Regulation of mRNA stability in mammalian cells, Gene, v. 265, 11-23; Dreyfus, M. And Regnier, P., 2002, The poly (A) tail of mRNAs: Bodyguard in eukaryotes, scavenger in bacteria, Cell, v.111, 611-613).

Poly (A) tailing of *in vitro* transcribed mRNA can be achieved using various approaches including, but not limited to, cloning of a poly (T) tract into the DNA template or by post-transcriptional addition using Poly (A) polymerase. The first case allows *in vitro* transcription of mRNA with poly (A) tails of defined length, depending on the size of the poly (T) tract, but requires additional manipulation of the template. The latter case involves the enzymatic addition of a poly (A) tail to *in vitro* transcribed mRNA using poly (A) polymerase which catalyzes the incorporation of adenine residues onto the 3'termini of RNA, requiring no additional manipulation of the DNA template, but results in mRNA with poly(A) tails of heterogeneous length. 5'-capping and 3'-poly (A) tailing can be performed using a variety of commercially available kits including, but not limited to Poly (A) Polymerase Tailing kit (EpiCenter), mMESSAGE mMACHINE T7 Ultra kit and Poly (A) Tailing kit (Life Technologies) as well as with commercially available reagents, various ARCA caps, Poly (A) polymerase, etc.

In addition to 5' cap and 3' poly adenylation, other modifications of the *in vitro* transcripts have been reported to provide benefits as related to efficiency of translation and stability. It is well known in the art that pathogenic DNA and RNA can be recognized by a variety of sensors within eukaryotes and trigger potent innate immune responses. The ability to discriminate between pathogenic and self DNA and RNA has been shown to be based, at least in part, on structure and nucleoside modifications since most nucleic acids from natural sources contain modified nucleosides. In contrast, *in vitro* synthesized RNA lacks these modifications, thus rendering it immunostimulatory which in turn can inhibit effective mRNA translation as outlined above. The introduction of modified nucleosides into *in vitro* transcribed mRNA can be used to prevent recognition and activation of RNA sensors, thus mitigating this undesired immunostimulatory activity and enhancing translation capacity (see, *e.g.,* Kariko, K. And Weissman, D. 2007, Naturally occurring nucleoside modifications suppress the immunostimulatory activity of RNA: implication for therapeutic RNA development, Curr Opin Drug Discov Devel, v.10 523-532; Pardi, N., Muramatsu, H., Weissman, D., Kariko, K., In vitro transcription of long RNA containing modified nucleosides in Synthetic Messenger RNA and Cell Metabolism Modulation in Methods in Molecular Biology v.969 (Rabinovich, P.H. Ed), 2013; Kariko, K., Muramatsu, H., Welsh, F.A., Ludwig, J., Kato, H., Akira, S., Weissman, D., 2008, Incorporation of Pseudouridine Into mRNA Yields Superior Nonimmunogenic Vector With Increased Translational Capacity and Biological Stability, Mol Ther v.16, 1833-1840). The modified nucleosides and nucleotides used in the synthesis of modified RNAs can be prepared monitored and utilized using general methods and procedures known in the art. A large variety of nucleoside modifications are available that may be incorporated alone or in combination with other modified nucleosides to some extent into the *in vitro* transcribed mRNA (see, *e.g.,* US 2012/0251618). *In vitro* synthesis of nucleoside-modified mRNA has been reported to have reduced ability to activate immune sensors with a concomitant enhanced translational capacity.

Other components of mRNA which can be modified to provide benefit in terms of translatability and stability include the 5' and 3' untranslated regions (UTR). Optimization of the UTRs (favorable 5' and 3' UTRs can be obtained from cellular or viral RNAs), either both or independently, have been shown to increase mRNA stability and translational efficiency of *in vitro* transcribed mRNA (see, *e.g.,* Pardi, N., Muramatsu, H., Weissman, D., Kariko, K., In vitro transcription of long RNA containing modified nucleosides in Synthetic Messenger RNA and Cell Metabolism Modulation in Methods in Molecular Biology v.969 (Rabinovich, P.H. Ed), 2013).

In addition to mRNA, other nucleic acid payloads may be used for this disclosure. For oligonucleotides, methods of preparation include but are not limited to chemical synthesis and enzymatic, chemical cleavage of a longer precursor, *in vitro* transcription as described above, etc. Methods of synthesizing DNA and RNA nucleotides are widely used and well known in the art (see, *e.g.,* Gait, M. J. (ed.) Oligonucleotide synthesis: a practical approach, Oxford [Oxfordshire], Washington, D.C.: IRL Press, 1984; and Herdewijn, P. (ed.) Oligonucleotide synthesis: methods and applications, Methods in Molecular Biology, v. 288 (Clifton, N.J.) Totowa, N.J.: Humana Press, 2005).

For plasmid DNA, preparation for use with embodiments of this disclosure commonly utilizes, but is not limited to, expansion and isolation of the plasmid DNA *in vitro* in a liquid culture of bacteria containing the plasmid of interest. The presence of a gene in the plasmid of interest that encodes resistance to a particular antibiotic (penicillin, kanamycin, etc.) allows those bacteria containing the plasmid of interest to selectively grow in antibiotic-containing cultures. Methods of isolating plasmid DNA are widely used and well known in the art (see, *e.g.,* Heilig, J., Elbing, K. L. and Brent, R., (2001), Large-Scale Preparation of Plasmid DNA, Current Protocols in Molecular Biology, 41:II:1.7:1.7.1-1.7.16; Rozkov, A., Larsson, B., Gillstrom, S., Björnestedt, R. and Schmidt, S. R., (2008), Large-scale production of endotoxin-free plasmids for transient expression in mammalian cell culture, Biotechnol. Bioeng., 99: 557-566; and US 6,197,553 B1). Plasmid isolation can be performed using a variety of commercially available kits including, but not limited to Plasmid Plus (Qiagen), GenJET plasmid MaxiPrep (Thermo), and PureYield MaxiPrep (Promega) kits as well as with commercially available reagents.

Various exemplary embodiments of the cationic lipids of the present disclosure, lipid nanoparticles and compositions comprising the same, and their use to deliver active (*e*.*g*., therapeutic agents), such as nucleic acids, to modulate gene and protein expression, are described in further detail below.

As used herein, the following terms have the meanings ascribed to them unless specified otherwise.

Unless the context requires otherwise, throughout the present specification and claims, the word "comprise" and variations thereof, such as, "comprises" and "comprising" are to be construed in an open and inclusive sense, that is, as "including, but not limited to".

Reference throughout this specification to "one embodiment" or "an embodiment" means that a particular feature, structure or characteristic described in connection with the embodiment is included in at least one embodiment of the present disclosure. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

Unless defined otherwise, all technical and scientific terms used herein have the same meaning as is commonly understood by one of skill in the art to which this disclosure belongs. As used in the specification and claims, the singular form "a", "an" and "the" include plural references unless the context clearly dictates otherwise.

The phrase "induce expression of a desired protein" refers to the ability of a nucleic acid to increase expression of the desired protein. To examine the extent of protein expression, a test sample *(e.g.,* a sample of cells in culture expressing the desired protein) or a test mammal (*e*.*g*., a mammal such as a human or an animal) model such as a rodent (*e.g.,* mouse) or a non-human primate (*e.g.,* monkey) model is contacted with a nucleic acid (*e.g.,* nucleic acid in combination with a lipid of the present disclosure). Expression of the desired protein in the test sample or test animal is compared to expression of the desired protein in a control sample *(e.g.,* a sample of cells in culture expressing the desired protein) or a control mammal (*e*.*g*., a mammal such as a human or an animal) model such as a rodent (*e*.*g*., mouse) or non-human primate (*e*.*g*., monkey) model that is not contacted with or administered the nucleic acid. When the desired protein is present in a control sample or a control mammal, the expression of a desired protein in a control sample or a control mammal may be assigned a value of 1.0. In particular embodiments, inducing expression of a desired protein is achieved when the ratio of desired protein expression in the test sample or the test mammal to the level of desired protein expression in the control sample or the control mammal is greater than 1, for example, about 1.1, 1.5, 2.0. 5.0 or 10.0. When a desired protein is not present in a control sample or a control mammal, inducing expression of a desired protein is achieved when any measurable level of the desired protein in the test sample or the test mammal is detected. One of ordinary skill in the art will understand appropriate assays to determine the level of protein expression in a sample, for example dot blots, northern blots, in situ hybridization, ELISA, immunoprecipitation, enzyme function, and phenotypic assays, or assays based on reporter proteins that can produce fluorescence or luminescence under appropriate conditions.

The phrase "inhibiting expression of a target gene" refers to the ability of a nucleic acid to silence, reduce, or inhibit the expression of a target gene. To examine the extent of gene silencing, a test sample (*e.g.,* a sample of cells in culture expressing the target gene) or a test mammal (*e*.*g*., a mammal such as a human or an animal) model such as a rodent (*e.g.,* mouse) or a non-human primate (*e.g.,* monkey) model is contacted with a nucleic acid that silences, reduces, or inhibits expression of the target gene. Expression of the target gene in the test sample or test animal is compared to expression of the target gene in a control sample (*e.g.,* a sample of cells in culture expressing the target gene) or a control mammal (*e*.*g*., a mammal such as a human or an animal) model such as a rodent (*e.g.,* mouse) or non-human primate (*e.g.,* monkey) model that is not contacted with or administered the nucleic acid. The expression of the target gene in a control sample or a control mammal may be assigned a value of 100%. In particular embodiments, silencing, inhibition, or reduction of expression of a target gene is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%. In other words, the nucleic acids are capable of silencing, reducing, or inhibiting the expression of a target gene by at least about 5%, 10%, 15%, 20%, 25%, 30%, 35%, 40%, 45%, 50%, 55%, 60%, 65%, 70%, 75%, 80%, 85%, 90%, 95%, or 100% in a test sample or a test mammal relative to the level of target gene expression in a control sample or a control mammal not contacted with or administered the nucleic acid. Suitable assays for determining the level of target gene expression include, without limitation, examination of protein or mRNA levels using techniques known to those of skill in the art, such as, *e.g.,* dot blots, northern blots, *in situ* hybridization, ELISA, immunoprecipitation, enzyme function, as well as phenotypic assays known to those of skill in the art.

An "effective amount" or "therapeutically effective amount" of an active agent or therapeutic agent such as a therapeutic nucleic acid is an amount sufficient to produce the desired effect, *e.g.,* an increase or inhibition of expression of a target sequence in comparison to the normal expression level detected in the absence of the nucleic acid. An increase in expression of a target sequence is achieved when any measurable level is detected in the case of an expression product that is not present in the absence of the nucleic acid. In the case where the expression product is present at some level prior to contact with the nucleic acid, an in increase in expression is achieved when the fold increase in value obtained with a nucleic acid such as mRNA relative to control is about 1.05, 1.1, 1.2, 1.3, 1.4, 1.5, 1.75, 2, 2.5, 3, 4, 5, 6, 7, 8, 9, 10, 15, 20, 25, 30, 40, 50, 75, 100, 250, 500, 750, 1000, 5000, 10000, or greater. Inhibition of expression of a target gene or target sequence is achieved when the value obtained with a nucleic acid such as antisense oligonucleotide relative to the control is about 95%, 90%, 85%, 80%, 75%, 70%, 65%, 60%, 55%, 50%, 45%, 40%, 35%, 30%, 25%, 20%, 15%, 10%, 5%, or 0%. Suitable assays for measuring expression of a target gene or target sequence include, *e*.*g*., examination of protein or RNA levels using techniques known to those of skill in the art such as dot blots, northern blots, *in situ* hybridization, ELISA, immunoprecipitation, enzyme function, fluorescence, or luminescence of suitable reporter proteins, as well as phenotypic assays known to those of skill in the art.

The term "nucleic acid" as used herein refers to a polymer containing at least two deoxyribonucleotides or ribonucleotides in either single- or double-stranded form and includes DNA, RNA, and hybrids thereof. DNA may be in the form of antisense molecules, plasmid DNA, cDNA, PCR products, or vectors. RNA may be in the form of small hairpin RNA (shRNA), messenger RNA (mRNA), antisense RNA, miRNA, micRNA, multivalent RNA, dicer substrate RNA or viral RNA (vRNA), and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (*e*.*g*., degenerate codon substitutions), alleles, orthologs, single nucleotide polymorphisms, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

The term "gene" refers to a nucleic acid (*e.g.,* DNA or RNA) sequence that comprises partial length or entire length coding sequences necessary for the production of a polypeptide or precursor polypeptide.

"Gene product," as used herein, refers to a product of a gene such as an RNA transcript or a polypeptide.

The term "lipid" refers to a group of organic compounds that include, but are not limited to, esters of fatty acids and are generally characterized by being poorly soluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids.

A "steroid" is a compound comprising the following carbon skeleton:

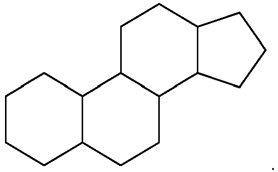

Non-limiting examples of steroids include cholesterol, and the like.

A "cationic lipid" refers to a lipid capable of being positively charged. Exemplary cationic lipids include one or more amine group(s) which bear the positive charge. Preferred cationic lipids are ionizable such that they can exist in a positively charged or neutral form depending on pH. The ionization of the cationic lipid affects the surface charge of the lipid nanoparticle under different pH conditions. This charge state can influence plasma protein absorption, blood clearance, and tissue distribution (Semple, S.C., et al., Adv. Drug Deliv Rev 32:3-17 (1998)) as well as the ability to form endosomolytic non-bilayer structures (Hafez, I.M., et al., Gene Ther 8:1188-1196 (2001)) critical to the intracellular delivery of nucleic acids.

The term "polymer conjugated lipid" refers to a molecule comprising both a lipid portion and a polymer portion. An example of a polymer conjugated lipid is a pegylated lipid. The term "pegylated lipid" refers to a molecule comprising both a lipid portion and a polyethylene glycol portion. Pegylated lipids are known in the art and include 1-(monomethoxy-polyethyleneglycol)-2,3-dimyristoylglycerol (PEG-DMG) and the like.

The term "neutral lipid" refers to any of a number of lipid species that exist either in an uncharged or neutral zwitterionic form at a selected pH. At physiological pH, such lipids include, but are not limited to, phosphotidylcholines such as 1,2-Distearoyl-*sn*-glycero-3-phosphocholine (DSPC), 1,2-Dipalmitoyl-*sn*-glycero-3-phosphocholine (DPPC), 1,2-Dimyristoyl-*sn*-glycero-3-phosphocholine (DMPC), 1-Palmitoyl-2-oleoyl-*sn*-glycero-3-phosphocholine (POPC), 1,2-dioleoyl-sn-glycero-3-phosphocholine (DOPC), phophatidylethanolamines such as 1,2-Dioleoyl-*sn*-glycero-3-phosphoethanolamine (DOPE), sphingomyelins (SM), ceramides, steroids such as sterols and their derivatives. Neutral lipids may be synthetic or naturally derived.

The term "charged lipid" refers to any of a number of lipid species that exist in either a positively charged or negatively charged form independent of the pH within a useful physiological range, *e.g.,* pH ~3 to pH ~9. Charged lipids may be synthetic or naturally derived. Examples of charged lipids include phosphatidylserines, phosphatidic acids, phosphatidylglycerols, phosphatidylinositols, sterol hemisuccinates, dialkyl trimethylammonium-propanes, (*e.g.,* DOTAP, DOTMA), dialkyl dimethylaminopropanes, ethyl phosphocholines, dimethylaminoethane carbamoyl sterols (*e*.*g*., DC-Chol).

The term "lipid nanoparticle" refers to particles having at least one dimension on the order of nanometers (*e*.*g*., 1-1,000 nm) which include one or more of the compounds of structure (I) or other specified cationic lipids. In some embodiments, lipid nanoparticles comprising the disclosed cationic lipids (*e*.*g*., compounds of structure (I)) are included in a formulation that can be used to deliver an active agent or therapeutic agent, such as a nucleic acid (*e.g.,* mRNA) to a target site of interest (*e.g.,* cell, tissue, organ, tumor, and the like). In some embodiments, the lipid nanoparticles comprise a compound of structure (I) and a nucleic acid. Such lipid nanoparticles typically comprise a compound of structure (I) and one or more excipient selected from neutral lipids, charged lipids, steroids, and polymer conjugated lipids. In some embodiments, the active agent or therapeutic agent, such as a nucleic acid, may be encapsulated in the lipid portion of the lipid nanoparticle or an aqueous space enveloped by some or all of the lipid portion of the lipid nanoparticle, thereby protecting it from enzymatic degradation or other undesirable effects induced by the mechanisms of the host organism or cells, *e.g.,* an adverse immune response.

In various embodiments, the lipid nanoparticles have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm. In some embodiments, the lipid nanoparticles are substantially non-toxic. In certain embodiments, nucleic acids, when present in the lipid nanoparticles, are resistant in aqueous solution to degradation with a nuclease. Lipid nanoparticles comprising nucleic acids and their method of preparation are disclosed in, *e.g.,* U.S. Patent Pub. Nos. 2004/0142025, 2007/0042031 and PCT Pub. Nos. WO 2013/016058 and WO 2013/086373.

As used herein, "lipid encapsulated" refers to a lipid nanoparticle that provides an active agent or therapeutic agent, such as a nucleic acid (*e.g.,* mRNA), with full encapsulation, partial encapsulation, or both. In an embodiment, the nucleic acid (*e.g.,* mRNA) is fully encapsulated in the lipid nanoparticle.

As used herein, the term "aqueous solution" refers to a composition comprising water.

"Serum-stable" in relation to nucleic acid-lipid nanoparticles means that the nucleotide is not significantly degraded after exposure to a serum or nuclease assay that would significantly degrade free DNA or RNA. Suitable assays include, for example, a standard serum assay, a DNAse assay, or an RNAse assay.

"Systemic delivery," as used herein, refers to delivery of a therapeutic product that can result in a broad exposure of an active agent within an organism. Some techniques of administration can lead to the systemic delivery of certain agents, but not others. Systemic delivery means that a useful, preferably therapeutic, amount of an agent is exposed to most parts of the body. Systemic delivery of lipid nanoparticles can be by any means known in the art including, for example, intravenous, intraarterial, subcutaneous, and intraperitoneal delivery. In some embodiments, systemic delivery of lipid nanoparticles is by intravenous delivery.

"Local delivery," as used herein, refers to delivery of an active agent directly to a target site within an organism. For example, an agent can be locally delivered by direct injection into a disease site such as a tumor, other target site such as a site of inflammation, or a target organ such as the liver, heart, pancreas, kidney, and the like. Local delivery can also include topical applications or localized injection techniques such as intramuscular, subcutaneous, or intradermal injection. Local delivery does not preclude a systemic pharmacological effect.

"Alkyl" refers to a straight or branched hydrocarbon chain radical consisting solely of carbon and hydrogen atoms, which is saturated, and having, for example, from one to twenty-four carbon atoms (C₁-C₂₄ alkyl), six to twenty-four carbon atoms (C₆-C₂₄ alkyl), four to twenty carbon atoms (C₄-C₂₀ alkyl), six to sixteen carbon atoms (C₆-C₁₆ alkyl), six to nine carbon atoms (C₆-C₉ alkyl), one to fifteen carbon atoms (C₁-C₁₅ alkyl),one to twelve carbon atoms (C₁-C₁₂ alkyl), one to eight carbon atoms (C₁-C₈ alkyl) or one to six carbon atoms (C₁-C₆ alkyl), or any ranges or specific values within the foregoing ranges, and which is attached to the rest of the molecule by a single bond, *e*.*g*., methyl, ethyl, *n*-propyl, 1-methylethyl (iso propyl), *n*-butyl, *n*-pentyl, 1,1-dimethylethyl (*t*-butyl), 3-methylhexyl, 2-methylhexyl, and the like.

"Alkenyl" refers to a straight or branched hydrocarbon chain radical consisting solely of carbon and hydrogen atoms, which is unsaturated (i.e., includes at least one carbon-carbon double bond), and having, for example, from two to twenty-four carbon atoms (C₂-C₂₄ alkenyl), six to twenty-four carbon atoms (C₆-C₂₄ alkenyl), four to twenty carbon atoms (C₄-C₂₀ alkenyl), six to sixteen carbon atoms (C₆-C₁₆ alkenyl), six to nine carbon atoms (C₆-C₉ alkenyl), two to fifteen carbon atoms (C₂-C₁₅ alkenyl), two to twelve carbon atoms (C₂-C₁₂ alkenyl), two to eight carbon atoms (C₂-C₈ alkenyl) or two to six carbon atoms (C₂-C₆ alkenyl), or any ranges or specific values within the foregoing ranges, and which is attached to the rest of the molecule by a single bond, *e*.*g*., ethenyl, *n*-propenyl, 1-methylethenyl, *n*-butenyl, *n*-pentenyl, 1,1-dimethylethenyl, 3-methylhexenyl, 2-methylhexenyl, and the like.

"Fluoroalkyl" refers to an alkyl group in which one or more fluorine atom (F) have been substituted for a hydrogen atom (H). Fluoroalkyl inlcudes straight or branched radicals consisting of either 1) carbon, hydrogen, and fluorine atoms, or 2) carbon and fluorine atoms. Fluoroalkyl can have for example, from one to twenty-four carbon atoms (C₁-C₂₄ fluoroalkyl), six to twenty-four carbon atoms (C₆-C₂₄ fluoroalkyl), four to twenty carbon atoms (C₄-C₂₀ fluoroalkyl), six to sixteen carbon atoms (C₆-C₁₆ fluoroalkyl), six to nine carbon atoms (C₆-C₉ fluoroalkyl), one to fifteen carbon atoms (C₁-C₁₅ fluoroalkyl),one to twelve carbon atoms (C₁-C₁₂ fluoroalkyl), one to eight carbon atoms (C₁-C₈ fluoroalkyl) or one to six carbon atoms (C₁-C₆ fluoroalkyl), or any ranges or specific values within the foregoing ranges, and which is attached to the rest of the molecule by a single bond, *e*.*g*., trifluoromethyl (-CF₃), perfluoroethyl (-CF₂CF₃), perfluoro n-propyl (-(CF₂)₂CF₃), perfluoro iso-propyl (-CF(CF₃)₂), perfluoro n-butyl (-(CF₂)₃CF₃), perfluoro iso-butyl (-CF₂CF(CF₃)₂), perfluoro tert-butyl (-C(CF₃)₃), perfluoro n-hexyl (-(CF₂)₅CF₃), perfluoro n-octyl (-(CF₂)₇CF₃), 2,2,2-trifluoroethyl (-CH₂CF₃), 4,4,4-trifluoro n-butyl (-(CH₂)₃CF₃), 7,7,7-trifluoro n-heptyl (-(CH₂)₆CF₃), or perfluoro n-heptyl (-(CF₂)₆CF₃), and the like. For example, C₁₂ fluoroalkyl includes 1,1,1,2,2,pentafluoro-3-dodecane (-CH(CF₂CF₃)(CH₂)₈CH₃). In another example, C₁₇ fluoroalkyl includes 1,1,1,2,2,3,3,4,4,5,5,6,6,12,12,13,13,14,14,15,15,16,16,17,17,17-hexacosafluoro-9-heptadecane (-CH((CH2)2(CF2)5CF3)2).

"Fluoroalkenyl" refers to an alkenyl group in which one or more fluorine atoms (F) have been substituted for a hydrogen atom (H). Fluoroalkenyl inlcudes straight or branched radicals consisting of either 1) carbon, hydrogen, and fluorine atoms, or 2) carbon and fluorine atoms. Fluoroalkenyl can have for example, from two to twenty-four carbon atoms (C₂-C₂₄ fluoroalkyl), six to twenty-four carbon atoms (C₆-C₂₄ fluoroalkenyl), four to twenty carbon atoms (C₄-C₂₀ fluoroalkenyl), six to sixteen carbon atoms (C₆-C₁₆ fluoroalkenyl), six to nine carbon atoms (C₆-C₉ fluoroalkenyl), two to fifteen carbon atoms (C₂-C₁₅ fluoroalkenyl), two to twelve carbon atoms (C₂-C₁₂ fluoroalkenyl), two to eight carbon atoms (C₂-C₈ fluoroalkenyl) or two to six carbon atoms (C₂-C₆ fluoroalkyl) or any ranges or specific values within the foregoing ranges, and which is attached to the rest of the molecule by a single bond, *e*.*g*., perfluoroethyl (-CF₂CF₃), perfluoro n-propyl (-(CF₂)₂CF₃), perfluoro iso-propyl (-CF(CF₃)₂), perfluoro n-butyl (-(CF₂)₃CF₃), perfluoro iso-butyl (-CF₂CF(CF₃)₂), perfluoro tert-butyl (-C(CF₃)₃), perfluoro n-hexyl (-(CF₂)₅CF₃), perfluoro n-octyl (-(CF₂)₇CF₃), 2,2,2-trifluoroethyl (-CH₂CF₃), 4,4,4-trifluoro n-butyl (-(CH₂)₃CF₃), 7,7,7-trifluoro n-heptyl (-(CH₂)₆CF₃), or perfluoro n-heptyl (-(CF₂)₆CF₃), and the like. For example, C₁₂ fluoroalkyl includes 1,1,1,2,2,pentafluoro-3-dodecane (-CH(CF₂CF₃)(CH₂)₈CH₃). In another example, C₁₇ fluoroalkyl includes 1,1,1,2,2,3,3,4,4,5,5,6,6,12,12,13,13,14,14,15,15,16,16,17,17,17-hexacosafluoro-9-heptadecane (-CH((CH₂)₂(CF₂)₅CF₃)₂).

"Perfluorinated substituent" or "perfluorinated compound" refers to a straight or branched substituent or compound wherein each C-H bond has been replaced with a C-F bond. Perfluorinated substituents or compounds typically contain only carbon-fluorine (C-F) and carbon-carbon bonds (C-C), however, in some embodiments perfluorinated substituent or compound include hetero atoms and/or functional groups such as OH, CO₂H, halides, O, and SO₃H, provided that the perfluorinated substituent or compound contains no C-H bonds and at least one C-F bond. Perfluorinated substituent or compound can be saturated, and having, for example, from one to twenty-four carbon atoms (C₁-C₂₄ perfluoroalkyl), four to twenty carbon atoms (C₄-C₂₀ perfluoroalkyl), six to sixteen carbon atoms (C₆-C₁₆ perfluoroalkyl), six to nine carbon atoms (C₆-C₉ perfluoroalkyl), one to fifteen carbon atoms (C₁-C₁₅ perfluoroalkyl),one to twelve carbon atoms (C₁-C₁₂ perfluoroalkyl), one to eight carbon atoms (C₁-C₈ perfluoroalkyl) or one to six carbon atoms (C₁-C₆ perfluoroalkyl) and which is attached to the rest of the molecule by a single bond, *e*.*g*., trifluoromethyl (-CF₃), perfluoroethyl (-CF₂CF₃), perfluoro n-propyl (-(CF₂)₂CF₃), perfluoro iso-propyl (-CF(CF₃)₂), perfluoro n-butyl (-(CF₂)₃CF₃), perfluoro iso-butyl (-CF₂CF(CF₃)₂), perfluoro tert-butyl (-C(CF₃)₃), perfluoro n-hexyl (-(CF₂)₅CF₃), perfluoro n-octyl (-(CF₂)₇CF₃), perfluoro n-heptyl (-(CF₂)₆CF₃), and the like.

"Alkylene" refers to a straight or branched divalent hydrocarbon chain linking the rest of the molecule to a radical group, consisting solely of carbon and hydrogen, which is saturated, and having, for example, from one to twenty-four carbon atoms (C₁-C₂₄ alkylene), one to fifteen carbon atoms (C₁-C₁₅ alkylene),one to twelve carbon atoms (C₁-C₁₂ alkylene), one to eight carbon atoms (C₁-C₈ alkylene), one to six carbon atoms (C₁-C₆ alkylene), two to four carbon atoms (C₂-C₄ alkylene), one to two carbon atoms (C₁-C₂ alkylene), or any ranges or specific values within the foregoing ranges, *e.g.,* methylene, ethylene, propylene, *n*-butylene, and the like. The alkylene chain is attached to the rest of the molecule through a single bond and to the radical group through a single bond. The points of attachment of the alkylene chain to the rest of the molecule and to the radical group can be through one carbon or any two carbons within the chain.

"Fluoroalkylene" refers to an alkylene as defined above, wherein at least one C-H bond is replaced with a C-F bond. Fluoroalkylenes have, for example, from one to twenty-four carbon atoms (C₁-C₂₄ fluoroalkylene), one to fifteen carbon atoms (C₁-C₁₅ fluoroalkylene),one to twelve carbon atoms (C₁-C₁₂ fluoroalkylene), one to eight carbon atoms (C₁-C₈ fluoroalkylene), one to six carbon atoms (C₁-C₆ fluoroalkylene), two to four carbon atoms (C₂-C₄ fluoroalkylene), one to two carbon atoms (C₁-C₂ fluoroalkylene), or any ranges or specific values within the foregoing ranges, *e.g.,* fluoromethylene, fluoroethylene, fluoropropylene, *n*-fluorobutylene, and the like. The fluoroalkylene chain is attached to the rest of the molecule through a single bond and to the radical group through a single bond. The points of attachment of the fluoroalkylene chain to the rest of the molecule and to the radical group can be through one carbon or any two carbons within the chain.

"Alkylacetal" refers a radical of the formula -R^{a}CH(OR^{b})(OR^{C}), wherein R^{a} is alkylene as defined above, and R^{b} and R^{c} are each independently alkyl or alkenyl as defined above. Alkylacetal groups include, for example, from one to twenty-four carbon atoms (C₁-C₂₄ alkylacetal), six to twenty-four carbon atoms (C₆-C₂₄ alkylacetal), four to twenty carbon atoms (C₄-C₂₀ alkylacetal), six to sixteen carbon atoms (C₆-C₁₆ alkylacetal), six to twenty-four carbon atoms (C₆-C₂₄ alkylacetal), six to nine carbon atoms (C₆-C₉ alkylacetal), one to fifteen carbon atoms (C₁-C₁₅ alkylacetal),one to twelve carbon atoms (C₁-C₁₂ alkylacetal), one to eight carbon atoms (C₁-C₈ alkylacetal) or one to six carbon atoms (C₁-C₆ alkylacetal).

"Fluoroalkylacetal" refers to an an alkylacetal as defined above, wherein at least one C-H bond in R^{a}, R^{b} and/or R^{c} is replaced with a C-F bond.Exemplary fluoroalkylacetals have, for example, from one to twenty-four carbon atoms (C₁-C₂₄ fluoroalkylacetal), six to twenty-four carbon atoms (C₆-C₂₄ alkylacetal), four to twenty carbon atoms (C₄-C₂₀ fluoroalkylacetal), six to sixteen carbon atoms (C₆-C₁₆ fluoroalkylacetal), six to twenty-four carbon atoms (C₆-C₂₄ fluoroalkylacetal), six to nine carbon atoms (C₆-C₉ fluoroalkylacetal), one to fifteen carbon atoms (C₁-C₁₅ fluoroalkylacetal),one to twelve carbon atoms (C₁-C₁₂ fluoroalkylacetal), one to eight carbon atoms (C₁-C₈ alkylacetal) or one to six carbon atoms (C₁-C₆ fluoroalkylacetal).

"Heterocyclic ring" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

The term "substituted" used herein means any of the above groups (*e*.*g*., alkyl, alkenyl, fluoroalkyl, fluoroalkenyl, perfluorinated substituent, perfluorinated compound, alkylene, fluoroalkylene, alkylacetal, fluoroalkylacetal and/or heterocyclic ring) wherein at least one hydrogen atom is replaced by a bond to a non-hydrogen atom such as, but not limited to: a halogen atom such as F, Cl, Br, or I; oxo groups (=O); hydroxyl groups (-OH); carboxyl groups -(CO₂H); C₁-C₁₂ alkyl groups; -(C=O)OR'; -O(C=O)R'; -C(=O)R'; -OR'; -S(O)ₓR'; -S-SR'; -C(=O)SR'; -SC(=O)R' ; -NR'R'; -NR'C(=O)R'; -C(=O)NR'R'; -NR'C(=O)NR'R'; -OC(=O)NR'R'; - NR'C(=O)OR'; -NR'S(O)ₓNR'R'; -NR'S(O)ₓR'; and -S(O)ₓNR'R', wherein: R' is, at each occurrence, independently H or C₁-C₁₅ alkyl and x is 0, 1 or 2. In some embodiments the substituent is a C₁-C₁₂ alkyl group. In other embodiments, the substituent is a halo group, such as fluoro. In other embodiments, the substituent is an oxo group. In other embodiments, the substituent is a hydroxyl group. In other embodiments, the substituent is an alkoxy group (-OR). In other embodiments, the substituent is a carboxyl group. In other embodiments, the substituent is an amine group (-NR'R').

"Optional" or "optionally" (*e*.*g*., optionally substituted) means that the subsequently described event of circumstances may or may not occur, and that the description includes instances where said event or circumstance occurs and instances in which it does not. For example, "optionally substituted alkyl" means that the alkyl radical may or may not be substituted and that the description includes both substituted alkyl radicals and alkyl radicals having no substitution.

The disclosure disclosed herein is also meant to encompass all pharmaceutically acceptable compounds of the compound of structure (I) being isotopically-labelled by having one or more atoms replaced by an atom having a different atomic mass or mass number. Examples of isotopes that can be incorporated into the disclosed compounds include isotopes of hydrogen, carbon, nitrogen, oxygen, phosphorous, fluorine, chlorine , and iodine , such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. These radiolabeled compounds could be useful to help determine or measure the effectiveness of the compounds, by characterizing, for example, the site or mode of action, or binding affinity to pharmacologically important site of action. Certain isotopically-labelled compounds of structure (I), (IA) or (IB), for example, those incorporating a radioactive isotope, are useful in drug and/or substrate tissue distribution studies. The radioactive isotopes tritium, *i.e.,* ³H, and carbon-14, *i.e.,* ¹⁴C, are particularly useful for this purpose in view of their ease of incorporation and ready means of detection.

Substitution with heavier isotopes such as deuterium, *i.e.,* ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of structure (I) can generally be prepared by conventional techniques known to those skilled in the art or by processes analogous to those described in the Preparations and Examples as set out below using an appropriate isotopically-labeled reagent in place of the non-labeled reagent previously employed.

"Stable compound" and "stable structure" are meant to indicate a compound that is sufficiently robust to survive isolation to a useful degree of purity from a reaction mixture, and formulation into an efficacious therapeutic agent.

"Mammal" includes humans and both domestic animals such as laboratory animals and household pets (*e.g.,* cats, dogs, swine, cattle, sheep, goats, horses, rabbits), and non-domestic animals such as wildlife and the like.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" includes both acid and base addition salts.

"Pharmaceutically acceptable acid addition salt" refers to those salts which retain the biological effectiveness and properties of the free bases, which are not biologically or otherwise undesirable, and which are formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, *p*-toluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, *N*-ethylpiperidine, polyamine resins and the like. Particularly preferred organic bases are isopropylamine, diethylamine, ethanolamine, trimethylamine, dicyclohexylamine, choline and caffeine.

Often crystallizations produce a solvate of a compound of the disclosure (*i.e., a* compound of structure (I)). As used herein, the term "solvate" refers to an aggregate that comprises one or more molecules of a compound of the disclosure with one or more molecules of solvent. The solvent may be water, in which case the solvate may be a hydrate. Alternatively, the solvent may be an organic solvent. Thus, the compounds of the present disclosure may exist as a hydrate, including a monohydrate, dihydrate, hemihydrate, sesquihydrate, trihydrate, tetrahydrate and the like, as well as the corresponding solvated forms. Solvates of compound of the disclosure may be true solvates, while in other cases the compound of the disclosure may merely retain adventitious water or be a mixture of water plus some adventitious solvent.

A "pharmaceutical composition" refers to a formulation of a compound of the disclosure and a medium generally accepted in the art for the delivery of the biologically active compound to mammals, *e*.*g*., humans. Such a medium includes all pharmaceutically acceptable carriers, diluents or excipients therefor.

"Effective amount" or "therapeutically effective amount" refers to that amount of a compound of the disclosure which, when administered to a mammal, preferably a human, is sufficient to effect treatment in the mammal, preferably a human. The amount of a lipid nanoparticle of the disclosure which constitutes a "therapeutically effective amount" will vary depending on the compound, the condition and its severity, the manner of administration, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, preferably a human, having the disease or condition of interest, and includes:
(i) preventing the disease or condition from occurring in a mammal, in particular, when such mammal is predisposed to the condition but has not yet been diagnosed as having it;
(ii) inhibiting the disease or condition, *i.e.,* arresting its development;
(iii) relieving the disease or condition, *i.e.,* causing regression of the disease or condition; or
(iv) relieving the symptoms resulting from the disease or condition, *i.e.,* relieving pain without addressing the underlying disease or condition. As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

The compounds of the disclosure, or their pharmaceutically acceptable salts may contain one or more stereocenters and may thus give rise to enantiomers, diastereomers, and other stereoisomeric forms that may be defined, in terms of absolute stereochemistry, as (*R*)- or (*S*)- or, as (D)- or (L)- for amino acids. The present disclosure is meant to include all such possible isomers, as well as their racemic and optically pure forms. Optically active (+) and (-), (*R*)- and (*S*)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centers of geometric asymmetry, and unless specified otherwise, it is intended that the compounds include both E and Z geometric isomers. Likewise, all tautomeric forms are also intended to be included.

A "stereoisomer" refers to a compound made up of the same atoms bonded by the same bonds but having different three-dimensional structures, which are not interchangeable. The present disclosure contemplates various stereoisomers and mixtures thereof and includes "enantiomers", which refers to two stereoisomers whose molecules are non-superimposable mirror images of one another.

A "tautomer" refers to a proton shift from one atom of a molecule to another atom of the same molecule. The present disclosure includes tautomers of any said compounds.

### Compounds

In an aspect, the disclosure provides novel fluorinated lipid compounds which are capable of combining with other lipid components such as neutral lipids, charged lipids, steroids and/or polymer conjugated-lipids to form lipid nanoparticles with therapeutic agents, such as oligonucleotides. Without wishing to be bound by theory, it is thought that these lipid nanoparticles shield the therapeutic agent from degradation in the serum and provide for effective delivery of the therapeutic agent to cells *in vitro* and *in vivo.*

In one embodiment, the compounds have the following structure (I): 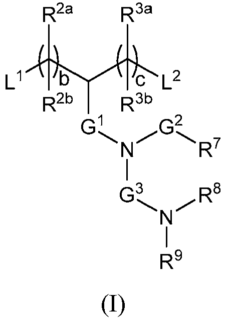 or a pharmaceutically acceptable salt, tautomer, stereoisomer thereof, wherein:
L¹ is -O(C=O)R^{1a}, -(C=O)OR^{1a} , -C(=O)R^{1a} , -OR^{1a} , -S(O)ₓR^{1a} , -S-SR^{1a} , - C(=O)SR^{1a}, -SC(=O)R^{1a}, -NR^{a}C(=O)R^{1a}, -C(=O)NR^{a}R^{1a} , -NR^{a}C(=O)NR^{a}R^{1a,} - OC(⁼O)NR^{a}R^{1a}, -NR^{a}C(=O)OR^{1a} or R^{1b};
L² is -O(C=O)R^{4a} , -(C=O)OR^{4a} , -C(=O)R^{4a} , -OR^{4a} , -S(O)ₓR^{4a} , -S-SR^{4a} , - C(=O)SR^{4a} , -SC(=O)R^{4a} , -NR^{a}C(=O)R^{4a} , -C(=O)NR^{a}R^{4a} , -NR^{a}C(=O)NR^{a}R^{4a}, - OC(=O)NR^{a}R^{4a} , -NR^{a}C(=O)OR^{4a}, or R^{4b};
G¹ is C₁-C₂ alkylene, - (C=O)-, -O(C=O)-, -SC(=O)-, -NR^{a}C(=O)- or a direct bond;
G² is -C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{4a} are each independently branched C₆-C₂₄ alkyl, branched C₆-C₂₄ alkenyl, branched C₆-C₂₄ fluoroalkyl, branched C₆-C₂₄ fluoroalkenyl, C₆-C₂₄ alkylacetal or C₆-C₂₄ fluoroalkylacetal;
R^{1b} and R^{4b} are each independently -CH(OR)(OR), wherein each R is independently linear or branched C₆-C₁₈ alkyl, linear or branched C₆-C₁₈ alkenyl, linear or branched C₆-C₁₈ fluoroalkyl, or linear or branched C₆-C₁₈ fluoroalkenyl
R^{2a} and R^{2b} are, at each occurrence, independently H, F, C₁-C₁₂ alkyl, or C₁-C₁₂ fluoroalkyl;
R^{3a} and R^{3b} are, at each occurrence, independently H, F, C₁-C₁₂ alkyl, or C₁-C₁₂ fluoroalkyl;
R⁷ is H, C₄-C₂₀ alkyl, or C₂-C₁₀ fluoroalkyl;
R⁸ and R⁹ are each independently C₁-C₁₂ alkyl; or R⁸ and R⁹, together with the nitrogen atom to which they are attached, form a 5, 6 or 7-membered heterocyclic ring;
b and c are each independently an integer from 1 to 24; and
wherein at least one of R^{2a}, R^{2b}, R^{3a}, and R^{3b} is F or C₁-C₁₂ fluoroalkyl; at least one of R^{1a} and R^{4a} is present and selected from branched C₆-C₂₄ fluoroalkyl, branched C₆-C₂₄ fluoroalkenyl and C₆-C₂₄ fluoroalkylacetal; at least one of R^{1b} and R^{4b} is present and selected from linear or branched C₆-C₁₈ fluoroalkyl and linear or branched C₆-C₁₈ fluoroalkenyl; G³ is C₁-C₆ fluoroalkylene; and/or R⁷ is C₂-C₁₀ fluoroalkyl.

In another embodiment, the compounds have the following structure (I): 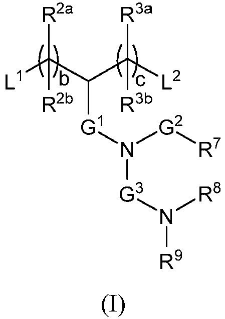 or a pharmaceutically acceptable salt, tautomer, stereoisomer thereof, wherein:
L¹ is -O(C=O)R^{1a}, -(C=O)OR^{1a} , -C(=O)R^{1a} , -OR^{1a} , -S(O)xR^{1a} , -S-SR^{1a} , - C(=O)SR^{1a} , -SC(=O)R^{1a} , -NR^{a}C(=O)R^{1a} , -C(=O)NR^{a}R^{1a} , -NR^{a}C(=O)NR^{a}R^{1a} , - OC(=O)NR^{a}R^{1a}, -NR^{a}C(=O)OR^{1a} or R^{1b};
L² is -O(C=O)R^{4a} , -(C=O)OR^{4a} , -C(=O)R^{4a} , -OR^{4a} , -S(O)ₓR^{4a} , -S-SR^{4a} , - C(=O)SR^{4a} , -SC(=O)R^{4a} , -NR^{a}C(=O)R^{4a} , -C(=O)NR^{a}R^{4a} , -NRaC(=O)NR^{a}R^{4a} , - OC(=O)NR^{a}R^{4a} , ⁻NR^{a}C(=O)OR^{4a}, or R^{4b};
G¹ is C₁-C₂ alkylene, - (C=O)-, -O(C=O)-, -SC(=O)-, -NR^{a}C(=O)- or a direct bond;
G² is -C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{4a} are each independently branched C₆-C₂₄ alkyl, branched C₆-C₂₄ alkenyl, branched C₆-C₂₄ fluoroalkyl, branched C₆-C₂₄ fluoroalkenyl, C₆-C₂₄ alkylacetal or C₆-C₂₄ fluoroalkylacetal;
R^{1b} and R^{4b} are each independently -CH(OR)(OR), wherein each R is independently linear or branched C₆-C₁₈ alkyl, linear or branched C₆-C₁₈ alkenyl, linear or branched C₆-C₁₈ fluoroalkyl, or linear or branched C₆-C₁₈ fluoroalkenyl
R^{2a} and R^{2b} are, at each occurrence, independently H, F, C₁-C₁₂ alkyl, or C₁-C₁₂ fluoroalkyl;
R^{3a} and R^{3b} are, at each occurrence, independently H, F, C₁-C₁₂ alkyl, or C₁-C₁₂ fluoroalkyl;
R⁷ is H, C₄-C₂₀ alkyl, or C₂-C₁₀ fluoroalkyl;
R⁸ and R⁹ are each independently C₁-C₁₂ alkyl; or R⁸ and R⁹, together with the nitrogen atom to which they are attached, form a 5, 6 or 7-membered heterocyclic ring;
b and c are each independently an integer from 1 to 24; and
wherein at least one of R^{2a}, R^{2b}, R^{3a}, and R^{3b} is F or C₁-C₁₂ fluoroalkyl; at least one of R^{1a} and R^{4a} is present and selected from branched C₆-C₂₄ fluoroalkyl, branched C₆-C₂₄ fluoroalkenyl and C₆-C₂₄ fluoroalkylacetal; at least one of R^{1b} and R^{4b} is present and selected from linear or branched C₆-C₁₈ fluoroalkyl and linear or branched C₆-C₁₈ fluoroalkenyl; and/or R⁷ is C₂-C₁₀ fluoroalkyl

In some embodiments, G¹ is independently -(C=O)- or a direct bond. In some embodiments, G² is -(C=O)- or a direct bond. In other embodiments, G¹ and G² are each independently -(C=O)- or a direct bond. As used herein, a "direct bond" means the group (e.g., G¹ or G²) is absent. For example, in some embodiments each of G¹ and G² is a direct bond.

In other of the foregoing embodiments, the compound has one of the following structures (IA) or (IB):

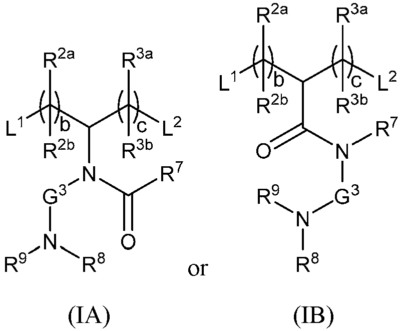

In some embodiments, the compound has structure (IA). In other embodiments, the compound has structure (IB).

In some embodiments, L¹ is -O(C=O)R^{1a} or -(C=O)OR^{1a}. In some embodiments, L² is -O(C=O)R^{4a} or -(C=O)OR^{4a}. For example, in some embodiments L¹ is -O(C=O)R^{1a} and L² is -O(C=O)R^{4a}. In another example, L¹ is -O(C=O)R^{1a} and L² is -(C=O)OR^{4a}. In yet another example, wherein L¹ is -(C=O)OR^{1a} and L² is - O(C=O)R^{4a}. In yet further example, wherein L¹ is -(C=O)OR^{1a} and L² is -(C=O)OR^{4a}.

In some embodiments, at least one of R^{2a}, R^{2b}, R^{3a}, and R^{3b} is F or C₁-C₁₂ fluoroalkyl. For example, in some embodiments R^{2a} and R^{3a} are each F. In another example in some embodiments R^{2b} and R^{3b} are each F. In yet another example, R^{2a} and R^{2b} are each F. In yet another example, R^{3a} and R^{3b} are each F. For example, in some embodiments R^{2a} and R^{3a} are each C₁-C₁₂ fluoroalkyl. In another example in some embodiments R^{2b} and R^{3b} are each C₁-C₁₂ fluoroalkyl. In yet another example, R^{2a} and R^{2b} are each C₁-C₁₂ fluoroalkyl. In yet another example, R^{3a} and R^{3b} are each C₁-C₁₂ fluoroalkyl. In some embodiments, at least one of R^{2a} and R^{3a} is H. For example, in some embodiments R^{2a} and R^{3a} are each H. In some embodiments, at least one of R^{2b} and R^{3b} is H. For example, in some embodiments R^{2b} and R^{3b} are each H.

In some embodiments, at least one of R^{1a} and R^{4a} is present and selected from branched C₆-C₂₄ fluoroalkyl, branched C₆-C₂₄ fluoroalkenyl and C₆-C₂₄ fluoroalkylacetal. In some embodiments, at least one of R^{1b} and R^{4b} is present and selected from linear or branched C₆-C₁₈ fluoroalkyl and linear or branched C₆-C₁₈ fluoroalkenyl.

In some embodiments, at least one of R^{1a}, R^{1b}, R^{4a}, or R^{4b} is 1,1,1,2,2,pentafluoro-3-dodecane (-CH(CF₂CF₃)(CH₂)₈CH₃). For example in some embodiments R^{1a}, R^{4a} or both are each 1,1,1,2,2,pentafluoro-3-dodecane (-CH(CF₂CF₃)(CH₂)₈CH₃). In another example, in some embodiments R^{1b}, R^{4b}, or both are each 1,1,1,2,2,pentafluoro-3-dodecane (-CH(CF₂CF₃)(CH₂)₈CH₃).

In some embodiments, at least one of R^{1a}, R^{1b}, R^{4a}, or R^{4b} is 1,1,1,2,2,3,3,4,4,5,5,6,6,12,12,13,13,14,14,15,15,16,16,17,17,17-hexacosafluoro-9-heptadecane (-CH((CH₂)₂(CF₂)₅CF₃)₂). For example, in some embodiment R^{1a}, R^{4a}, or both are each 1,1,1,2,2,3,3,4,4,5,5,6,6,12,12,13,13,14,14,15,15,16,16,17,17,17-hexacosafluoro-9-heptadecane (-CH((CH₂)₂(CF₂)₅CF₃)₂). In another example, in some embodiments R^{1b}, R^{4b}, or both are each 1,1,1,2,2,3,3,4,4,5,5,6,6,12,12,13,13,14,14,15,15,16,16,17,17,17-hexacosafluoro-9-heptadecane (-CH((CH₂)₂(CF₂)₅CF₃)₂).

In some embodiments, at least one of R^{1a}, R^{1b}, R^{4a}, or R^{4b} has one of the following structures:

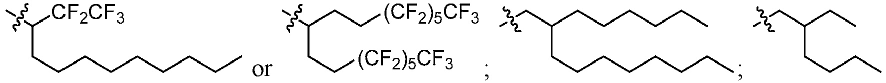

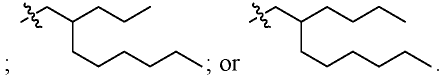

In some embodiments, at least one of R^{1a} and R^{4a} is C₆-C₂₄ alkylacetal or C₆-C₂₄ fluoroalkylacetal. For example, in some embodiments at least one of R^{1a} and R^{4a} has the following structure:

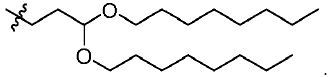

In some embodiments, at least one of L¹ and L² is R^{1b} or R^{4b}, respectively. For example, in some embodiments R^{1b} or R^{4b}, or both, have the following structure:

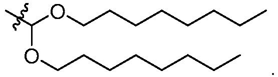

In some embodiments, R⁷ is H, C₆-C₁₆ alkyl, or C₂-C₁₀ fluoroalkyl. In some embodiments, R⁷ is H, C₆-C₉ alkyl, or C₂-C₇ fluoroalkyl. For example, in some embodiments the C₆-C₉ alkyl is n-heptyl (-(CH₂)₆CH₃). In some embodiments, R⁷ is C₂-C₁₀ fluoroalkyl. In other embodiments, R⁷ is C₂-C₁₀ fluoroalkyl. In still more embodiments, R⁷ is perfluoroalkyl, such as C₂-C₇ perfluoroalkyl. In another example, in some embodiments C₂-C₇ fluoroalkyl is 2,2,2-trifluoroethyl (-CH₂CF₃), 4,4,4-trifluoro n-butyl (-(CH₂)₃CF₃), perfluoro n-butyl (-(CF₂)₃CF₃), 7,7,7-trifluoro n-heptyl (-(CH₂)₆CF₃), or perfluoro n-heptyl (-(CF₂)₆CF₃). For example, in some embodiments R⁷ is perfluoro n-heptyl (-(CF₂)₆CF₃). In another example, in some embodiments R⁷ is perfluoro n-butyl (-(CF₂)₃CF₃). In yet further another example, in some embodiments R⁷ is 2,2,2-trifluoroethyl (-CH₂CF₃). In yet further another example, in some embodiments R⁷ is 4,4,4-trifluoro n-butyl (-(CH₂)₃CF₃).

In some embodiments, at least one of R⁸ and R⁹ is methyl (-CH₃). For example, in some embodiments each of R⁸ and R⁹ is methyl (-CH₃). In this regard, the structure (I), (IA), or (IB) having a methyl group on each of R⁸ and R⁹ has a dimethylamine moiety.

In some embodiments, R⁸ and R⁹, together with the nitrogen atom to which they are attached, form a 5, 6 or 7-membered heterocyclic ring. For example in some embodiments the heterocyclic ring is a pyrrolidine. In another example in some embodiments the heterocyclic ring is a piperidine. In yet another example in some embodiments the heterocyclic ring is an azepane. In some embodiments, 5, 6 or 7-membered heterocyclic ring has more than one heteroatoms. For example, in some embodiments the heterocyclic ring is an imidazolidine or pyrazolidine. In another example in some embodiments the heterocyclic ring is a 1,2-diazinane, 1,3-diazinane, or 1,4-diazinane (piperazine). In some embodiments, the heterocyclic ring is substituted. For example, the heterocyclic ring is a 4-methyl piperazine.

In some embodiments, G³ is C₂-C₅ alkylene. For example, in some embodiments G³ is C₂ alkylene including an ethylene. In another example, in some embodiments G³ is C₃ alkylene including an n-propylene. In yet another example, in some embodiments G³ is C₄ alkylene including an n-butylene. In yet another example, in some embodiments G³ is C₅ alkylene including an n-pentylene.

In some other embodiments, G³ is C₁-C₆ fluoroalkylene, for example mono-fluorohexylene.

In some embodiments, b is 5. In some embodiments, b is 8. In some embodiments, c is 5. In some embodiments, c is 8. In some embodiments, b is 8 and c is 8. In some embodiments, b is 5 and c is 5.

In some embodiments, the compound has at least two fluorine atoms. In some embodiments, the compound has at least three fluorine atoms. In some embodiments, the compound has at least one perfluorinated substituent (e.g., trifluoromethyl, trifluoroethyl, trifluoropropyl, trifluorobutyl, trifluoropentyl, trifluorohexyl, or trifluoroheptyl). In some embodiments, the compound is a perfluorinated compound. In some embodiments, the compound has one of the structures set forth in Table 1 below.

**Table 1. Representative Compounds**

| **No.** | **Structure** |
|---|---|
| I-1 | 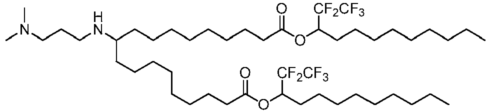 |
| I-2 | 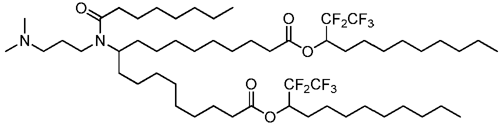 |
| I-3 | 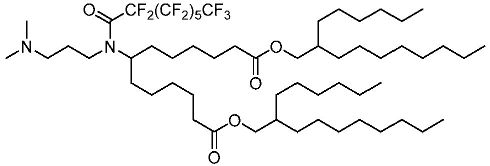 |
| I-4 | 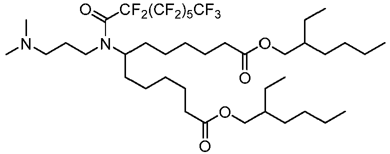 |
| I-5 | 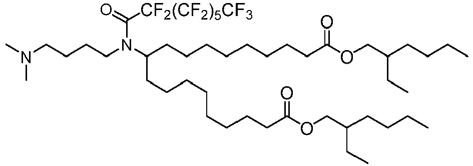 |
| I-6 | 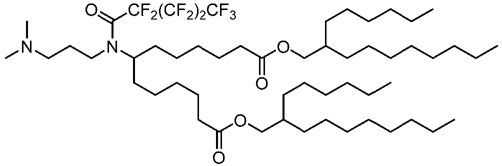 |
| I-7 | 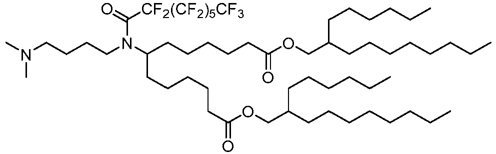 |
| I-8 | 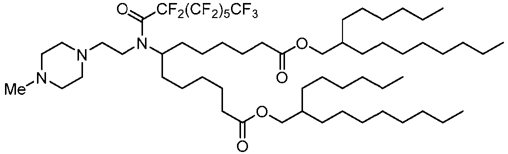 |
| I-9 | 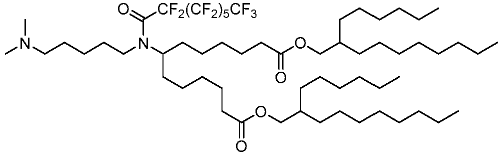 |
| I-10 | 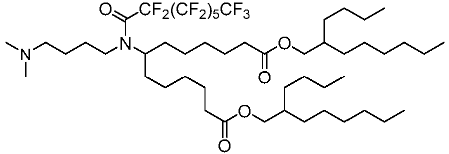 |
| I-11 | 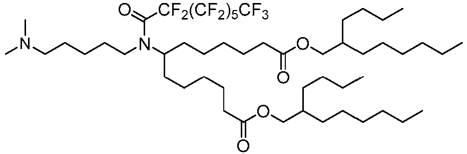 |
| I-12 | 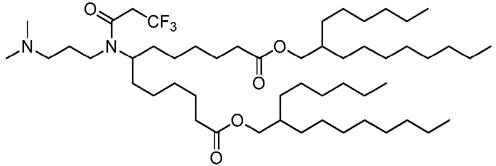 |
| I-13 | 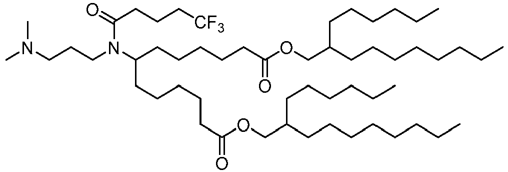 |
| I-14 | 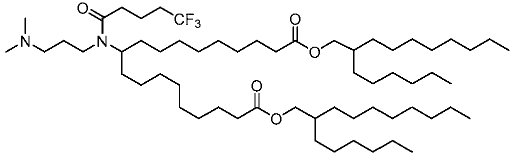 |
| I-15 | 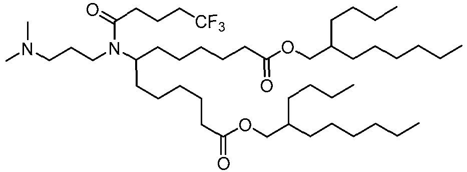 |
| I-16 | 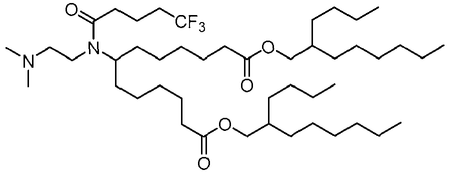 |
| I-17 | 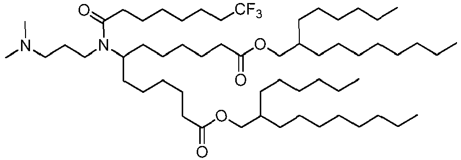 |
| I-18 | 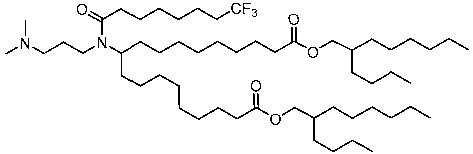 |
| I-19 | 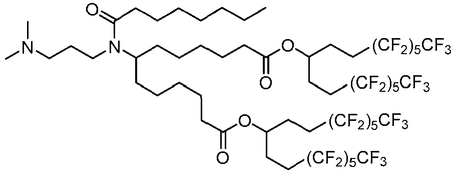 |
| I-20 | 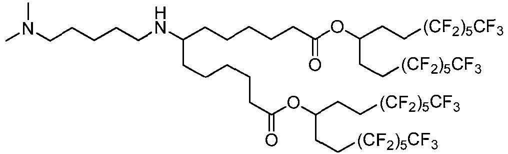 |
| 1-21 | 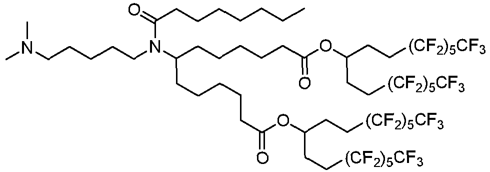 |
| I-22 | 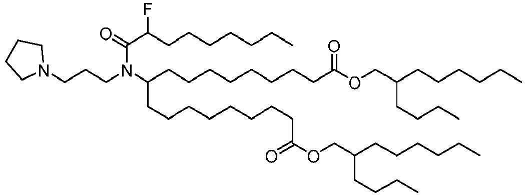 |
| I-23 | 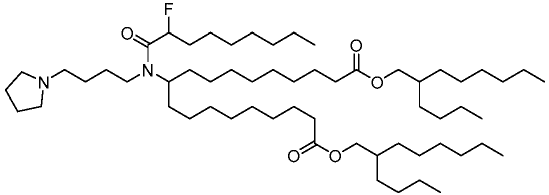 |
| I-24 | 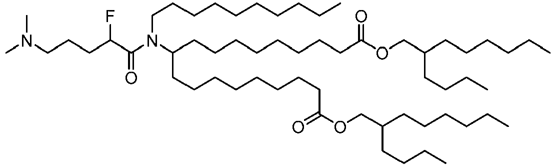 |
| I-25 | 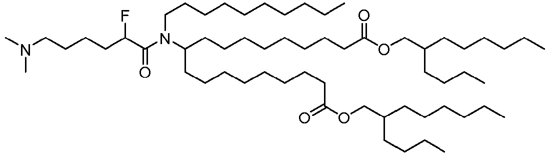 |

It is understood that any embodiment of the compounds of structure (I), as set forth above, and any specific substituent and/or variable in the compound of structure (I), as set forth above, may be independently combined with other embodiments and/or substituents and/or variables of compounds of structure (I) to form embodiments of the disclosures not specifically set forth above. In addition, in the event that a list of substituents and/or variables is listed for any particular R group, G group, L group or variable b and c, , in a particular embodiment and/or claim, it is understood that each individual substituent and/or variable may be deleted from the particular embodiment and/or claim and that the remaining list of substituents and/or variables will be considered to be within the scope of the disclosure.

It is understood that in the present description, combinations of substituents and/or variables of the depicted formulae are permissible only if such contributions result in stable compounds.

In some embodiments, compositions comprising a compound of structure (I) are provided. In some embodiments, the compositions comprise lipid nanoparticles comprising a compound of structure (I) are provided. The lipid nanoparticles optionally include excipients selected from a neutral lipid, a steroid and a polymer conjugated lipid.

In some embodiments, lipid nanoparticles comprising any one or more of the compounds of structure (I) and a therapeutic agent are provided. For example, in some embodiments, the lipid nanoparticles comprise any of the compounds of structure (I) and a therapeutic agent and one or more excipient selected from neutral lipids, steroids and polymer conjugated lipids. Other pharmaceutically acceptable excipients and/or carriers are also included in various embodiments of the lipid nanoparticles.

In some embodiments, the neutral lipid is selected from DSPC, DPPC, DMPC, DOPC, POPC, DOPE and SM. In some embodiments, the neutral lipid is DSPC. In various embodiments, the molar ratio of the compound to the neutral lipid ranges from about 2:1 to about 8:1.

In various embodiments, the lipid nanoparticles further comprise a steroid or steroid analogue. In certain embodiments, the steroid or steroid analogue is cholesterol. In some of these embodiments, the molar ratio of the compound to cholesterol ranges from about 5:1 to 1:1. In some of these embodiments, the molar ratio of the compound to cholesterol ranges from about 2:1 to 1:1.

In various embodiments, the polymer conjugated lipid is a pegylated lipid. For example, some embodiments include a pegylated diacylglycerol (PEG-DAG) such as 1-(monomethoxy-polyethyleneglycol)-2,3-dimyristoylglycerol (PEG-DMG), a pegylated phosphatidylethanoloamine (PEG-PE), a PEG succinate diacylglycerol (PEG-S-DAG) such as 4-O-(2',3'-di(tetradecanoyloxy)propyl-1-O-(ω-methoxy(polyethoxy)ethyl)butanedioate (PEG-S-DMG), a pegylated ceramide (PEG-cer), or a PEG dialkoxypropylcarbamate such as ω-methoxy(polyethoxy)ethyl-N-(2,3- di(tetradecanoxy)propyl)carbamate or 2,3-di(tetradecanoxy)propyl-N-(ω-methoxy(polyethoxy)ethyl)carbamate. In various embodiments, the molar ratio of the compound to the pegylated lipid ranges from about 100:1 to about 25:1. In some embodiments, the molar ratio of the compound to the pegylated lipid ranges from about 100:1 to about 20:1. In some embodiments, the molar ratio of the compound to the pegylated lipid ranges from about 100:1 to about 20:1 or about 100:1 to 10:1 (5% molar ratio of PEG lipid based on a ~50% amino lipid composition).

In some embodiments, the lipid nanoparticles comprises a pegylated lipid having the following structure (II): 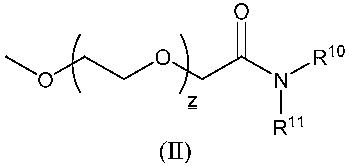 or a pharmaceutically acceptable salt, tautomer or stereoisomer thereof, wherein:
R¹⁰ and R¹¹ are each independently a straight or branched, saturated or unsaturated alkyl chain containing from 10 to 30 carbon atoms, wherein the alkyl chain is optionally interrupted by one or more ester bonds; and
z has a mean value ranging from 30 to 60.

In some embodiments, R¹⁰ and R¹¹ are each independently straight, saturated alkyl chains containing from 12 to 16 carbon atoms. In other embodiments, the average z ranges from about 42 to 55, for example about 49.

In some embodiments of the foregoing lipid nanoparticles, the therapeutic agent comprises a nucleic acid. For example, in some embodiments, the nucleic acid is selected from antisense and messenger RNA.

In other different embodiments, the disclosure is directed to a therapeutic agent for use in a method of treating a patient in need thereof, the method comprising preparing or providing any of the foregoing compositions and administering the composition to the patient

For the purposes of administration, embodiments of the compounds of the present disclosure (typically in the form of lipid nanoparticles in combination with a therapeutic agent) may be administered as a raw chemical or may be formulated as pharmaceutical compositions. Pharmaceutical compositions of embodiments of the present disclosure comprise a compound of structure (I) and one or more pharmaceutically acceptable carrier, diluent or excipient. In some embodiments, the compound of structure (I) is present in the composition in an amount which is effective to form a lipid nanoparticle and deliver the therapeutic agent, *e.g.,* for treating a particular disease or condition of interest. Appropriate concentrations and dosages can be readily determined by one skilled in the art.

Administration of the compositions of embodiments of the disclosure can be carried out via any of the accepted modes of administration of agents for serving similar utilities. The pharmaceutical compositions of embodiments of the disclosure may be formulated into preparations in solid, semi-solid, liquid or gaseous forms, such as tablets, capsules, powders, granules, ointments, solutions, suspensions, suppositories, injections, inhalants, gels, microspheres, and aerosols. Typical routes of administering such pharmaceutical compositions include, without limitation, oral, topical, transdermal, inhalation, parenteral, sublingual, buccal, rectal, vaginal, and intranasal. The term parenteral as used herein includes subcutaneous injections, intravenous, intramuscular, intradermal, intrasternal injection or infusion techniques. Pharmaceutical compositions of embodiments of the disclosure are formulated so as to allow the active ingredients contained therein to be bioavailable upon administration of the composition to a patient. Compositions that will be administered to a subject or patient in some embodiments take the form of one or more dosage units, where for example, a tablet may be a single dosage unit, and a container of a compound of an embodiments of the disclosure in aerosol form may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000). In some embodiments, the composition to be administered will, in any event, contain a therapeutically effective amount of a compound of the disclosure, or a pharmaceutically acceptable salt thereof, for treatment of a disease or condition of interest in accordance with the teachings of this disclosure.

A pharmaceutical composition of embodiments of the disclosure may be in the form of a solid or liquid. In one aspect, the carrier(s) are particulate, so that the compositions are, for example, in tablet or powder form. The carrier(s) may be liquid, with the compositions being, for example, oral syrup, injectable liquid or an aerosol, which is useful in, for example, inhalatory administration.

When intended for oral administration, the pharmaceutical composition of certain embodiments is preferably in either solid or liquid form, where semi-solid, semi-liquid, suspension and gel forms are included within the forms considered herein as either solid or liquid.

As a solid composition for oral administration, the pharmaceutical composition of some embodiments may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavoring agent such as peppermint, methyl salicylate or orange flavoring; and a coloring agent.

When the pharmaceutical composition of some embodiments is in the form of a capsule, for example, a gelatin capsule, it may contain, in addition to materials of the above type, a liquid carrier such as polyethylene glycol or oil.

The pharmaceutical composition of some embodiments may be in the form of a liquid, for example, an elixir, syrup, solution, emulsion or suspension. The liquid may be for oral administration or for delivery by injection, as two examples. When intended for oral administration, preferred composition contain, in addition to a compound of structure (I), one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions of embodiments of the disclosure, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose; agents to act as cryoprotectants such as sucrose or trehalose. The parenteral preparation can be enclosed in ampoules, disposable syringes or multiple dose vials made of glass or plastic. Physiological saline is a preferred adjuvant. An injectable pharmaceutical composition is preferably sterile.

The pharmaceutical composition of embodiments of the disclosure may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device.

The pharmaceutical composition of embodiments of the disclosure may include various materials, which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule.

The pharmaceutical composition of embodiments of the disclosure in solid or liquid form may include an agent that binds to the compound of the disclosure and thereby assists in the delivery of the LNP. Suitable agents that may act in this capacity include a monoclonal or polyclonal antibody, or a protein.

The pharmaceutical composition of embodiments of the disclosure may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of LNPs of embodiments of the disclosure may be delivered in single phase, bi-phasic, or tri-phasic systems in order to deliver the active ingredient(s). Delivery of the aerosol includes the necessary container, activators, valves, sub-containers, and the like, which together may form a kit. One skilled in the art, without undue experimentation, may determine preferred aerosols.

The pharmaceutical compositions of embodiments of the disclosure may be prepared by methodology well known in the pharmaceutical art. For example, a pharmaceutical composition intended to be administered by injection can be prepared by combining the lipid nanoparticles of the disclosure with sterile, distilled water or other carrier so as to form a solution. A surfactant may be added to facilitate the formation of a homogeneous solution or suspension. Surfactants are compounds that non-covalently interact with the compound of the disclosure so as to facilitate dissolution or homogeneous suspension of the compound in the aqueous delivery system.

The compositions of embodiments of the disclosure, or their pharmaceutically acceptable salts, are administered in a therapeutically effective amount, which will vary depending upon a variety of factors including the activity of the specific therapeutic agent employed; the metabolic stability and length of action of the therapeutic agent; the age, body weight, general health, sex, and diet of the patient; the mode and time of administration; the rate of excretion; the drug combination; the severity of the particular disorder or condition; and the subject undergoing therapy.

Compositions of embodiments of the disclosure may also be administered simultaneously with, prior to, or after administration of one or more other therapeutic agents. Such combination therapy includes administration of a single pharmaceutical dosage formulation of a composition of embodiments of the disclosure and one or more additional active agents, as well as administration of the composition of embodiments of the disclosure and each active agent in its own separate pharmaceutical dosage formulation. For example, a composition of embodiments of the disclosure and the other active agent can be administered to the patient together in a single oral dosage composition such as a tablet or capsule, or each agent administered in separate oral dosage formulations. Where separate dosage formulations are used, the compounds of embodiments of the disclosure and one or more additional active agents can be administered at essentially the same time, *i.e.,* concurrently, or at separately staggered times, *i.e.,* sequentially; combination therapy is understood to include all these regimens.

Preparation methods for the above compounds and compositions are described herein below and/or known in the art.

It will be appreciated by those skilled in the art that in the process described herein the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (for example, *t*-butyldimethylsilyl, *t*-butyldiphenylsilyl or trimethylsilyl), tetrahydropyranyl, benzyl, and the like. Suitable protecting groups for amino, amidino and guanidino include *t*-butoxycarbonyl, benzyloxycarbonyl, and the like. Suitable protecting groups for mercapto include -C(O)-R" (where R" is alkyl, aryl or arylalkyl), *p*-methoxybenzyl, trityl and the like. Suitable protecting groups for carboxylic acid include alkyl, aryl or arylalkyl esters. Protecting groups may be added or removed in accordance with standard techniques, which are known to one skilled in the art and as described herein. The use of protecting groups is described in detail in Green, T.W. and P.G.M. Wutz, Protective Groups in Organic Synthesis (1999), 3rd Ed., Wiley. As one of skill in the art would appreciate, the protecting group may also be a polymer resin such as a Wang resin, Rink resin or 2-chlorotrityl-chloride resin.

Furthermore, compounds of embodiments of the disclosure which exist in free base or acid form can be converted to their pharmaceutically acceptable salts by treatment with the appropriate inorganic or organic base or acid by methods known to one skilled in the art. Salts of compounds of embodiments of the disclosure can be converted to their free base or acid form by standard techniques.

The following General Reaction Schemes 1 and 2 illustrate exemplary methods to make compounds of this disclosure, *i.e.,* compounds of structure (I): 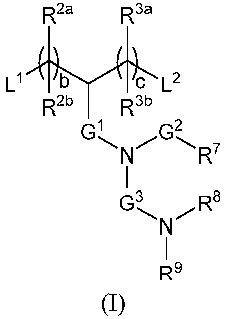 or a pharmaceutically acceptable salt, tautomer, or stereoisomer thereof, wherein b, c, G¹, G², G³ , L¹, L², R^{2a} , R^{2b} , R^{3a} , R^{3b} , R⁷, R⁸, and R⁹ are as defined herein. It is understood that one skilled in the art may be able to make these compounds by similar methods or by combining other methods known to one skilled in the art. It is also understood that one skilled in the art would be able to make, in a similar manner as described below, other compounds of structure (I) not specifically illustrated below by using the appropriate starting components and modifying the parameters of the synthesis as needed. In general, starting components may be obtained from sources such as Sigma Aldrich, Lancaster Synthesis, Inc., Maybridge, Matrix Scientific, TCI, and Fluorochem USA, etc. or synthesized according to sources known to those skilled in the art (see, for example, Advanced Organic Chemistry: Reactions, Mechanisms, and Structure, 5th edition (Wiley, December 2000)) or prepared as described in this disclosure.

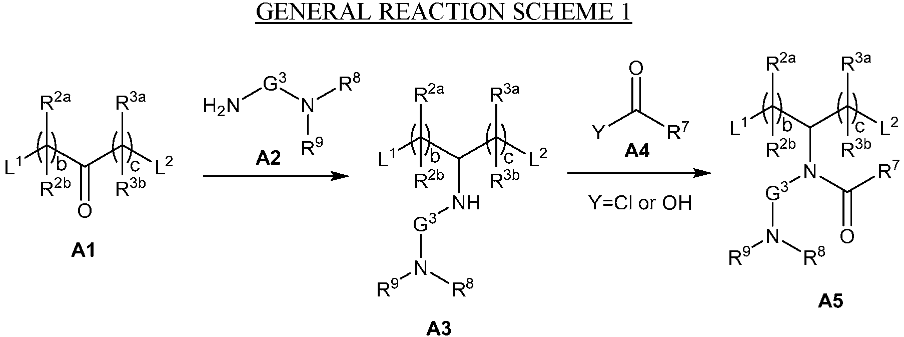

General Reaction Scheme I provides an exemplary method for preparation of a compound of structure (IA) (i.e., A5). b, c, G³, L¹, L², R^{2a}, R^{2b}, R^{3a}, R^{3b}, R⁷, R⁸, and R⁹ in General reaction Scheme 1 are as defined herein. Intermediates and reagents (e.g., A1 and A2) needed for preparation of the compounds according to General Reaction Scheme I can be purchased or prepared according to the examples below or methods known by one of ordinary skill in the art.

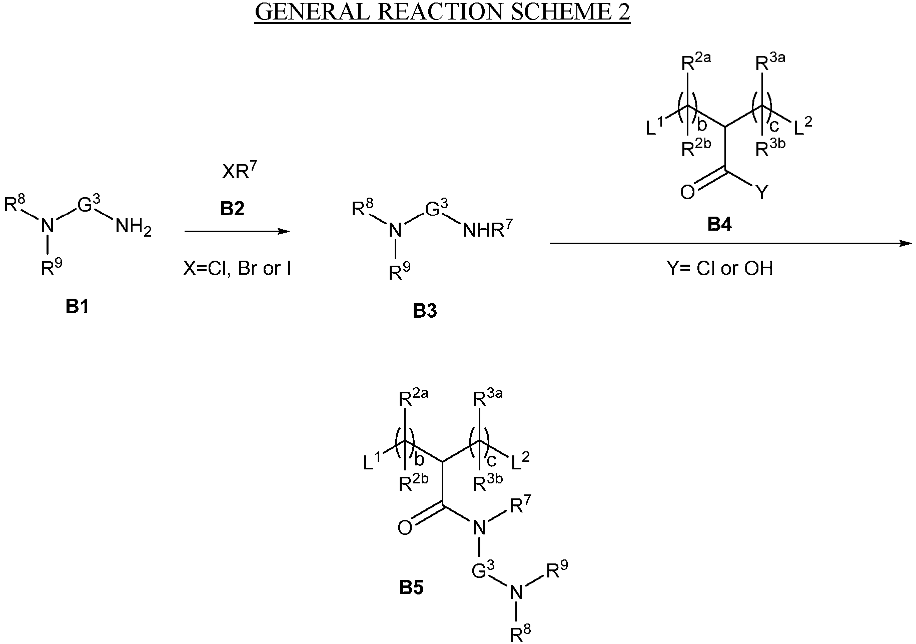

Embodiments of the compound of structure (IB) can be prepared according to General Reaction Scheme 2, wherein R^{2a}, R^{2b} , R^{3a} , R^{3b} , R⁷, R⁸, R⁹ , L¹, L², G³, b, and c are as defined herein. Referring to General Reaction Scheme 2, compounds of structure B1 and B2 can be purchased from commercial sources or prepared according to methods familiar to one of ordinary skill in the art. A mixture of B1 (in excess), B2 and a base (e.g., potassium carbonate) is heated to obtain B3 after any necessary work up. A solution of B3 and a base (e.g. trimethylamine, DMAP) is treated with acyl chloride B4 (or carboxylic acid and DCC) to obtain B5 (structure (IB)) after any necessary work up and/or purification.

It should be noted that various alternative strategies for preparation of compounds of structure (I) are available to those of ordinary skill in the art. For example, other compounds of structure (I) wherein can be prepared according to analogous methods using the appropriate starting material. The use of protecting groups as needed and other modification to the above General Reaction Scheme will be readily apparent to one of ordinary skill in the art.

The following examples are provided for purpose of illustration and not limitation.

### EXAMPLE 1

### LUCIFERASE MRNA IN VIVO EVALUATION USING THE LIPID NANOPARTICLE COMPOSITIONS

Lipid nanoparticles were prepared and tested according to the general procedures described in PCT Pub. Nos. WO 2015/199952 and WO 2017/004143. Briefly, cationic lipid, DSPC, cholesterol and PEG-lipid were solubilized in ethanol at a molar ratio of about 50:10:38.5:1.5 or about 47.5:10:40.7:1.8. Lipid nanoparticles (LNP) were prepared at a total lipid to mRNA weight ratio of approximately 10:1 to 40:1. The mRNA is diluted to 0.2 mg/mL in 10 to 50 mM citrate or acetate buffer, pH 4. Syringe pumps were used to mix the ethanolic lipid solution with the mRNA aqueous solution at a ratio of about 1:5 to 1:3 (vol/vol) with total flow rates above 15 mL/min. The ethanol was then removed and the external buffer replaced with PBS by dialysis. Finally, the lipid nanoparticles were filtered through a 0.2 µm pore sterile filter. Lipid nanoparticle particle size was approximately 55-95 nm diameter, and in some instances approximately 70-90 nm diameter as determined by quasi-elastic light scattering using a Malvern Zetasizer Nano ZS (Malvern, UK).

Studies were performed in 6-8 week old female C57BL/6 mice (Charles River) or 8-10 week old CD-1 (Harlan) mice (Charles River) according to guidelines established by an institutional animal care committee (ACC) and the Canadian Council on Animal Care (CCAC). Varying doses of mRNA-lipid nanoparticle were systemically administered by tail vein injection and animals euthanized at a specific time point (*e.g.,* 4 hours) post-administration. Liver and spleen were collected in preweighed tubes, weights determined, immediately snap frozen in liquid nitrogen and stored at -80°C until processing for analysis.

For liver, approximately 50 mg was dissected for analyses in a 2 mL FastPrep tubes (MP Biomedicals, Solon OH). ¼" ceramic sphere (MP Biomedicals) is added to each tube and 500 µL of Glo Lysis Buffer - GLB (Promega, Madison WI) equilibrated to room temperature is added to liver tissue. Liver tissues were homogenized with the FastPrep24 instrument (MP Biomedicals) at 2 × 6.0 m/s for 15 seconds. Homogenate was incubated at room temperature for 5 minutes prior to a 1:4 dilution in GLB and assessed using SteadyGlo Luciferase assay system (Promega). Specifically, 50 µL of diluted tissue homogenate was reacted with 50 µL of SteadyGlo substrate, shaken for 10 seconds followed by 5 minute incubation and then quantitated using a CentroXS³ LB 960 luminometer (Berthold Technologies, Germany). The amount of protein assayed was determined by using the BCA protein assay kit (Pierce, Rockford IL). Relative luminescence units (RLU) were then normalized to total µg protein assayed. To convert RLU to ng luciferase a standard curve was generated with QuantiLum Recombinant Luciferase (Promega).

The FLuc mRNA (L-6107 or L-7202) from Trilink Biotechnologies will express a luciferase protein, originally isolated from the firefly, *photinus pyralis.* FLuc is commonly used in mammalian cell culture to measure both gene expression and cell viability. It emits bioluminescence in the presence of the substrate, luciferin. This capped and poly-adenylated mRNA was fully substituted with respect to uridine and/or cytidine nucleosides.

### EXAMPLE 2

### DETERMINATION OF PK_{A} OF FORMULATED LIPIDS

As described elsewhere, the pKₐ of formulated cationic lipids is correlated with the effectiveness of LNPs for delivery of nucleic acids (see Jayaraman et al, Angewandte Chemie, International Edition (2012), 51(34), 8529-8533; Semple et al, Nature Biotechnology 28, 172-176 (2010)). The preferred range of pKₐ is ~5 to ~7. The pKₐ of each cationic lipid was determined in lipid nanoparticles using an assay based on fluorescence of 2-(p-toluidino)-6-napthalene sulfonic acid (TNS). Lipid nanoparticles comprising cationic lipid/DSPC/cholesterol/PEG-lipid (47.5/10/40.7/1.8 mol%) in PBS at a concentration of 0.4 mM total lipid were prepared using the in-line process as described in Example 1. TNS was prepared as a 100 µM stock solution in distilled water. Vesicles were diluted to 24 µM lipid in 2 mL of buffered solutions containing, 10 mM HEPES, 10 mM MES, 10 mM ammonium acetate, 130 mM NaCl, where the pH ranged from 2.5 to 11. An aliquot of the TNS solution was added to give a final concentration of 1 µM and following vortex mixing fluorescence intensity was measured at room temperature in a SLM Aminco Series 2 Luminescence Spectrophotometer using excitation and emission wavelengths of 321 nm and 445 nm. A sigmoidal best fit analysis was applied to the fluorescence data and the pKₐ was measured as the pH giving rise to half-maximal fluorescence intensity.

### EXAMPLE 3

### DETERMINATION OF EFFICACY OF LIPID NANOPARTICLE FORMULATIONS CONTAINING VARIOUS CATIONIC LIPIDS USING AN IN VIVO LUCIFERASE MRNA EXPRESSION RODENT MODEL

Representative compounds of the disclosure shown in Table 2 were formulated using the following molar ratio: 50% cationic lipid / 10% distearoylphosphatidylcholine (DSPC) / 38.5% Cholesterol / 1.5% PEG lipid 2-[2-(ω-methoxy(polyethyleneglycol₂₀₀₀)ethoxy]-N,N-ditetradecylacetamide) or 47.5% cationic lipid / 10% DSPC / 40.7% Cholesterol / 1.8% PEG lipid. Relative activity was determined by measuring luciferase expression in the liver 4 hours following administration via tail vein injection as described in Example 1. The activity was compared at a dose of 1.0 or 0.5 or 0.3 mg mRNA/kg and expressed as ng luciferase/g liver measured 4 hours after administration, as described in Example 1. Compound numbers in Table 2 refer to the compound numbers of Table 1. The luciferase assay was also conducted for fluorinated compounds including I-5 and I-11 and the corresponding non-fluorinated analogues as a control. Both cases the assay was performed at 0.3 mg/kg and 1.0 mg/kg as noted in Table 2. The fluorinated compounds I-5 and I-11 showed increased efficacy toward activation of the Luciferase mRNA expression as compared with their non-flourinated analogues (I-5 analogue and I-11 analogue, respectively). In some embodiments, compared to non-fluorinated compounds, the reduced total chain length of the fluorinated compounds show better results, which is not possible without the fluorination.

**Table 2. Lipids and Associated Activity**

| **Cmp. No.** | **pKa** | **Liver Luc @ 0.5 mg/kg (ng luc/g liver)** | **Structure** |
|---|---|---|---|
| I-2 | 6.22 | 1935 ± 416 | 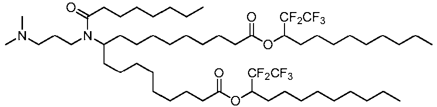 |
| I-5 | 5.92 | 3645 ± 1347* | 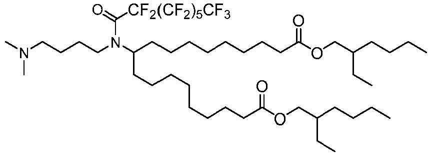 |
| | | * Determined at 0.3 mg/kg | |
| | | 21585 ± 6204** | |
| | | ** Determined at 1.0 mg/kg | |
| I-5 analogue | 6.86 | 666 ± 589* | 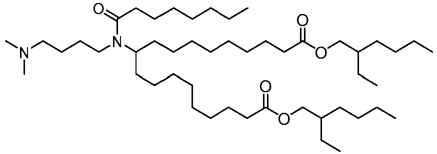 |
| | | * Determined at 0.3 mg/kg | |
| | | 1338 ± 798** | |
| | | ** Determined at 1.0 mg/kg | |
| I-7 | 6.08 | 39 ± 14 | 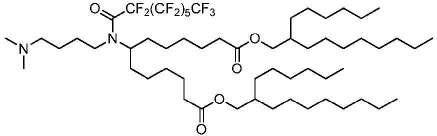 |
| I-8 | 5.58 | 0 | 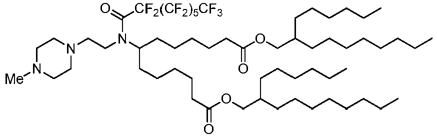 |
| I-9 | 6.32 | 25 ± 16 | 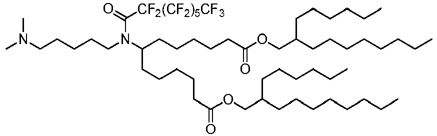 |
| I-10 | 6.18 | 62 ± 44 | 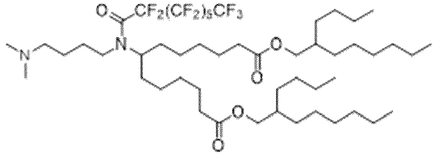 |
| I-11 | 6.28 | 3411 ± 803* | 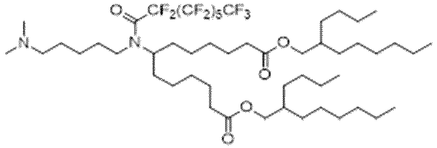 |
| | | * Determined at 0.3 mg/kg | |
| | | 45575 ± 11088** | |
| | | ** Determined at 1.0 mg/kg | |
| I-11 analogue | 7.04 | 1626 ± 472* | 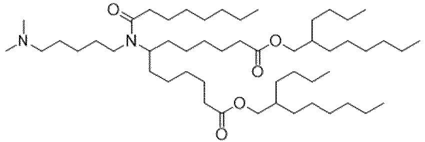 |
| | | * Determined at 0.3 mg/kg | |
| | | 8102 ± 4319** | |
| | | ** Determined at 1.0 mg/kg | |
| I-12 | 6.22 | 138 ± 64* | 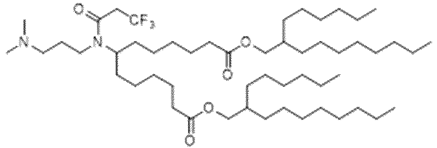 |
| | | * Determined at 0.3 mg/kg | |
| | | 559 ± 207** | |
| | | ** Determined at 1.0 mg/kg | |
| 1-13 | 6.68 | 658 ± 262 | 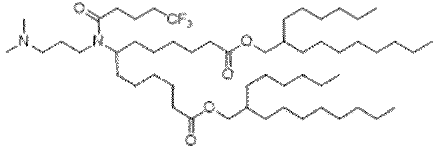 |
| I-14 | 6.28 | 331 ± 127 | 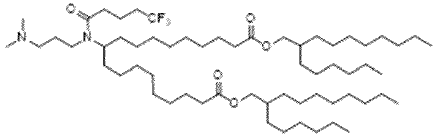 |
| I-15 | 6.78 | 1007 ± 671 | 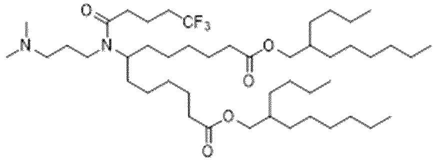 |
| 1-16 | 5.97 | 1233 ± 305 | 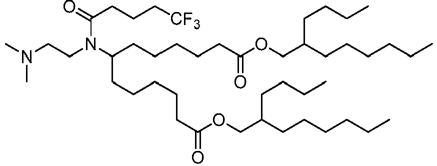 |
| I-22 | 5.96 | 10062 ± 2580* | 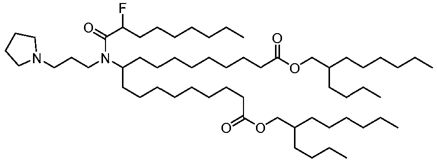 |
| | | * Determined at 0.3 mg/kg | |
| | | 82847 ± 27713** | |
| | | ** Determined at 1.0 mg/kg | |
| 1-24 | 6.04 | 9411 ± 1733* | 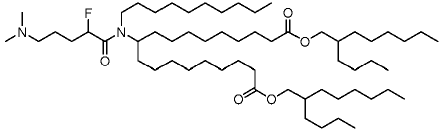 |
| | | * Determined at 0.3 mg/kg | |
| | | 67383 ± 23824** | |
| | | ** Determined at 1.0 mg/kg | |

### EXAMPLE 4

### BIS(1,1,1,2,2-PENTAFLUORODODECAN-3-YL) 10-(N-(3-(DIMETHYLAMINO)PROPYL)OCTANAMIDO)NONADECANEDIOATE (COMPOUND 1-2)

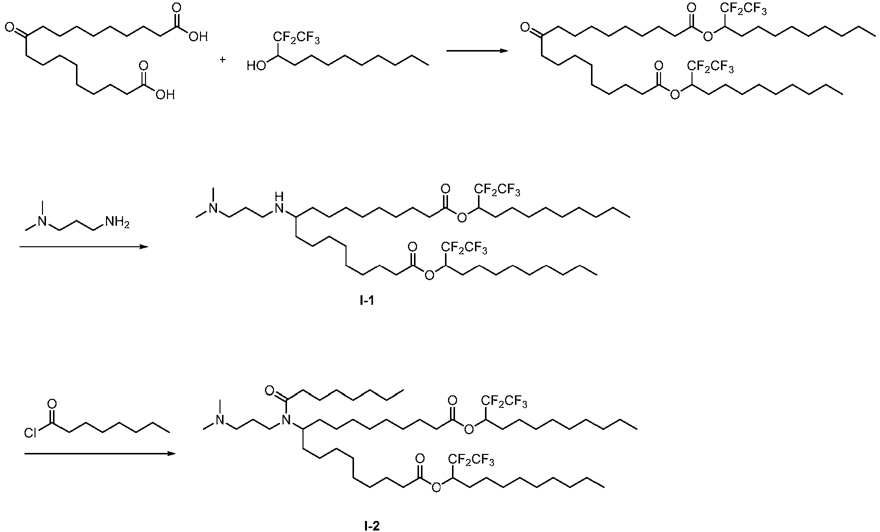

### Synthesis of bis(1,1,1,2,2-pentafluorododecan-3-yl) 10-oxononadecanedioate

To a solution of 1,1,1,2,2-pentafluorododecan-3-ol (0.50 g, 1.80 mmol), 10-oxononadecanedioic acid (0.20 g, 0.58 mmol) and 4-dimethylaminopyridine (DMAP) (0.22 g, 1.8 mmol) in anhydrous DCM was added DCC (0.50 g, 2.34 mmol). The resulting mixture was allowed to stir overnight at room temperature. The solid was then filtered and washed with DCM. The filtrate was concentrated. The residue was purified by column chromatography on silica gel (0-5% ethyl acetate in hexane). The desired product was obtained as a colorless oil (0.61 g, 0.71 mmol, 88%).

### Synthesis of I-1

A solution of 3-(dimethylamino)-1-propylamine (0.10 g, 0.99 mmol) and bis(1,1,1,2,2-pentafluorododecan-3-yl) 10-oxononadecanedioate (0.60 g, 0.70 mmol) in DCE was treated with sodium triacetoxyborohydride (0.21 g, 0.99 mmol) and AcOH (55 µL, 0.98 mmol) overnight. The solution was washed with dilute aqueous sodium hydroxide solution (1 N NaOH). The organic phase was washed with brine, dried over anhydrous sodium sulfate, filtered and the solvent removed. The residue was passed down a small pad of silica gel, washed with a mixture of DCM/MeOH/Et₃N (85:15:1). Purification of the residue by column chromatography on silica gel (0-5% MeOH in DCM gradient) gave compound **I-1** as a colorless oil (170 mg, 0.18 mmol, 26%). ¹HNMR (400 MHz, CDCl₃) δ: 5.51-5.38 (m, 2H), 2.61 (t, 6.8 Hz, 2H), 2.48-2.40 (m, 1H), 2.40-2.35 (t, 7.4 Hz, 4H), 2.34-2.29 (t, 7.4 Hz, 2H), 2.23 (s, 6H), 1.86-1.17 (m, 62H), 0.89 (t, 6.9 Hz, 6H). ESI-MS: MW for C₄₈H₈₆F₁₀N₂O₄ [M+H]⁺ Calc. 945.6; Found 945.8.

### Synthesis of Compound 1-2

A solution of octanoyl chloride (83 mg, 0.51 mmol) in anhydrous benzene (5 mL) was added via syringe to a solution of compound **I-1** (0.24 g, 0.25 mmol), triethylamine (0.3 mL, 2.5 mmol) and DMAP (5 mg) in benzene (10 mL) at RT over the period of 5 min. The mixture was allowed to stir for 2 h and then Methanol (0.5 mL) was added to remove excess acyl chloride. The resulting mixture was stirred for another hour and then filtered through a pad of silica gel, washed with a mixture of hexane/EtOAc/Et₃N (70:30:1) and concentrated. The residue was passed down a silica gel column (0-4% MeOH in DCM gradient), yielding compound **I-2** as a colorless oil (170 mg, 0.16 mmol, 62%). ¹HNMR (400 MHz, CDCl₃) δ: 5.53-5.36 (m, 2H), 3.69-3.57 (m, 1H), 3.18-3.05 (m, 2H), 2.45-2.14 (m, 14H), 1.86-1.12 (m, 72H), 0.92-0.85 (m, 9H). ESI-MS: MW for C₅₆H₁₀₀F₁₀N₂O₅ [M+H]⁺ Calc. 1071.8; Found 1071.9.

### EXAMPLE 5

### BIS(2-HEXYLDECYL) 7-(N-(3-(DIMETHYLAMINO)PROPYL)-3,3,3-TRIFLUOROPROPANAMIDO)TRIDECANEDIOATE (COMPOUND 1-12)

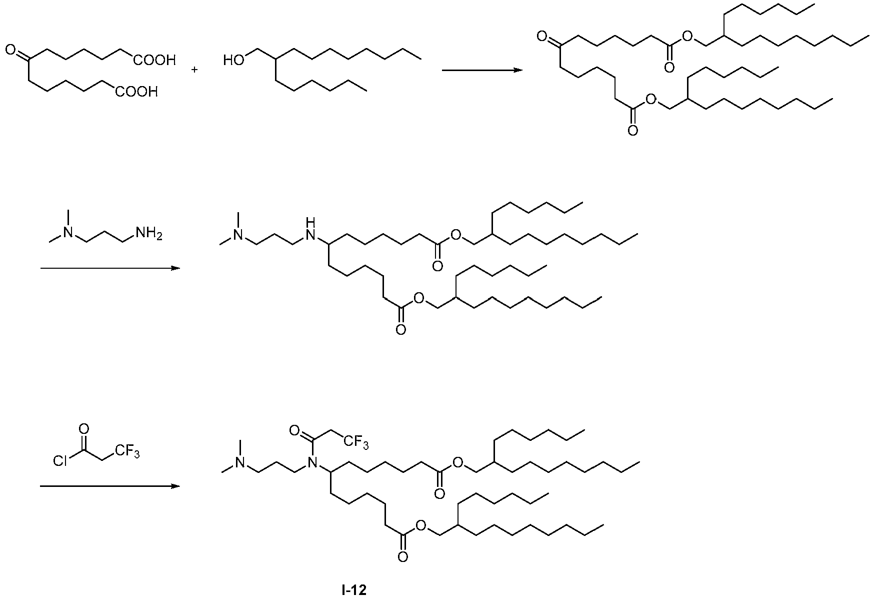

### Synthesis of bis(2-hexyldecyl) 7-oxotridecanedioate

To a solution of 2-hexyl-1-decanol (2.2 mL, 7.75 mmol), 7-oxotridecanedioic acid (0.50 g, 1.94 mmol) and 4-dimethylaminopyridine (DMAP) (0.71 g, 5.81 mmol) in anhydrous DCM was added DCC (1.6 g, 7.75 mmol). The resulting mixture was allowed to stir overnight at room temperature. The solid was then filtered and washed with DCM. The filtrate was concentrated. The residue (oil/solid) was purified by column chromatography on silica gel (0-5% ethyl acetate in hexane). The desired product was obtained as a colorless oil (1.21 g, 1.71 mmol, 88%).

### Synthesis of bis(2-hexyldecyl) 7-((3-(dimethylamino)propyl)amino)tridecanedioate

A solution of 3-(dimethylamino)-1-propylamine (0.10 g, 0.99 mmol) and bis(2-hexyldecyl) 7-oxotridecanedioate (0.50 g, 0.70 mmol) in DCE was treated with sodium triacetoxyborohydride (0.21 g, 0.99 mmol) and AcOH (55 µL, 0.98 mmol) overnight. The solution was washed with dilute aqueous sodium hydroxide solution (1 N NaOH). The organic phase was washed with brine, dried over anhydrous sodium sulfate, filtered and the solvent removed. The residue was passed down a small pad of silica gel, washed with a mixture of DCM/MeOH/Et₃N (85:15:1). The filtrate was concentrated to give the desired product as a slightly yellow oil (360 mg, 0.45 mmol, 65%).

### Synthesis of 1-12

A solution of 3,3,3-trifluoropropionyl chloride (0.13 g, 0.91 mmol) in anhydrous benzene (5 mL) was added to a solution of bis(2-hexyldecyl) 7-((3-(dimethylamino)propyl)amino)tridecanedioate (0.36 g, 0.45 mmol), triethylamine (0.3 mL, 2.5 mmol) and DMAP (5 mg) in benzene (10 mL) at RT over the period of 5 min. The mixture was allowed to stir for 2 h and then Methanol (0.5 mL) was added to remove excess acyl chloride. The resulting mixture was stirred for another hour and then filtered through a pad of silica gel, washed with a mixture of hexane/EtOAc/Et₃N (70:30:1) and concentrated. The residue was passed down a silica gel column (0-4% MeOH in DCM gradient), yielding compound **I-12** as a colorless oil (0.35 g, 0.39 mmol, 87%). ¹HNMR (400 MHz, CDCl₃) δ: 4.38-4.26 (very br., estimated 0.3H, due to slow isomerization about amide bond), 3.99-3.92 (m, 4H), 3.50-3.39 (m, 0.7H), 3.36-3.13 (m, 4H), 2.32-2.23 (m, 6H), 2.22 (s, 6H), 1.63-1.55 (m, 8H), 1.54-1.45 (m, 4H), 1.36-1.18 (m, 56H), 0.91-0.84 (m, 12H). ESI-MS: MW for C₅₃H₁₀₁F₃N₂O₅ [M+H]⁺ Calc. 903.8; Found 904.0.

### EXAMPLE 6

### BIS(2-HEXYLDECYL) 7-(N-(3-(DIMETHYLAMINO)PROPYL)-2,2,3,3,4,4,5,5,6,6,7,7,8,8,8-PENTADECAFLUOROOCTANAMIDO)TRIDECANEDIOATE (COMPOUND 1-3)

Compound I-3 was prepared according to the general procedures of Example 5, to yield 0.26 g of colorless oil, 0.22 mmol, 50%. ¹HNMR (400 MHz, CDCl₃) δ: 3.97, 3.96 (two sets of doublets, 5.8 Hz, 4H), 4.93-4.84 (m, 1H), 3.38-3.19 (m, 2H), 2.35-2.24 (m, 6H), 2.21 (s, 6H), 1.83-1.48 (m, 12H), 1.36-1.21 (m, 56H), 0.89 (t, 6.8 Hz, 12H). ESI-MS: MW for C₅₈H₉₉F₁₅N₂O₅ [M+H]⁺ Calc. 1189.7; Found 1189.8.

### EXAMPLE 7

### BIS(2-ETHYLHEXYL) 7-(N-(3-(DIMETHYLAMINO)PROPYL)-2,2,3,3,4,4,5,5,6,6,7,7,8,8,8-PENTADECAFLUOROOCTANAMIDO)TRIDECANEDIOATE (COMPOUND 1-4)

Compound I-4 was prepared according to the general procedures of Example 5, to yield 0.27 g of colorless oil, 0.28 mmol, 45%. ¹HNMR (400 MHz, CDCl₃) δ: 4.04-3.93 (m, 4H), 3.93-3.83 (m, 1H), 3.38-3.19 (m, 2H), 2.35-2.24 (m, 6H), 2.21 (s, 6H), 1.82-1.45 (m, 14H), 1.42-1.19 (m, 22H), 0.89 (t, 7.3 Hz, 12H). ESI-MS: MW for C₄₂H₆₇F₁₅N₂O₅ [M+H]⁺ Calc. 965.5; Found 965.6.

### EXAMPLE 8

### BIS(2-ETHYLHEXYL) 10-(N-(4-(DIMETHYLAMINO)BUTYL)-2,2,3,3,4,4,5,5,6,6,7,7,8,8,8-PENTADECAFLUOROOCTANAMIDO)NONADECANEDIOATE (COMPOUND 1-5)

Compound I-5 was prepared according to the general procedures of Example 5, to yield 0.40 g of colorless oil, 0.38 mmol, 75%. ¹HNMR (400 MHz, CDCl₃) δ: 4.03-3.93 (m, 4H), 3.92-3.81 (m, 1H), 3.31-3.14 (m, 2H), 2.35-2.24 (m, 6H), 2.22 (s, 6H), 1.74-1.40 (m, 14H), 1.39-1.16 (m, 36H), 0.92-1.84 (m, 12H). ESI-MS: MW for C₄₉H₈₁F₁₅N₂P₅ [M+H]⁺ Calc. 1063.6; Found 1063.7.

### EXAMPLE 9

### BIS(2-HEXYLDECYL) 7-(N-(4-(DIMETHYLAMINO)BUTYL)-2,2,3,3,4,4,5,5,6,6,7,7,8,8,8-PENTADECAFLUOROOCTANAMIDO)TRIDECANEDIOATE (COMPOUND 1-7)

Compound I-7 was prepared according to the general procedures of Example 5, to yield 0.16 g of colorless oil, 0.13 mmol, 28%. ¹HNMR (400 MHz, CDCl₃) δ: 3.97, 3.96 (two sets of doublets, 5.8 Hz, 4H), 3.93-3.83 (m, 1H), 3.32-3.15 (m, 2H), 2.34-2.24 (m, 6H), 2.22 (s, 6H), 1.73-1.39 (m, 14H), 1.38-1.18 (m, 56H), 0.88 (t, 7.0 Hz, 12H). ESI-MS: MW for C₅₉H₁₀₁F₁₅N₂O₅ [M+H]⁺ Calc. 1203.8; Found 1203.8.

### EXAMPLE 10

### BIS(2-HEXYLDECYL) 7-(2,2,3,3,4,4,5,5,6,6,7,7,8,8,8-PENTADECAFLUORO-N-(2-(4-METHYLPIPERAZIN-1-YL)ETHYL)OCTANAMIDO)TRIDECANEDIOATE (COMPOUNDI-8)

Compound I-8 was prepared according to the general procedures of Example 5, to yield 0.35 g of colorless oil, 0.28 mmol, 75%. ¹HNMR (400 MHz, CDCl₃) δ: 4.02-3.92 (m, 4H), 3.92-3.82 (m, 1H), 3.47-3.30 (m, 2H), 2.68-2.35 (m, 10H), 2.34-2.21 (m, 7H), 1.82-1.40 (m, 10H), 1.39-1.16 (m, 56H), 0.88 (t, 6.8 Hz, 12H). ESI-MS: MW for C₆₀H₁₀₂F₁₅N₃O₅ [M+H]⁺ Calc. 1230.8; Found 1230.8.

### EXAMPLE 11

### BIS(2-HEXYLDECYL) 7-(N-(5-(DIMETHYLAMINO)PENTYL)-2,2,3,3,4,4,5,5,6,6,7,7,8,8,8-PENTADECAFLUOROOCTANAMIDO)TRIDECANEDIOATE (COMPOUND 1-9)

Compound I-9 was prepared according to the general procedures of Example 5, to yield 80 mg of colorless oil, 0.07 mmol, 22%. ¹HNMR (400 MHz, CDCl₃) δ: 3.96, 3.95 (two sets of doublets, 5.8 Hz, 4H), 3.92-3.82 (m, 1H), 3.28-3.11 (m, 2H), 2.62-2.21 (m, 12H), 1.73-1.42 (m, 16H), 1.41-1.16 (m, 56H), 0.88 (t, 7.0 Hz, 12H). ESI-MS: MW for C₆₀H₁₀₃F₁₅N₂O₅ [M+H]⁺ Calc. 1217.8; Found 1217.8.

### EXAMPLE 12

### BIS(2-BUTYLOCTYL) 7-(N-(4-(DIMETHYLAMINO)BUTYL)-2,2,3,3,4,4,5,5,6,6,7,7,8,8,8-PENTADECAFLUOROOCTANAMIDO)TRIDECANEDIOATE (COMPOUND I-10)

Compound I-10 was prepared according to the general procedures of Example 5, to yield 80 mg of colorless oil, 0.07 mmol, 22%. ¹HNMR (400 MHz, CDCl₃) δ: 3.98, 3.97 (two sets of doublets, 5.9 Hz, 4H), 3.93-3.83 (m, 1H), 3.32-3.15 (m, 2H), 2.34-2.25 (m, 6H), 2.22 (s, 6H), 1.73-1.40 (m, 14H), 1.39-1.19 (m, 40H), 0.95-0.83 (m, 12H). ESI-MS: MW for C₅₁H₈₅F₁₅N₂O₅ [M+H]⁺ Calc. 1091.6; Found 1091.8.

### EXAMPLE 13

### BIS(2-BUTYLOCTYL) 7-(N-(5-(DIMETHYLAMINO)PENTYL)-2,2,3,3,4,4,5,5,6,6,7,7,8,8,8-PENTADECAFLUOROOCTANAMIDO)TRIDECANEDIOATE (COMPOUND I-11)

Compound I-11 was prepared according to the general procedures of Example 5, to yield 80 mg of colorless oil, 0.07 mmol, 15%. ¹HNMR (400 MHz, CDCl₃) δ: 3.97, 3.96 (two sets of doublets, 5.8 Hz, 4H), 3.91-3.81 (m, 1H), 3.28-3.12 (m, 2H), 2.33-2.17 (m, 12H), 1.76-1.40 (m, 14H), 1.38-1.18 (m, 42H), 0.93-0.82 (m, 12H). ESI-MS: MW for C₅₂H₈₇F₁₅N₂O₅ [M+H]⁺ Calc. 1105.6; Found 1105.8.

### EXAMPLE 14

### BIS(2-HEXYLDECYL) 7-(N-(3-(DIMETHYLAMINO)PROPYL)-5,5,5-TRIFLUOROPENTANAMIDO)TRIDECANEDIOATE (COMPOUND 1-13)

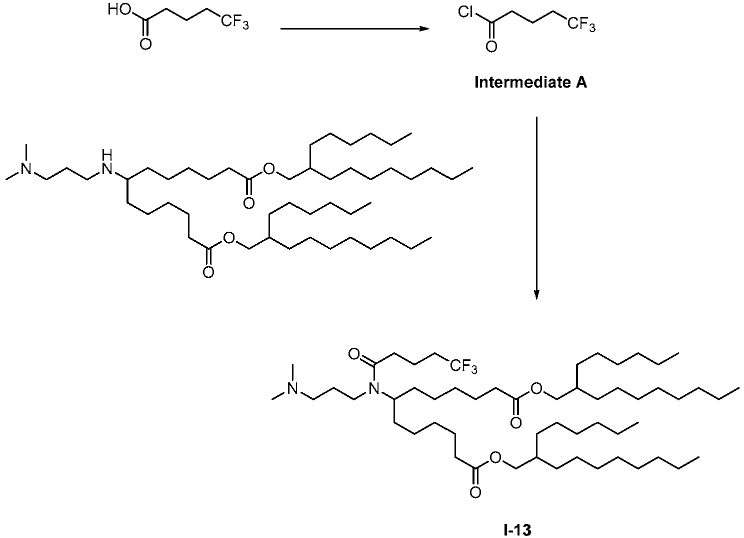

### Synthesis of 5,5,5-trifluoropentanoyl chloride (Intermediate A)

To a solution of 5,5,5-trifluoropentanoic acid (177 mg, 1.13 mmol) in anhydrous DCM (10 mL) and DMF (5-10 µL) was added oxalyl chloride (0.4 mL, 4.54 mmol) at RT. The mixture was allowed to stir over night and then concentrated in the fume hood. The residue was taken up in DCM (10 mL) and concentrated again to remove any oxalyl chloride. The crude product (light yellow oil) was used in the following step without further purification.

### Synthesis of I-13

The residual oil of 5,5,5-trifluoropentanoyl chloride was taken up in 5 mL of anhydrous benzene and added via syringe to a solution of bis(2-hexyldecyl) 7-((3-(dimethylamino)propyl)amino)tridecanedioate (270 mg, 0.34 mmol), triethylamine (0.3 mL, 2.5 mmol) and DMAP (5 mg) in anhydrous benzene (10 mL) at RT over the period of 5 min. After addition, the resulting mixture was stirred at RT for 2 h and then Methanol (0.5 mL) was added to remove excess acyl chloride. The resulting mixture was stirred for another hour, filtered through a pad of silica gel, washed with a mixture of hexane/EtOAc/Et₃N (70:30:1) and concentrated. Purification of the residue by column chromatography on silica gel (0-5% MeOH in DCM gradient) gave compound **I-13** as a colorless oil (135 mg, 0.14 mmol, 43%). ¹HNMR (400 MHz, CDCl₃) δ: 4.54-4.31 (very br., estimated 0.4H, due to slow isomerization about amide bond), 4.02-3.91 (m, 4H), 3.64-3.53 (m, 0.6H), 3.18-3.07 (m, 2H), 2.48-2.10 (m, 16H), 1.94 (sext, 7.2 Hz, 2H), 1.77-1.54 (m, 8H), 1.53-1.40 (m, 4H), 1.39-1.15 (m, 56H), 0.88 (t, 6.8 Hz, 12H). ESI-MS: MW for C₅₅H₁₀₅F₃N₂O₅ [M+H]⁺ Calc. 931.8; Found 932.0.

### EXAMPLE 15

### BIS(2-HEXYLDECYL) 10-(N-(3-(DIMETHYLAMINO)PROPYL)-5,5,5-TRIFLUOROPENTANAMIDO)NONADECANEDIOATE(COMPOUND 1-14)

Compound I-14 was prepared according to the general procedures of Example 14, to yield 0.13 g of colorless oil, 0.13 mmol, 58%. ¹HNMR (400 MHz, CDCl₃) δ: 4.51-4.31 (br., 0.4H, due to slow isomerization about amide bond), 3.99-3.94 (m, 4H), 3.63-3.52 (m, 0.6H), 3.18-3.06 (m, 2H), 2.47-2.09 (m, 16H), 1.94 (sext, 7.2 Hz, 2H), 1.78-1.53 (m, 8H), 1.52-1.38 (m, 4H), 1.36-1.14 (m, 68H), 0.88 (t, 6.9 Hz, 12H). ESI-MS: MW for C₆₁H₁₁₇F₃N₂O₅ [M+H]⁺ Calc. 1015.9; Found 1016.0.

### EXAMPLE 16

### BIS(2-BUTYLOCTYL) 7-(N-(3-(DIMETHYLAMINO)PROPYL)-5,5,5-TRIFLUOROPENTANAMIDO)TRIDECANEDIOATE (COMPOUND I-15)

Compound I-15 was prepared according to the general procedures of Example 14, to yield 0.15 g of colorless oil, 0.18 mmol, 35%. ¹HNMR (400 MHz, CDCl₃) δ: 4.53-4.28 (br., 0.4H, due to slow isomerization about amide bond), 3.96, 3.95 (two sets of doublets, 5.6 Hz, 4H), 3.64-3.51 (m, 0.6H), 3.17-3.05 (m, 2H), 2.45-2.10 (m, 16H), 1.92 (sext, 7.2 Hz, 2H), 1.76-1.52 (m, 8H), 1.52-1.39 (m, 4H), 1.37-1.15 (m, 40H), 0.93-0.83 (m, 12H). ESI-MS: MW for C₄₇H₈₉F₃N₂O₅ [M+H]⁺ Calc. 819.7; Found 819.8.

### EXAMPLE 17

### BIS(2-BUTYLOCTYL) 7-(N-(2-(DIMETHYLAMINO)ETHYL)-5,5,5-TRIFLUOROPENTANAMIDO)TRIDECANEDIOATE (COMPOUND I-16)

Compound I-16 was prepared according to the general procedures of Example 14, to yield 0.23 g of colorless oil, 0.28 mmol, 48%. ¹HNMR (400 MHz, CDCl₃) δ: 4.53-4.33 (br., 0.4H, due to slow isomerization about amide bond), 3.96, 3.95 (two sets of doublets, 5.7 Hz, 4H), 3.64-3.51 (m, 0.6H), 3.27-3.15 (m, 2H), 2.47-2.33 (m, 4H), 2.32-2.23 (m, 10H), 2.22-2.11 (m, 2H), 1.93 (sext, 7.2 Hz, 2H), 1.65-1.54 (m, 6H), 1.51-1.39 (m, 4H), 1.36-1.15 (m, 40H), 0.93-0.83 (m, 12H). ESI-MS: MW for C₄₆H₈₇F₃N₂O₅ [M+H]⁺ Calc. 805.7; Found 805.8.

### EXAMPLE 18

### BIS(1,1,1,2,2,3,3,4,4,5,5,6,6,12,12,13,13,14,14,15,15,16,16,17,17,17-HEXACOSAFLUOROHEPTADECAN-9-YL) 7-(N-(3-(DIMETHYLAMINO)PROPYL)OCTANAMIDO)TRIDECANEDIOATE (COMPOUND I-19)

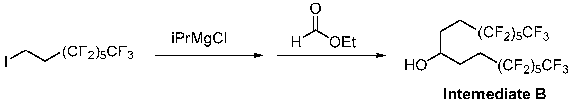

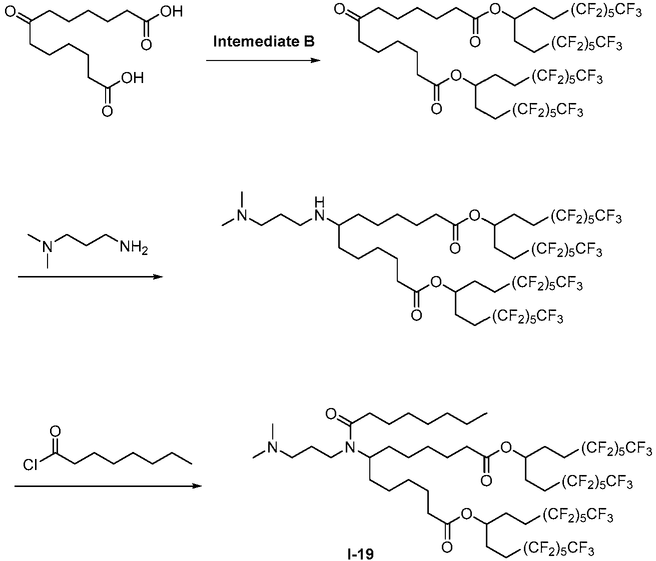

### Synthesis of 1,1,1,2,2,3,3,4,4,5,5,6,6,12,12,13,13,14,14,15,15,16,16,17,17,17-hexacosafluoroheptadecan-9-ol (Intermediate B)

1H,1H,2H,2H-Perfluorooctyl iodide (5 g, 10.55 mmol) was added to an ice-cooled solution of isopropylmagnesium chloride (2.0 M in THF, 4.5ml) and anhydrous THF (10 mL) slowly so that the internal temperature was kept below 15 °C. After 20 min, ethyl formate (0.35 mL, 4.36 mmol) was added over the period of 5 min. The ice-water bath was removed and the reaction mixture stirred for half an hour. The reaction flask was cooled on ice-water again, and cold solution of HCl (1N, 20 mL) was added slowly. It was extracted with Et₂O, the organic layer was washed with aqueous Na₂SO₄, dried over MgSO₄, filtered and evaporated under reduced pressure to obtain white solid. Recrystallization from methylene chloride gave pure 1,1,1,2,2,3,3,4,4,5,5,6,6,12,12,13,13,14,14,15,15,16,16,17,17,17-hexacosafluoroheptadecan-9-ol (3.5 g, 4.83 mmol, 91%).

### Synthesis of bis(1,1,1,2,2,3,3,4,4,5,5,6,6,12,12,13,13,14,14,15,15,16,16,17,17,17-hexacosafluoroheptadecan-9-yl) 7-oxotridecanedioate

To a solution of 1,1,1,2,2,3,3,4,4,5,5,6,6,12,12,13,13,14,14,15,15, 16, 16, 17, 17, 17-hexacosafluoroheptadecan-9-ol (1.9 g, 2.62 mmol), 7-oxotridecanedioic acid (0.17 g, 0.66 mmol) and 4-dimethylaminopyridine (DMAP) (0.25 g, 2.04 mmol) in anhydrous DCM was added DCC (0.56 g, 2.71 mmol). The mixture was allowed to stir overnight at room temperature. The solid was then filtered and washed with EtOAc. The filtrate was concentrated. The residue (oil/solid) was filtered through a pad of silica, washed with a mixture of hexane/EtOAc/Et₃N (70:30:1) and concentrated. The crude product was purified several times by column chromatography on silica gel (0-5% MeOH in DCM gradient) to yield bis(1,1,1,2,2,3,3,4,4,5,5,6,6,12,12,13,13,14,14,15,15,16,16,17,17,17-hexacosafluoroheptadecan-9-yl) 7-oxotridecanedioate as a colorless oil (0.75 g, 0.45 mmol, 68%).

### Synthesis of bis(1,1,1,2,2,3,3,4,4,5,5,6,6,12,12,13,13,14,14,15,15,16,16,17,17,17-hexacosafluoroheptadecan-9-yl) 7-((3-(dimethylamino)propyl)amino)tridecanedioate

A solution of 3-(dimethylamino)-1-propylamine (0.13 g, 1.25 mmol) and bis(1,1,1,2,2,3,3,4,4,5,5,6,6,12,12,13,13,14,14,15,15,16,16,17,17,17-hexacosafluoroheptadecan-9-yl) 7-oxotridecanedioate (1.50 g, 0.89 mmol) in THF was treated with sodium triacetoxyborohydride (0.28 g, 1.32 mmol) and AcOH (55 µL, 0.98 mmol) overnight. The solution was washed with dilute aqueous sodium hydroxide solution (1 N NaOH). The organic phase was washed with brine, dried over anhydrous sodium sulfate, filtered and the solvent removed. The crude product was purified by short column chromatography on silica gel eluted with a mixture of Ether/MeOH/Et₃N (85:15:1). The filtrate was concentrated to give the desired product as a slightly yellow oil (0.5 g, 0.28 mmol, 31%).

### Synthesis of I-19

A solution of octanoyl chloride (93 mg, 0.57 mmol) in anhydrous benzene (5 mL) was added via syringe to a solution of bis(1,1,1,2,2,3,3,4,4,5,5,6,6,12,12,13,13,14,14,15,15,16,16,17,17,17-hexacosafluoroheptadecan-9-yl) 7-((3-(dimethylamino)propyl)amino)tridecanedioate (0.50 g, 0.28 mmol), triethylamine (0.3 mL, 2.5 mmol) and DMAP (5 mg) in benzene (10 mL) at RT over the period of 5 min. The mixture was allowed to stir for 2 h and then Methanol (0.5 mL) was added to remove excess acyl chloride. The resulting mixture was stirred for another hour and then filtered through a pad of silica gel, washed with a mixture of hexane/EtOAc/Et₃N (80:20:2) and concentrated. The residue was passed down a silica gel column (0-4% MeOH in DCM gradient), yielding compound **I-19** as a colorless oil (40 mg, 0.02 mmol, 8%). ¹HNMR (400 MHz, CDCl₃) δ: 5.05-4.95 (m, 2H), 4.61-4.34 (very br., estimated 0.4H, due to slow isomerization about amide bond), 3.68-3.57 (m, 0.6H), 3.15-3.03 (m, 2H), 2.36-2.01 (m, 22H), 1.95-1.82 (m, 8H), 1.73-1.53 (m, 8H) 1.50-1.37 (m, 4H), 1.35-1.17 (m, 16H), 0.91-0.82 (m, 3H).

### EXAMPLE 19

### BIS(2-BUTYLOCTYL) 10-(2-FLUORO-N-(3-(PYRROLIDIN-1-YL)PROPYL)NONANAMIDO)NONADECANEDIOATE (COMPOUND 1-22)

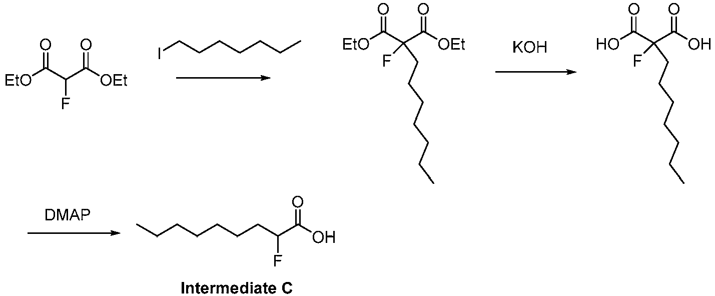

### Synthesis of diethyl 2-fluoro-2-heptylmalonate

To a suspension of 60% sodium hydride (2.3 mmol, 94 mg) in DMF (3 mL) was added diethyl 2-fluoromalonate (1.68 mmol, 300 mg) in DMF (1 mL) at 5 °C. The reaction mixture was stirred at 5 °C for 15 min. 1-iodoheptane (5.0 mmol, 0.82 mL) was added and the reaction mixture was stirred at room temperature for 80 min. The reaction mixture was partitioned between EtOAc and sat. NaHCO₃. The organic layer was separated, dried over Na₂SO₄ and concentrated. Purification via automated flash chromatography (0% to 25% EtOAc in hexanes) gave diethyl 2-fluoro-2-heptylmalonate (319 mg, 69%).

### Synthesis of 2-fluoro-2-heptylmalonic acid

A mixture of diethyl 2-fluoro-2-heptylmalonate (1.1 mmol, 300 mg) and potassium hydroxide (13.2 mmol, 730 mg) in MeOH:water:THF (1.0 mL:1.0 mL:0.1 mL) was heated at 80°C for 2 h. The reaction mixture was acidified with 1M HCl and extracted with EtOAc to give 2-fluoro-2-heptylmalonic acid (221 mg, 91%) which was used in the next step without further purification.

### Synthesis 2-fluorononanoic acid (Intermediate C)

A solution of 2-fluoro-2-heptylmalonic acid (0.9 mmol, 196 mg) and 4-(N,N-Dimethylamino)pyridine (catalytic) in DMF (1.0 mL) was heated at 180 °C for 10 min. The reaction mixture was acidified with 1M HCl and extracted with EtOAc to give 2-fluorononanoic acid (150 mg, 95%) which was used in the next step without further purification.

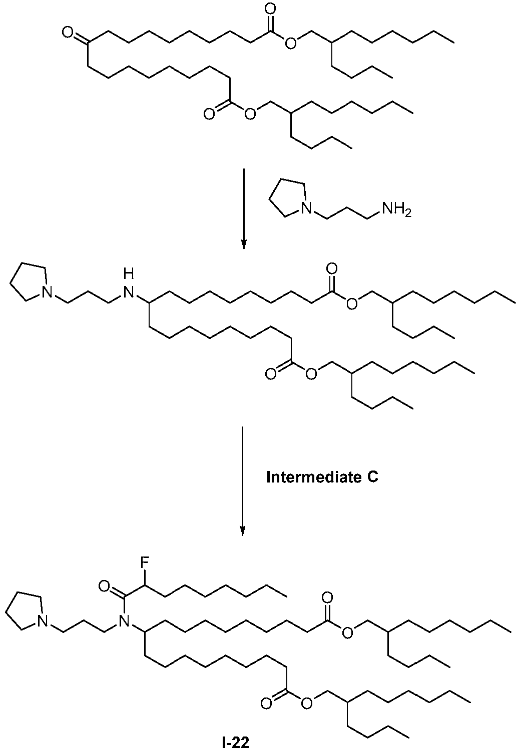

### Synthesis of bis(2-butyloctyl) 10-((3-(pyrrolidin-1-yl)propyl)amino)nonadecanedioate

A mixture of bis(2-butyloctyl) 10-oxononadecanedioate (was prepared according to the general procedures of Example 5, 1.5 mmol, 1.0 g) and 3-(pyrrolidin-1-yl)propan-1-amine (2.2 mmol, 280 mg) in dichloroethane (8 mL) was stirred at room temperature for 20 min. Acetic acid (2.1 mmol, 0.12 mL) and sodium triacetoxyborohydride (2.2 mmol, 470 mg) was then added and the reaction was stirred at room temperature for 19 h. An additional amount of sodium triacetoxyborohydride (0.44 mmol, 94 mg) was added and the reaction was stirred for another 24 h. The reaction mixture was concentrated and partitioned between EtOAc and sat NaHCO₃. The organic layer was separated, dried over Na₂SO₄ and concentrated. Purification via automated flash chromatography (10% EtOAc, then 5% to 15% MeOH in DCM with 1% Et₃N) gave bis(2-butyloctyl) 10-((3-(pyrrolidin-1-yl)propyl)amino)nonadecanedioate (866 mg, 75%).

### Synthesis of I-22

A mixture of bis(2-butyloctyl) 10-((3-(pyrrolidin-1-yl)propyl)amino)nonadecanedioate (0.13 mmol, 100 mg), **Intermediate** C (0.16 mmol, 27 mg), DIEA (0.47 mmol, 0.08 mL), HATU (0.20 mmol, 74 mg) in DCM (2 mL) was stirred at room temperature overnight. The reaction mixture was partitioned between EtOAc and sat. NaHCO₃. The organic layer was separated, dried over Na₂SO₄ and concentrated. Purification via automated flash chromatography (5% to 65% EtOAc in hexanes plus 1% Et₃N) gave **I-22** (100 mg, 81%). ¹H NMR (600 MHz, CDCl₃) δ 5.12 (dddd, *J =* 48.7, 39.8, 8.5, 4.2 Hz, 1H), 3.99 (d, *J =* 5.7 Hz, 4H), 3.70 (t, *J =* 7.2 Hz, 1H), 3.29 - 3.13 (m, 2H), 2.54 - 2.43 (m, 6H), 2.34 - 2.28 (m, 4H), 2.00 - 1.86 (m, 2H), 1.85 - 1.75 (m, 6H), 1.68 - 1.58 (m, 10H), 1.57 - 1.47 (m, 4H), 1.47 - 1.15 (m, 64H), 0.94 - 0.87 (m, 15H). ESI-MS: MW for C₅₉H₁₁₃FN₂O₅ [M+H]⁺ Calc. 949.9; Found 950.0.

### EXAMPLE 20

### BIS(2-BUTYLOCTYL) 10-(N-DECYL-5-(DIMETHYLAMINO)-2-FLUOROPENTANAMIDO)NONADECANEDIOATE (COMPOUND 1-24)

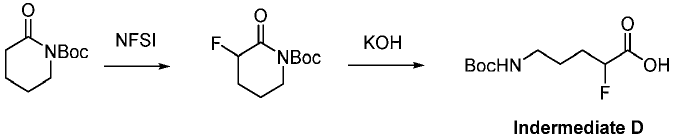

### Synthesis of tert-butyl 3-fluoro-2-oxopiperidine-1-carboxylate

To a solution of tert-butyl 2-oxopiperidine-1-carboxylate (5.0 mmol, 1.0g) in THF (8 mL) was added 1M LiHMDS in THF (5.3 mmol, 5.3 mL) at -78 °C. The reaction was stirred at -78 °C for 1 h. N-Fluorobenzenesulfonimide (5.3 mmol, 1.7 g) was then added and the reaction mixture was allowed to warm to -40 °C over 2 h. The reaction mixture was quenched with sat. NaHCO₃ and extracted with EtOAc. The organic layer was separated, dried over Na₂SO₄ and concentrated. Purification via automated flash chromatography (5% to 100% EtOAc in hexanes with 1% Et₃N) gave tert-butyl 3-fluoro-2-oxopiperidine-1-carboxylate (730 mg, 68%).

### Synthesis of 5-((tert-butoxycarbonyl)amino)-2-fluoropentanoic acid (Intermediate D)

A mixture of tert-butyl 3-fluoro-2-oxopiperidine-1-carboxylate (0.90 mmol, 200 mg) and potassium hydroxide (1.2 mmol, 67 mg) in MeOH:water (1.0 mL:1.0 mL) was stirred at room temperature overnight. The reaction mixture was acidified with 1M HCl and extracted with EtOAc. The organic layer was separated, dried over Na₂SO₄, and concentrated to give **Intermediate D** (166 mg, 79%) which was used in the next step without further purification.

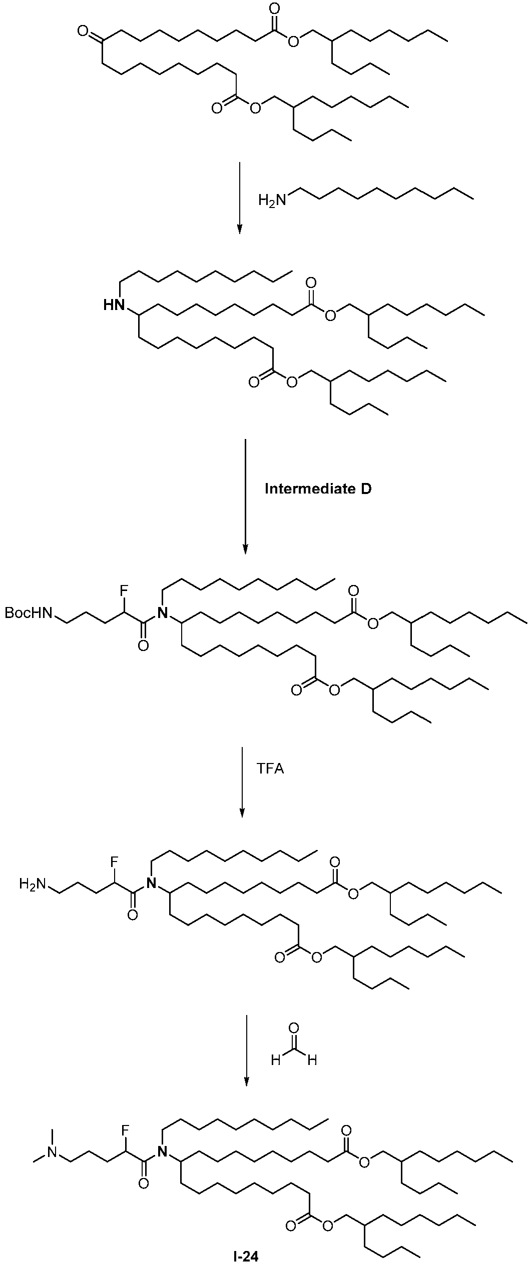

### Synthesis of bis(2-butyloctyl) 10-(decylamino)nonadecanedioate

A mixture of bis(2-butyloctyl) 10-oxononadecanedioate (was prepared according to the general procedures of Example 5, 1.5 mmol, 1.0 g) and decan-1-amine (2.2 mmol, 0.44 mL) in dichloroethane (8 mL) was stirred at room temperature for 20 min. Acetic acid (2.1 mmol, 0.12 mL) and sodium triacetoxyborohydride (2.2 mmol, 470 mg) was then added and the reaction was stirred at room temperature for 19 h. The reaction mixture was concentrated and partitioned between EtOAc and sat. NaHCO₃. The organic layer was separated, dried over Na₂SO₄ and concentrated. Purification via automated flash chromatography (2% to 10% MeOH in DCM) followed by a second purification via automated flash chromatography (5% to 100% EtOAc in hexanes with 1% Et₃N) gave bis(2-butyloctyl) 10-(decylamino)nonadecanedioate (735 mg, 61%).

### Synthesis of bis(2-butyloctyl) 10-(5-((tert-butoxycarbonyl)amino)-N-decyl-2-fluoropentanamido)nonadecanedioate

A mixture of bis(2-butyloctyl) 10-(decylamino)nonadecanedioate (0.24 mmol, 200 mg), **Intermediate D** (0.29 mmol, 68 mg), DIEA (0.86 mmol, 0.15 mL) and HATU (0.36 mmol, 136 mg) in DCM (2 mL) was stirred at room temperature for 30 min. The reaction mixture was partitioned between EtOAc and sat. NaHCO₃. The organic layer was separated, dried over Na₂SO₄ and concentrated. Purification via automated flash chromatography (5% to 100% EtOAc in hexanes) gave bis(2-butyloctyl) 10-(5-((tert-butoxycarbonyl)amino)-N-decyl-2-fluoropentanamido)nonadecanedioate (227 mg, 91%).

### Synthesis of bis(2-butyloctyl) 10-(5-amino-N-decyl-2-fluoropentanamido)nonadecanedioate

A mixture of bis(2-butyloctyl) 10-(5-((tert-butoxycarbonyl)amino)-N-decyl-2-fluoropentanamido)nonadecanedioate (0.33 mmol, 340 mg) and Trifluoroacetic acid (1.0 mL) in DCM (2 mL) was stirred at room temperature for 30 min. The reaction mixture was concentrated and partitioned between EtOAc and sat. NaHCO₃. The organic layer was separated, dried over Na₂SO₄ and concentrated to give bis(2-butyloctyl) 10-(5-amino-N-decyl-2-fluoropentanamido)nonadecanedioate (320 mg, quantitative) which was used in the next step without further purification.

### Synthesis of I-24

A mixture of bis(2-butyloctyl) 10-(5-amino-N-decyl-2-fluoropentanamido)nonadecanedioate (0.30 mmol, 280 mg) and formic acid (37 wt% in water, 0.7 mL) in MeOH (5 mL) was stirred at room temperature for 15 min. Sodium triacetoxyborohydride (1.3 mmol, 266 mg) was then added and the reaction mixture was stirred at room temperature overnight. The reaction mixture was concentrated and partitioned between EtOAc and sat. NaHCO₃. The organic layer was separated, dried over Na₂SO₄ and concentrated. Purification via automated flash chromatography (5% to 65% EtOAc in hexanes with 1% Et₃N) gave **I-24** (197 mg, 68%). ¹H NMR (400 MHz, CDCl₃) δ 5.23 - 4.97 (m, 1H), 3.96 (d, *J =* 5.7 Hz, 4H), 3.67 (t, *J =* 7.1 Hz, 1H), 3.22 - 2.92 (m, 2H), 2.35 - 2.24 (m, 6H), 2.23 - 2.18 (m, 6H), 2.02 - 1.73 (m, 2H), 1.72 - 1.39 (m, 18H), 1.37 - 1.10 (m, 67H), 0.93 - 0.84 (m, 15H). ESI-MS: MW for C₆₀H₁₁₇FN₂O₅ [M+H]⁺ Calc. 965.9; Found 966.0.

### EXAMPLE 21

### BIS(2-BUTYLOCTYL) 10-(2-FLUORO-N-(4-(PYRROLIDIN-1-YL)BUTYL)NONANAMIDO)NONADECANEDIOATE (COMPOUND 1-23)

Compound I-23 was prepared according to the general procedures of Example 19, to yield 82 mg of product, 69%. ¹H NMR (400 MHz, CDCl₃) δ 5.06 (ddt, *J =* 49.1, 8.7, 4.7 Hz, 1H), 3.96 (d, *J =* 5.7 Hz, 4H), 3.74 - 3.61 (m, 1H), 3.24 - 2.96 (m, 2H), 2.52 - 2.40 (m, 6H), 2.33 - 2.24 (m, 4H), 2.02 - 1.70 (m, 6H), 1.67 - 1.56 (m, 9H), 1.55 - 1.39 (m, 9H), 2.02 - 1.70 (m, 62H), 0.93 - 0.84 (m, 15H).ESI-MS: MW for C₆₀H₁₁₅FN₂O₅ [M+H]⁺ Calc. 962.9; Found 963.9.

## Claims

1. A compound having the following structure (I): 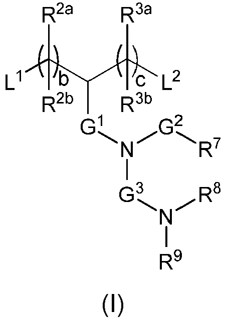 or a pharmaceutically acceptable salt, tautomer, or stereoisomer thereof, wherein:
L¹ is -O(C=O)R^{1a}, -(C=O)OR^{1a} , -C(=O)R^{1a} , -OR^{1a} , -S(O)ₓR^{1a} , -S-SR^{1a} , -C(=O)SR^{1a} , -SC(=O)R^{1a} , -NR^{a}C(=O)R^{1a} , -C(=O)NR^{a}R^{1a} , -NR^{a}C(=O)NR^{a}R^{1a} , -OC(=O)NR^{a}R^{1a}, -NR^{a}C(=O)OR^{1a} or R^{1b};
L² is -O(C=O)R^{4a} , -(C=O)OR^{4a} , -C(=O)R^{4a} , -OR^{4a} , -S(O)ₓR^{4a} , -S-SR^{4a} , -C(=O)SR^{4a} , -SC(=O)R^{4a} , -NR^{a}C(=O)R^{4a} , -C(=O)NR^{a}R^{4a} , -NR^{a}C(=O)NR^{a}R^{4a} , -OC(=O)NR^{a}R^{4a} , -NR^{a}C(=O)OR^{4a}, or R^{4b};
G¹ is C₁-C₂ alkylene, - (C=O)-, -O(C=O)-, -SC(=O)-, -NR^{a}C(=O)- or a direct bond;
G² is -C(=O)-, -(C=O)O-, -C(=O)S-, -C(=O)NR^{a}- or a direct bond;
G³ is C₁-C₆ alkylene;
R^{a} is H or C₁-C₁₂ alkyl;
R^{1a} and R^{4a} are each independently branched C₆-C₂₄ alkyl, branched C₆-C₂₄ alkenyl, branched C₆-C₂₄ fluoroalkyl, branched C₆-C₂₄ fluoroalkenyl, C₆-C₂₄ alkylacetal or C₆-C₂₄ fluoroalkylacetal;
R^{1b} and R^{4b} are each independently -CH(OR)(OR), wherein each R is independently linear or branched C₆-C₁₈ alkyl, linear or branched C₆-C₁₈ alkenyl, linear or branched C₆-C₁₈ fluoroalkyl, or linear or branched C₆-C₁₈ fluoroalkenyl
R^{2a} and R^{2b} are, at each occurrence, independently H, F, C₁-C₁₂ alkyl, or C₁-C₁₂ fluoroalkyl;
R^{3a} and R^{3b} are, at each occurrence, independently H, F, C₁-C₁₂ alkyl, or C₁-C₁₂ fluoroalkyl;
R⁷ is H, C₄-C₂₀ alkyl, or C₂-C₁₀ fluoroalkyl;
R⁸ and R⁹ are each independently C₁-C₁₂ alkyl; or R⁸ and R⁹, together with the nitrogen atom to which they are attached, form a 5, 6 or 7-membered heterocyclic ring;
b and c are each independently an integer from 1 to 24; and
wherein at least one of R^{2a}, R^{2b}, R^{3a}, and R^{3b} is F or C₁-C₁₂ fluoroalkyl; at least one of R^{1a} and R^{4a} is present and selected from branched C₆-C₂₄ fluoroalkyl, branched C₆-C₂₄ fluoroalkenyl and C₆-C₂₄ fluoroalkylacetal; at least one of R^{1b} and R^{4b} is present and selected from linear or branched C₆-C₁₈ fluoroalkyl and linear or branched C₆-C₁₈ fluoroalkenyl; G³ is C₁-C₆ fluoroalkylene; and/or R⁷ is C₂-C₁₀ fluoroalkyl.

2. The compound of claim 1, wherein at least one of R^{2a}, R^{2b}, R^{3a}, and R^{3b} is For C₁-C₁₂ fluoroalkyl; at least one of R^{1a} and R^{4a} is present and selected from branched C₆-C₂₄ fluoroalkyl, branched C₆-C₂₄ fluoroalkenyl and C₆-C₂₄ fluoroalkylacetal; at least one of R^{1b} and R^{4b} is present and selected from linear or branched C₆-C₁₈ fluoroalkyl and linear or branched C₆-C₁₈ fluoroalkenyl; and/or R⁷ is C₂-C₁₀ fluoroalkyl.

3. The compound of claim 1 or 2, wherein G¹ is independently - (C=O)- or a direct bond and G² is -(C=O)- or a direct bond.

4. The compound of any one of claims 1-3, having one of the following structures (IA) or (IB):

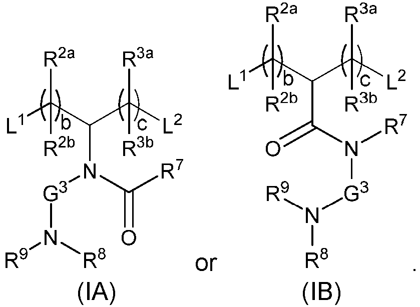

5. The compound of any one of claims 1-4, wherein:
a) L¹ is -O(C=O)R^{1a} or -(C=O)OR^{1a} and L² is -O(C=O)R^{4a} or - (C=O)OR^{4a}; and/or
b) at least one of R^{2a}, R^{2b}, R^{3a}, and R^{3b} is For C₁-C₁₂ fluoroalkyl; and/or
c) at least one of R^{1a} and R^{4a} is present and selected from branched C₆-C₂₄ fluoroalkyl, branched C₆-C₂₄ fluoroalkenyl and C₆-C₂₄ fluoroalkylacetal; and/or
d) at least one of R^{1b} and R^{4b} is present and selected from linear or branched C₆-C₁₈ fluoroalkyl and linear or branched C₆-C₁₈ fluoroalkenyl; and/or
e) at least one of R^{2a} and R^{3a} is H; and/or
f) at least one of R^{2b} and R^{3b} is H; and/or
g) R^{2a} and R^{3a} are each F; and/or
h) R^{2b} and R^{3b} are each F.

6. The compound of any one of claims 1-5, wherein:
a) at least one of R^{1a}, R^{1b}, R^{4a}, or R^{4b} is 1,1,1,2,2-pentafluoro-3-dodecane (-CH(CF₂CF₃)(CH₂)₈CH₃); or
b) R^{1a}, R^{4a} or both are each 1,1,1,2,2,pentafluoro-3-dodecane (-CH(CF₂CF₃)(CH₂)₈CH₃).
c) R^{1b}, R^{4b}, or both are each 1,1,1,2,2,pentafluoro-3-dodecane (-CH(CF₂CF₃)(CH₂)₈CH₃); or
d) at least one of R^{1a}, R^{1b}, R^{4a}, or R^{4b} is 1,1,1,2,2,3,3,4,4,5,5,6,6,12,12,13,13,14,14,15,15,16,16,17,17,17-hexacosafluoro-9-heptadecane (-CH((CH₂)₂(CF₂)₅CF₃)₂); or
e) R^{1a}, R^{4a}, or both are each 1,1,1,2,2,3,3,4,4,5,5,6,6,12,12,13,13,14,14,15,15,16,16,17,17,17-hexacosafluoro-9-heptadecane (-CH((CH₂)₂(CF₂)₅CF₃)₂); or
f) R^{1b}, R^{4b}, or both are each 1,1,1,2,2,3,3,4,4,5,5,6,6,12,12,13,13,14,14,15,15,16,16,17,17,17-hexacosafluoro-9-heptadecane (-CH((CH₂)₂(CF₂)₅CF₃)₂); or
g) at least one of R^{1a}, R^{1b}, R^{4a}, or R^{4b} has one of the following structures: 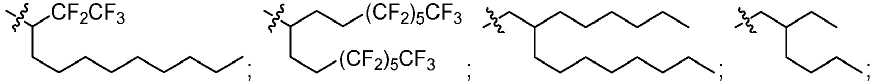 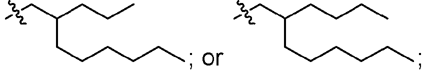 or
h) at least one of R^{1a} and R^{4a} is C₆-C₂₄ alkylacetal or C₆-C₂₄ fluoroalkylacetal.

7. The compound of any one of claims 1-6, wherein R⁷ is H, C₆-C₁₆ alkyl, or C₂-C₁₀ fluoroalkyl, preferably wherein R⁷ is H, C₆-C₉ alkyl, or C₂-C₇ fluoroalkyl, more preferably wherein the C₆-C₉ alkyl is n-heptyl (-(CH₂)₆CH₃), even more preferably wherein C₂-C₇ fluoroalkyl is 2,2,2-trifluoroethyl (-CH₂CF₃), 4,4,4-trifluoro n-butyl (-(CH₂)₃CF₃), perfluoro n-butyl (-(CF₂)₃CF₃), 7,7,7-trifluoro n-heptyl (-(CH₂)₆CF₃), or perfluoro n-heptyl (-(CF₂)₆CF₃).

8. The compound of any one of claims 1-6, wherein R⁷ is C₂-C₁₀ fluoroalkyl, perfluoroalkyl or C₂-C₇ perfluoroalkyl.

9. The compound of any one of claims 1-8, wherein:
a) at least one of R⁸ and R⁹ is methyl (-CH₃); or
b) each of R⁸ and R⁹ is methyl (-CH₃); or
c) R⁸ and R⁹, together with the nitrogen atom to which they are attached, form a 5, 6 or 7-membered heterocyclic ring, preferably wherein the heterocyclic ring is piperazinyl.

10. The compound of any one of claims 1-9, wherein G³ is C₂-C₅ alkylene, C₃ alkylene, C₁-C₆ fluoroalkylene or mono-fluorohexylene.

11. The compound of any one of claims 1-10, wherein:
a) b is 8 and c is 8; or
b) b is 5 and c is 5.

12. The compound of any one of claims 1-11, having at least two fluorine atoms, or at least three fluorine atoms, preferably wherein the compound has at least one perfluorinated substituent, more preferably wherein the compound is a perfluorinated compound.

13. A compound having one of the following structures: 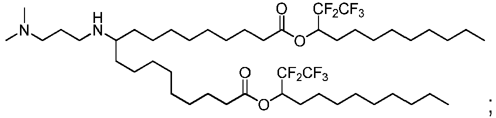 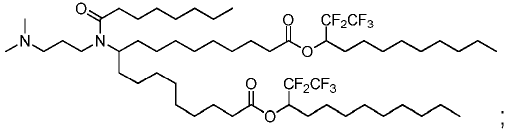 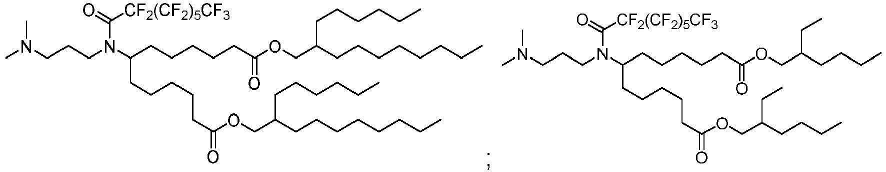 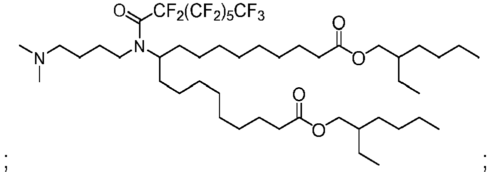 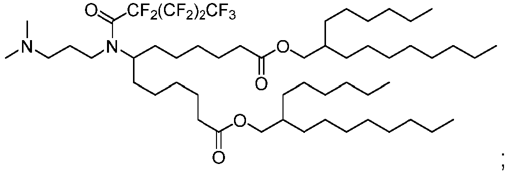 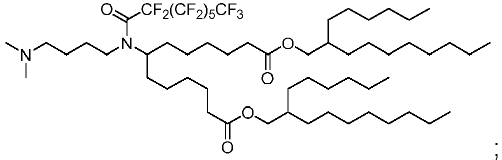 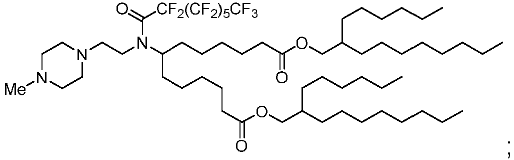 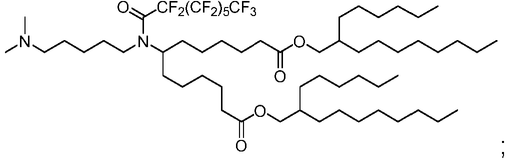 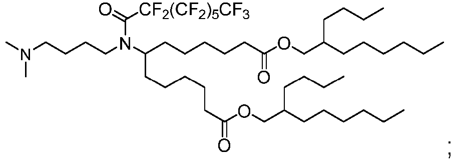 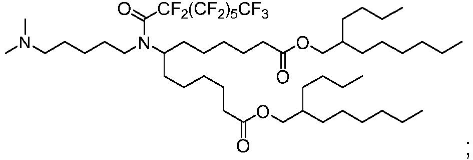 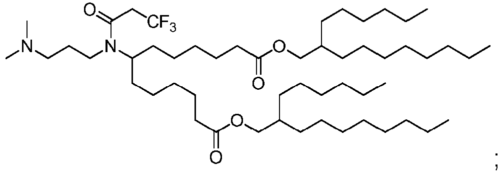 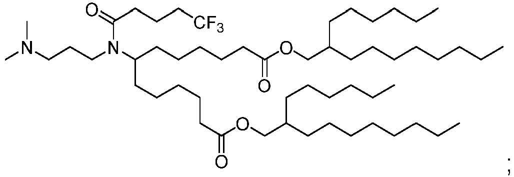 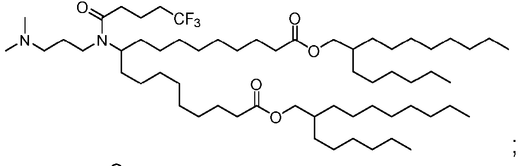 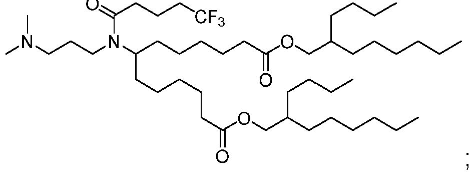 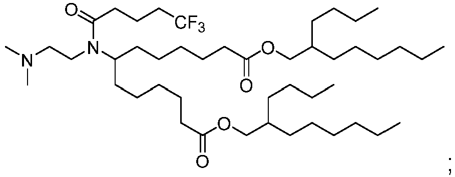 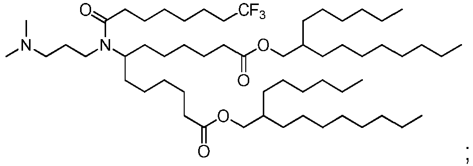 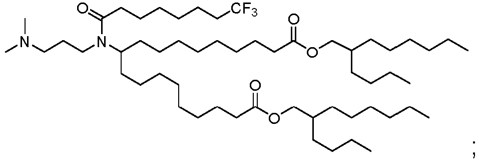 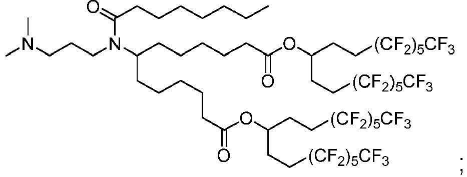 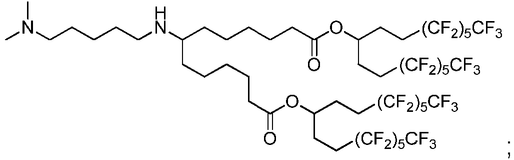 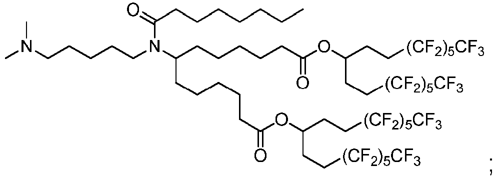 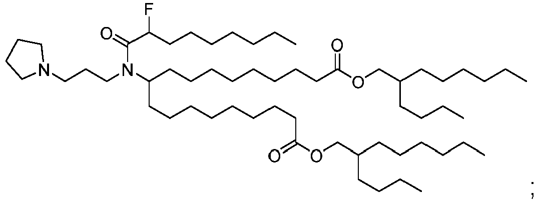 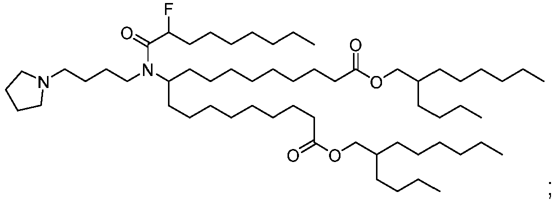 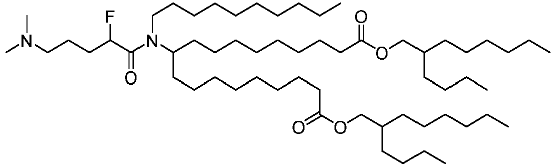 or

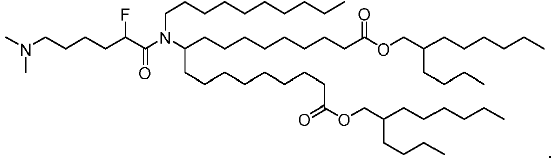

14. A lipid nanoparticle or a composition comprising the compound of any one of claims 1-13 and a therapeutic agent, preferably wherein the therapeutic agent comprises a nucleic acid, preferably wherein the nucleic acid is selected from antisense and messenger RNA.

15. Lipid nanoparticle or composition of claim 14 for use in a method of therapy.

## Patentansprüche

1. Verbindung mit der folgenden Struktur (I): 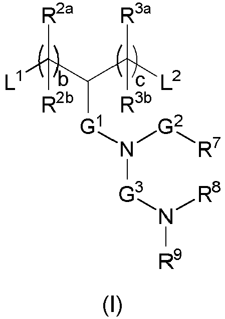 oder ein pharmazeutisch verträgliches Salz, Tautomer oder Stereoisomer davon, wobei:
L¹ -O(C=O)R^{1a}, -(C=O)OR^{1a}, -C(=O)R^{1a}, -OR^{1a}, -S(O)ₓR^{1a}, -S-SR^{1a}, -C(=O)SR^{1a}, -SC(=O)R^{1a}, -NR^{a}C(=O)R^{1a}, -C(=O)NR^{a}R^{1a}, -NR^{a}C(=O)NR^{a}R^{1a}, -OC(=O)NR^{a}R^{1a}, -NR^{a}C(=O)OR^{1a} oder R^{1b} ist;
L² -O(C=O)R^{4a}, -(C=O)OR^{4a}, -C(=O)R^{4a}, -OR^{4a}, -S(O)ₓR^{4a}, -S-SR^{4a}, -C(=O)SR^{4a}, -SC(=O)R^{4a}, -NR^{a}C(=O)R^{4a}, -C(=O)NR^{a}R^{4a}, -NR^{a}C(=O)NR^{a}R^{4a}, -OC(=O)NR^{a}R^{4a}, -NR^{a}C(=O)OR^{4a} oder R^{4b} ist;
G¹ C₁-C₂-Alkylen, -(C=O)-, -O(C=O)-, -SC(=O)-, -NR^{a}C(=O)- oder eine direkte Bindung ist;
G² -C(=O)-, -(C=O)O-, -C(=O)S-, -C(=O)NR^{a}- oder eine direkte Bindung ist;
G³ C₁-C₆-Alkylen ist;
R^{a} H oder C₁-C₁₂-Alkyl ist;
R^{1a} und R^{4a} jeweils unabhängig voneinander verzweigtes C₆-C₂₄-Alkyl, verzweigtes C₆-C₂₄-Alkenyl, verzweigtes C₆-C₂₄-Fluoralkyl, verzweigtes C₆-C₂₄-Fluoralkenyl, C₆-C₂₄-Alkylacetal oder C₆-C₂₄-Fluoralkylacetal sind;
R^{1b} und R^{4b} jeweils unabhängig voneinander -CH(OR)(OR) sind, wobei jedes R unabhängig voneinander lineares oder verzweigtes C₆-C₁₈-Alkyl, lineares oder verzweigtes C₆-C₁₈-Alkenyl, lineares oder verzweigtes C₆-C₁₈-Fluoralkyl oder lineares oder verzweigtes C₆-C₁₈-Fluoralkenyl ist;
R^{2a} und R^{2b} bei jedem Auftreten unabhängig voneinander H, F, C₁-C₁₂-Alkyl oder C₁-C₁₂-Fluoralkyl sind;
R^{3a} und R^{3b} bei jedem Auftreten unabhängig voneinander H, F, C₁-C₁₂-Alkyl oder C₁-C₁₂-Fluoralkyl sind;
R⁷ H, C₄-C₂₀-Alkyl oder C₂-C₁₀-Fluoralkyl ist;
R⁸ und R⁹ jeweils unabhängig voneinander C₁-C₁₂-Alkyl sind; oder R⁸ und R⁹ zusammen mit dem Stickstoffatom, an das sie gebunden sind, einen 5-, 6- oder 7-gliedrigen heterocyclischen Ring bilden;
b und c jeweils unabhängig voneinander eine ganze Zahl von 1 bis 24 sind; und
wobei mindestens einer der Reste R^{2a} , R^{2b}, R^{3a} und R^{3b} F oder C₁-C₁₂-Fluoralkyl ist; mindestens einer der Reste R^{1a} und R^{4a} vorhanden ist und aus verzweigtem C₆-C₂₄-Fluoralkyl, verzweigtem C₆-C₂₄-Fluoralkenyl und C₆-C₂₄-Fluoralkylacetal ausgewählt ist; mindestens einer der Reste R^{1b} und R^{4b} vorhanden ist und aus linearem oder verzweigtem C₆-C₁₈-Fluoralkyl und linearem oder verzweigtem C₆-C₁₈-Fluoralkenyl ausgewählt ist; G³ C₁-C₆-Fluoralkylen ist; und/oder R⁷ C₂-C₁₀-Fluoralkyl ist.

2. Die Verbindung nach Anspruch 1, wobei mindestens einer der Reste R^{2a} , R^{2b}, R^{3a} und R^{3b} F oder C₁-C₁₂-Fluoralkyl ist; mindestens einer der Reste R^{1a} und R^{4a} vorhanden ist und aus verzweigtem C₆-C₂₄-Fluoralkyl, verzweigtem C₆-C₂₄-Fluoralkenyl und C₆-C₂₄-Fluoralkylacetal ausgewählt ist; mindestens einer der Reste R^{1b} und R^{4b} vorhanden ist und aus linearem oder verzweigtem C₆-C₁₈-Fluoralkyl und linearem oder verzweigtem C₆-C₁₈-Fluoralkenyl ausgewählt ist; und/oder R⁷ C₂-C₁₀-Fluoralkyl ist.

3. Die Verbindung nach Anspruch 1 oder 2, wobei G¹ unabhängig -(C=O)- oder eine direkte Bindung ist und G² -(C=O)- oder eine direkte Bindung ist.

4. Die Verbindung nach einem der Ansprüche 1 bis 3, die eine der folgenden Strukturen (IA) oder (IB) aufweist:

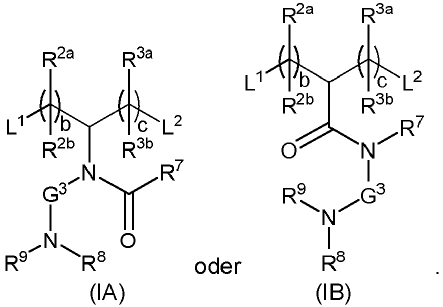

5. Die Verbindung nach einem der Ansprüche 1 bis 4, wobei:
a) L¹ -O(C=O)R^{1a} oder -(C=O)OR^{1a} ist und L² -O(C=O)R^{4a} oder -(C=O)OR^{4a} ist; und/oder
b) mindestens einer der Reste R^{2a}, R^{2b}, R^{3a} und R^{3b} F oder C₁-C₁₂-Fluoralkyl ist; und/oder
c) mindestens einer der Reste R^{1a} und R^{4a} vorhanden ist und aus verzweigtem C₆-C₂₄-Fluoralkyl, verzweigtem C₆-C₂₄-Fluoralkenyl und C₆-C₂₄-Fluoralkylacetal ausgewählt ist; und/oder
d) mindestens einer der Reste R^{1b} und R^{4b} vorhanden ist und aus linearem oder verzweigtem C₆-C₁₈-Fluoralkyl und linearem oder verzweigtem C₆-C₁₈-Fluoralkenyl ausgewählt ist; und/oder
e) mindestens einer der Reste R^{2a} und R^{3a} H ist; und/oder
f) mindestens einer der Reste R^{2b} und R^{3b} H ist; und/oder
g) R^{2a} und R^{3a} jeweils F sind; und/oder
h) R^{2b} und R^{3b} jeweils F sind.

6. Verbindung nach einem der Ansprüche 1 bis 5, wobei
a) mindestens einer der Reste R^{1a}, R^{1b}, R^{4a} oder R^{4b} 1,1,1,2,2-Pentafluor-3-dodecan (-CH(CF₂CF₃)(CH₂)₈CH₃) ist; oder
b) R^{1a}, R^{4a} oder beide jeweils 1,1,1,2,2-Pentafluor-3-dodecan (-CH(CF₂CF₃) (CH₂)_{8C}H₃) sind;
c) R^{1b}, R^{4b} oder beide jeweils 1,1,1,2,2-Pentafluor-3-dodecan (-CH(CF₂CF₃) (CH₂)₈CH₃) sind; oder
d) mindestens einer der Reste R^{1a}, R^{1b}, R^{4a} oder R^{4b} 1,1,1,2,2,3,3,4,4,5,5,6,6,12,12,13,13,14,14,15,15,16,16,17,17,17-Hexacosafluor-9-heptadecan (-CH((CH₂)₂(CF₂)₅CF₃)₂) ist; oder
e) R^{1a}, R^{4a} oder beide jeweils 1,1,1,2,2,3,3,4,4,5,5,6,6,12,12,13,13,14,14,15,15,16,16,17,17,17-Hexacosafluor-9-heptadecan (-CH((CH₂)₂(CF₂)₅CF₃)₂) sind; oder
f) R^{1b}, R^{4b} oder beide jeweils 1,1,1,2,2,3,3,4,4,5,5,6,6,12,12,13,13,14,14,15,15,16,16,17,17,17-Hexacosafluor-9-heptadecan (-CH((CH₂)₂(CF₂)₅CF₃)₂) sind; oder
g) mindestens einer der Reste R^{1a}, R^{1b}, R^{4a} oder R^{4b} eine der folgenden Strukturen aufweist: 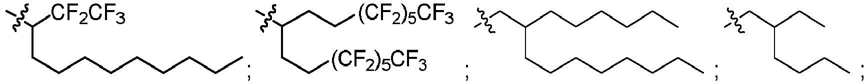 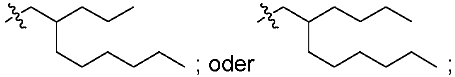 oder
h) mindestens einer der Reste R^{1a} und R^{4a} C₆-C₂₄-Alkylacetal oder C₆-C₂₄-Fluoralkylacetal ist.

7. Die Verbindung nach einem der Ansprüche 1 bis 6, wobei R⁷ H, C₆-C₁₆-Alkyl oder C₂-C₁₀-Fluoralkyl ist, vorzugsweise wobei R⁷ H, C₆-C₉-Alkyl oder C₂-C₇-Fluoralkyl ist, noch bevorzugter, wobei das C₆-C₉-Alkyl n-Heptyl (-(CH₂)₆CH₃) ist, noch bevorzugter, wobei C₂-C₇-Fluoralkyl 2,2,2-Trifluorethyl (-CH₂CF₃), 4,4,4-Trifluor-n-butyl (-(CH₂)₃CF₃), Perfluor-n-butyl (-(CF₂)₃CF₃), 7,7,7-Trifluor-n-heptyl (-(CH₂)₆CF₃) oder Perfluor-n-heptyl (-(CF₂)₆CF₃) ist.

8. Die Verbindung nach einem der Ansprüche 1 bis 6, wobei R⁷ C₂-C₁₀-Fluoralkyl, Perfluoralkyl oder C₂-C₇-Perfluoralkyl ist.

9. Die Verbindung nach einem der Ansprüche 1 bis 8, wobei:
a) mindestens einer der Reste R⁸ und R⁹ Methyl (-CH₃) ist; oder
b) jedes von R⁸ und R⁹ Methyl (-CH₃) ist; oder
c) R⁸ und R⁹ zusammen mit dem Stickstoffatom, an das sie gebunden sind, einen 5-, 6- oder 7-gliedrigen heterocyclischen Ring bilden, wobei der heterocyclische Ring vorzugsweise Piperazinyl ist.

10. Die Verbindung nach einem der Ansprüche 1 bis 9, wobei G³ C₂-C₅-Alkylen, C₃-Alkylen, C₁-C₆-Fluoralkylen oder Mono-fluorhexylen ist.

11. Die Verbindung nach einem der Ansprüche 1 bis 10, wobei:
a) b 8 ist und c 8 ist; oder
b) b 5 ist und c 5 ist.

12. Die Verbindung nach einem der Ansprüche 1 bis 11, die mindestens zwei Fluoratome oder mindestens drei Fluoratome aufweist, wobei die Verbindung vorzugsweise mindestens einen perfluorierten Substituenten aufweist, noch bevorzugter, wenn die Verbindung eine perfluorierte Verbindung ist.

13. Verbindung mit einer der folgenden Strukturen: 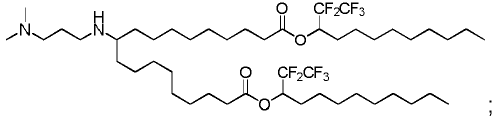 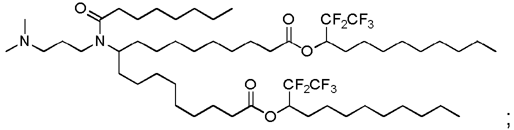 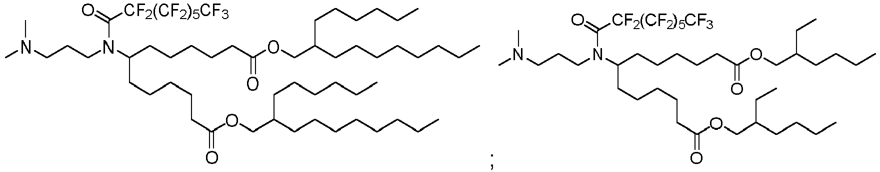 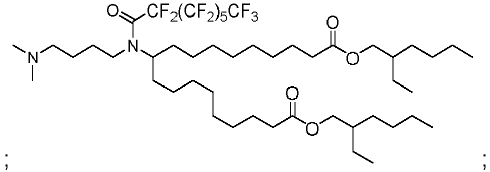 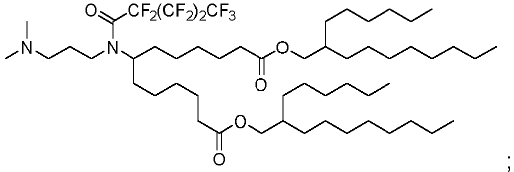 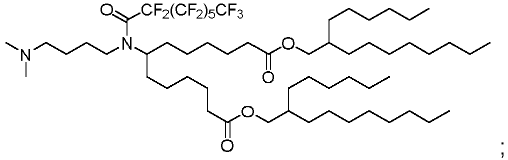 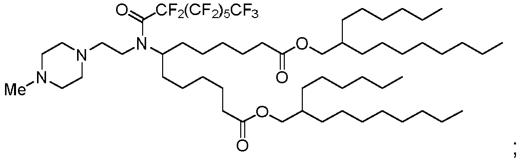 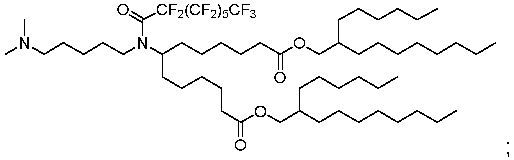 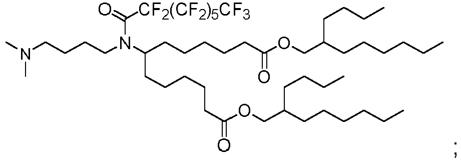 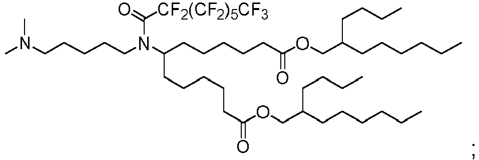 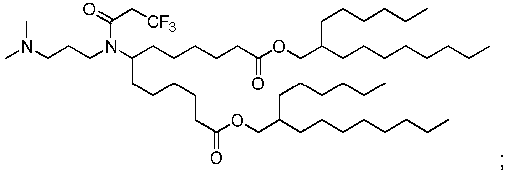 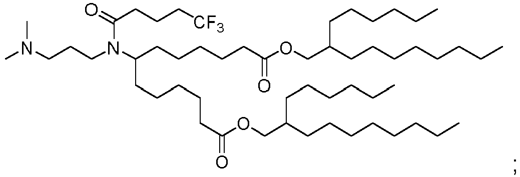 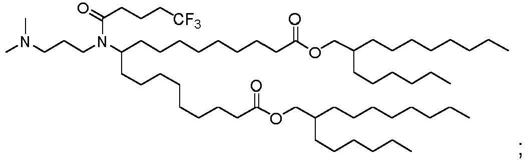 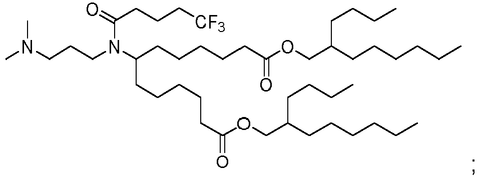 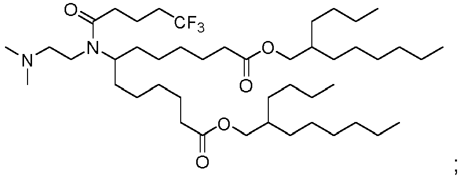 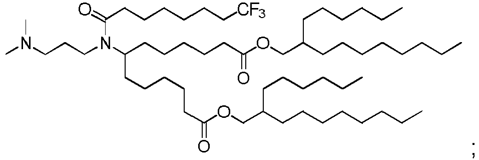 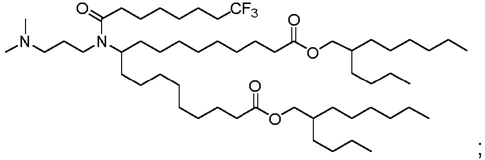 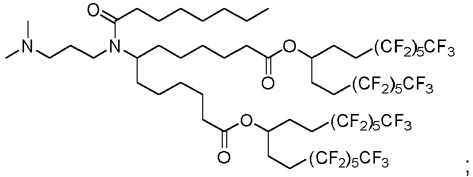 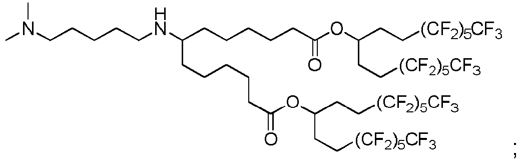 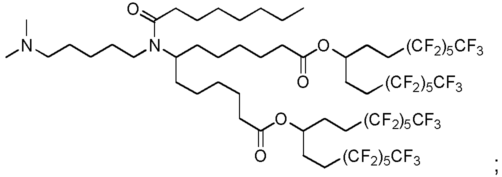 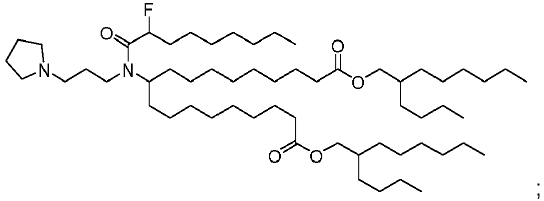 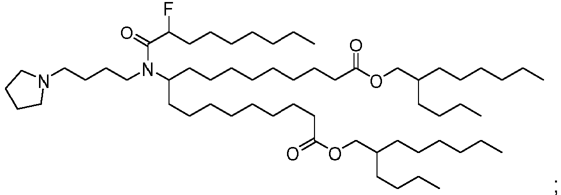 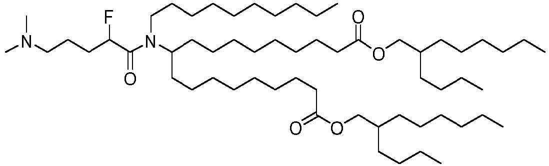 oder

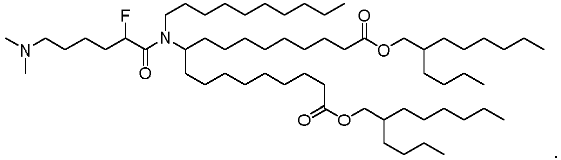

14. Lipidnanopartikel oder Zusammensetzung, umfassend die Verbindung nach einem der Ansprüche 1 bis 13 und einen therapeutischen Wirkstoff, vorzugsweise wobei der therapeutische Wirkstoff eine Nukleinsäure umfasst, vorzugsweise wobei die Nukleinsäure aus Antisense- und Messenger-RNA ausgewählt ist.

15. Lipidnanopartikel oder Zusammensetzung nach Anspruch 14 zur Verwendung in einem Therapieverfahren.

## Revendications

1. Composé de structure suivante (I) : 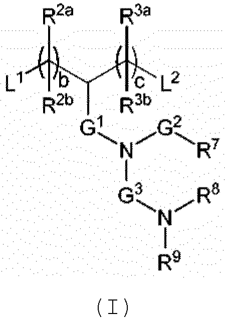 ou son sel, tautomère, ou stéréoisomère pharmaceutiquement acceptable,
dans lequel :
L¹ est -O(C=O)R^{1a}, -(C=O)O^{R1a}, -C(=O)R^{1a}, -OR^{1a}, - S(O)ₓR^{1a}, -S-SR^{1a}, -C(=O)SR^{1a}, -SC(=O)R^{1a}, -NR^{a}C(=O)R^{1a}, - C(=O)NR^{a}R^{1a}, -NR^{a}C(=O) NR^{a}R^{1a}, -OC (=O) NR^{a}R^{1a}, -NR^{a}C(=O)OR^{1a} ou R^{1b} ;
L² est -O(C=O)R^{4a}, -(C=O)OR^{4a}, -C(=O)R^{4a}, -OR^{4a}, - S(O)ₓR^{4a}, -S-SR^{4a}, -C(=O)SR^{4a}, -SC(=O)R^{4a}, -NR^{a}C(=O)R^{4a}, - C (=O) NR^{a}R^{4a}, -NR^{a}C(=O) NR^{a}R^{4a}, -OC(=O) NR^{a}R^{4a}, -NR^{a}C(=O)OR^{4a}, ou R^{4b} ;
G¹ est un groupe alcylène en C₁ à C₂, -(C=O)-, - O(C=O) -, -SC(=O)-, -NR^{a}C(=O) - ou une liaison directe ;
G² est -C(=O)-, -(C=O)O-, -C(=O)S-, -C(=O)NR^{a}- ou une liaison directe ;
G³ est un groupe alcylène en C₁ à C₆ ;
R^{a} est un groupe H ou alkyle en C₁ à C₁₂ ;
R^{1a} et R^{4a} sont chacun indépendamment un groupe alkyle en C₆ à C₂₄ ramifié, alcényle en C₆ à C₂₄ ramifié, fluoroalkyle en C₆ à C₂₄ ramifié, fluoroalcényle en C₆ à C₂₄, alkylacétal en C₆ à C₂₄ ou fluoroalkylacétal en C₆ à C₂₄ ;
R^{1b} et R^{4b} sont chacun indépendamment un groupe - CH(OR)(OR), dans lequel chaque R est indépendamment un groupe alkyle en C₆ à C₁₈ linéaire ou ramifié, alcényle en C₆ à C₁₈ linéaire ou ramifié, fluoroalkyle en C₆ à C₁₈ linéaire ou ramifié, ou fluoroalcényle en C₆ à C₁₈ linéaire ou ramifié
R^{2a} et R²¹ sont, à chaque apparition, indépendamment un groupe H, F, alkyle en C₁ à C₁₂, ou fluoroalkyle en C₁ à C₁₂ ;
R^{3a} et R^{3b} sont, à chaque apparition, indépendamment un groupe H, F, alkyle en C₁ à C₁₂, ou fluoroalkyle en C₁ à C₁₂ ;
R⁷ est un groupe H, alkyle en C₄ à C₂₀, ou fluoroalkyle en C₂ à C₁₀ ;
R⁸ et R⁹ sont chacun indépendamment un groupe alkyle en C₁ à C₁₂ ; ou R⁸ et R⁹, conjointement à l'atome d'azote auquel ils sont fixés, forment un cycle hétérocyclique à 5, 6 ou 7 chaînons ;
b et c sont chacun indépendamment un nombre entier d'une valeur de 1 à 24 ; et
dans lequel au moins l'un de R^{2a}, R^{2b}, R^{3a}, et R^{3b} est un groupe F ou fluoroalkyle en C₁ à C₁₂ ; au moins l'un de R^{1a} et R^{4a} est présent et sélectionné parmi un groupe fluoroalkyle en C₆ à C₂₄ ramifié, fluoroalcényle en C₆ à C₂₄ ramifié et fluoroalkylacétal en C₆ à C₂₄ ; au moins l'un de R^{1b} et R^{4b} est présent et sélectionné parmi un groupe fluoroalkyle en C₆ à C₁₈ linéaire ou ramifié et fluoroalcényle en C₆ à C₁₈ linéaire ou ramifié ; G³ est un groupe fluoroalcylène en C₁ à C₆ ; et/ou R⁷ est un groupe fluoroalkyle en C₂ à C₁₀.

2. Composé selon la revendication 1, dans lequel au moins l'un de R^{2a}, R^{2b}, R^{3a}, et R^{3b} est un groupe F ou fluoroalkyle en C₁ à C₁₂ ; au moins l'un de R^{1a} et R^{4a} est présent et sélectionné parmi un groupe fluoroalkyle en C₆ à C₂₄ ramifié, fluoroalcényle en C₆ à C₂₄ ramifié et fluoroalkylacétal en C₆ à C₂₄ ; au moins l'un de R^{1b} et R^{4b} est présent et sélectionné parmi un groupe fluoroalkyle en C₆ à C₁₈ linéaire ou ramifié et fluoroalcényle en C₆ à C₁₈ linéaire ou ramifié ; et/ou R⁷ est un groupe fluoroalkyle en C₂ à C₁₀.

3. Composé selon la revendication 1 ou 2, dans lequel G¹ est indépendamment -(C=O)- ou une liaison directe et G² est -(C=O)- ou une liaison directe.

4. Composé selon l'une quelconque des revendications 1 à 3, ayant l'une des structures suivantes (IA) ou (IB) :

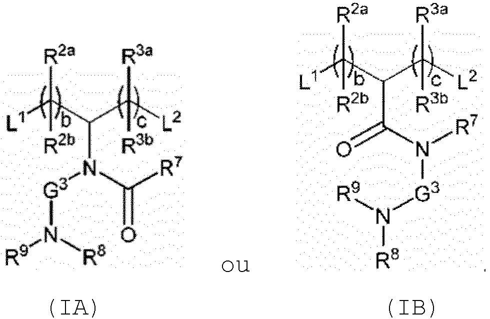

5. Composé selon l'une quelconque des revendications 1 à 4, dans lequel :
a) L¹ est -O(C=O)R^{1a} ou -(C=O)OR^{1a} et L² est -O(C=O)R^{4a} ou -(C=O)OR^{4a} ; et/ou
b) au moins l'un de R^{2a}, R^{2b}, R^{3a}, et R^{3b} est un groupe F ou fluoroalkyle en C₁ à C₁₂ ; et/ou
c) au moins l'un de R^{1a} et R^{4a} est présent et sélectionné parmi un groupe fluoroalkyle en C₆ à C₂₄ ramifié, fluoroalcényle en C₆ à C₂₄ ramifié et fluoroalkylacétal en C₆ à C₂₄ ; et/ou
d) au moins l'un de R^{1b} et R^{4b} est présent et sélectionné parmi un groupe fluoroalkyle en C₆ à C₁₈ linéaire ou ramifié et fluoroalcényle en C₆ à C₁₈ linéaire ou ramifié ; et/ou
e) au moins l'un de R^{2a} et R^{3a} est un groupe H ; et/ou
f) au moins l'un de R^{2b} et R^{3b} est un groupe H ; et/ou
g) R^{2a} et R^{3a} sont chacun un groupe F ; et/ou
h) R^{2b} et R^{3b} sont chacun un groupe F.

6. Composé selon l'une quelconque des revendications 1 à 5, dans lequel :
a) au moins l'un de R^{1a}, R^{1b}, R^{4a}, ou R^{4b} est le 1,1,1,2,2-pentafluoro-3-dodécane (-CH(CF₂CF₃)(CH₂)₈CH₃); ou
b) R^{1a}, R^{4a}, ou les deux sont chacun un groupe 1,1,1,2,2-pentafluoro-3-dodécane (-CH(CF₂CF₃)(CH₂)₈CH₃);
c) R^{1b}, R^{4b}, ou les deux sont chacun un groupe 1,1,1,2,2-pentafluoro-3-dodécane (-CH(CF₂CF₃)(CH₂)₈CH₃); ou
d) au moins l'un de R^{1a}, R^{1b}, R^{4a}, ou R^{4b} est un groupe 1,1,1,2,2,3,3,4,4,5,5,6,6,12,12,13,13,14,14,15,15,16,16, 17,17,17-hexacosafluoro-9-heptadécane (-CH ( (CH₂) ₂ (CF₂) ₅CF₃) ₂) ; ou
e) R^{1a}, R^{4a}, ou les deux sont chacun un groupe 1,1,1,2,2,3,3,4,4,5,5,6,6,12,12,13,13,14,14,15,15,16,16, 17,17,17-hexacosafluoro-9-heptadécane (-CH ( (CH₂) ₂ (CF₂) ₅CF₃) ₂) ; ou
f) R^{1b}, R^{4b}, ou les deux sont chacun un groupe 1,1,1,2,2,3,3,4,4,5,5,6,6,12,12,13,13,14,14,15,15,16,16, 17,17,17-hexacosafluoro-9-heptadécane (-CH ( (CH₂) ₂ (CF₂) ₅CF₃) ₂) ; ou
g) au moins l'un de R^{1a}, R^{1b}, R^{4a}, ou R^{4b} présente l'une des structures suivantes : 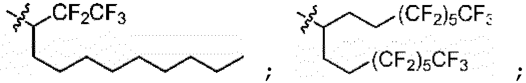 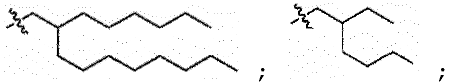 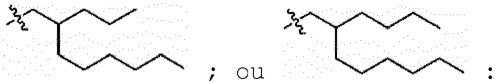 ou
h) au moins l'un de R^{1a} et R^{4a} est un groupe alkylacétal en C₆ à C₂₄ ou fluoroalkylacétal en C₆ à C₂₄.

7. Composé selon l'une quelconque des revendications 1 à 6, dans lequel R⁷ est un groupe H, alkyle en C₆ à C₁₆, ou fluoroalkyle en C₂ à C₁₀, préférablement dans lequel R⁷ est un groupe H, alkyle en C₆ à C₉, ou fluoroalkyle en C₂ à C₇, plus préférablement dans lequel le groupe alkyle en C₆ à C₉ est un groupe n-heptyl (-(CH₂)₆CH₃), même plus préférablement dans lequel le groupe fluoroalkyle en C₂ à C₇ est un groupe 2,2,2-trifluoroéthyl (-CH₂CF₃), 4,4,4-trifluoro n-butyl (-(CH₂) ₃CF₃) , perfluoro n-butyl (- (CF₂) ₃CF₃) , 7,7, 7-trifluoro n-heptyl (- (CH₂) ₆CF₃) , ou perfluoro n-heptyl (- (CF₂) ₆CF₃) .

8. Composé selon l'une quelconque des revendications 1 à 6, dans lequel R⁷ est un groupe fluoroalkyle en C₂ à C₁₀, perfluoroalkyle ou perfluoroalkyle en C₂ à C₇.

9. Composé selon l'une quelconque des revendications 1 à 8, dans lequel :
a) au moins l'un de R⁸ et R⁹ est un groupe méthyl (-CH₃) ; ou
b) chacun de R⁸ et R⁹ est un groupe méthyl (-CH₃) ; ou
c) R⁸ et R⁹, conjointement à l'atome d'azote auquel ils sont fixés, forment un cycle hétérocyclique à 5, 6 ou 7 chaînons, préférablement dans lequel le cycle hétérocyclique est le pipérazinyle.

10. Composé selon l'une quelconque des revendications 1 à 9, dans lequel G³ est un groupe alcylène en C₂ à C₅, alcylène en C₃, fluoroalcylène en C₁ à C₆ ou mono-fluorohexylène.

11. Composé selon l'une quelconque des revendications 1 à 10, dans lequel :
a) b est 8 et c est 8 ; ou
b) b est 5 et c est 5.

12. Composé selon l'une quelconque des revendications 1 à 11, ayant au moins deux atomes de fluor, ou au moins trois atomes de fluor, préférablement dans lequel le composé présente au moins un substituant perfluoré, plus préférablement dans lequel le composé est un composé perfluoré.

13. Composé ayant l'une des structures suivantes : 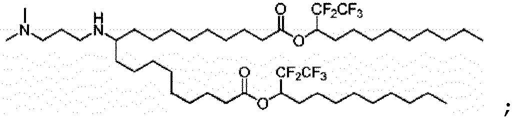 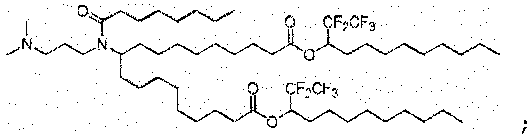 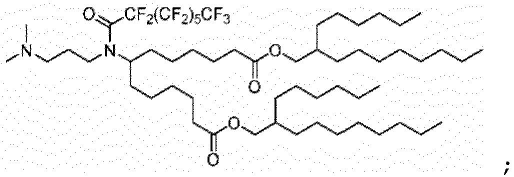 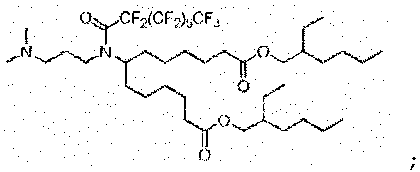 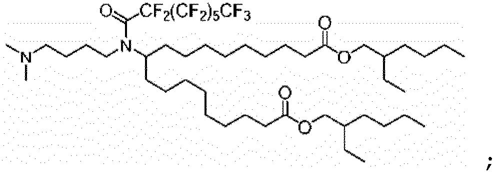 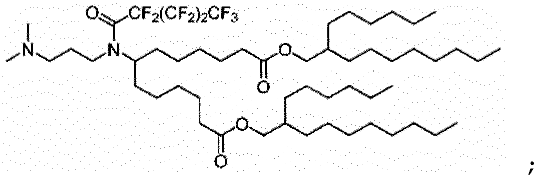 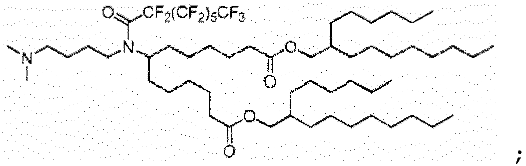 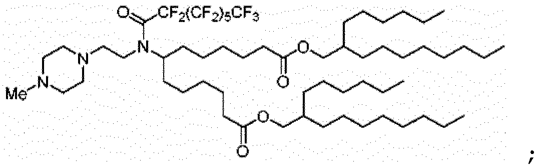 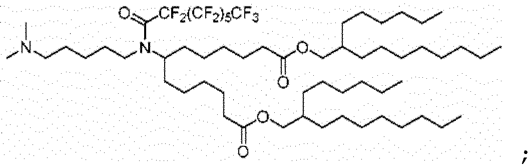 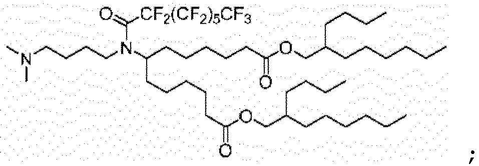 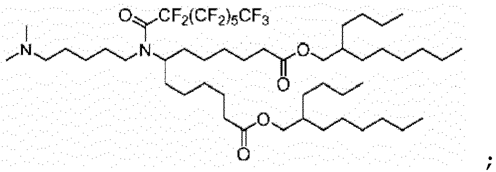 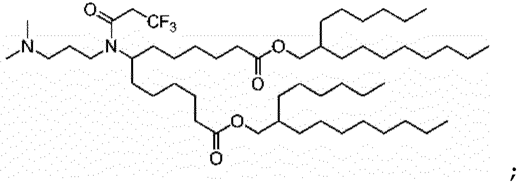 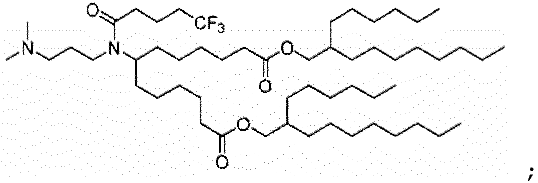 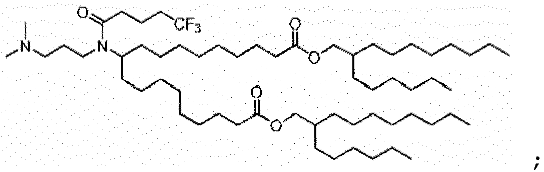 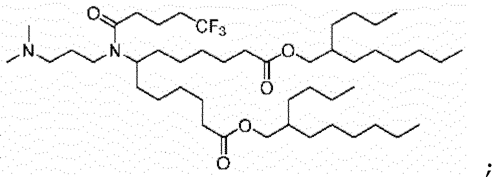 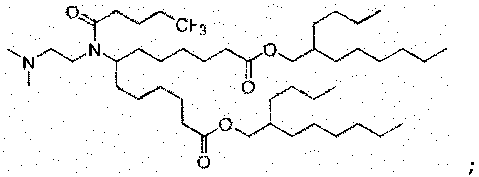 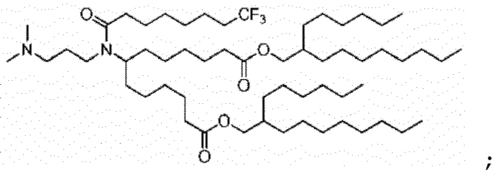 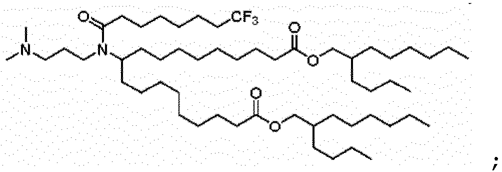 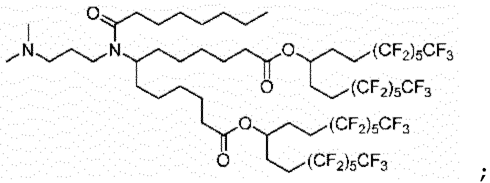 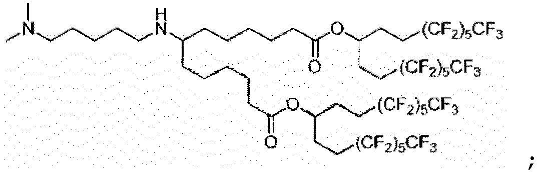 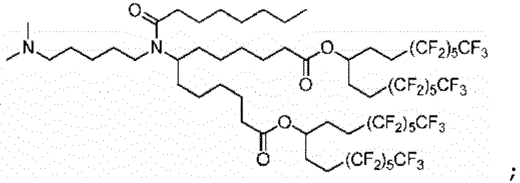 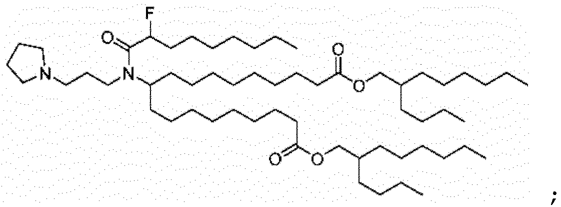 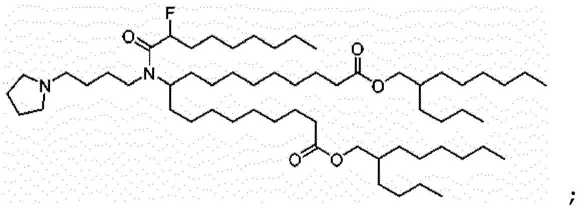 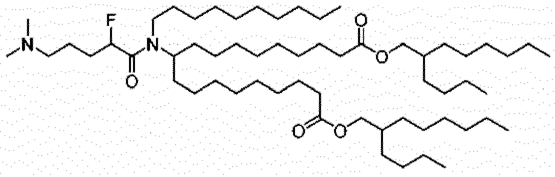 ou

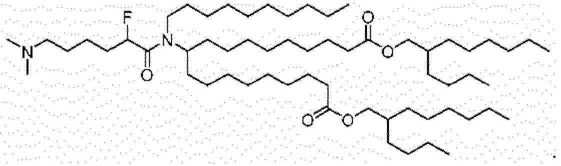

14. Nanoparticule ou composition lipidique comprenant le composé selon l'une quelconque des revendications 1 à 13 et un agent thérapeutique, préférablement dans laquelle l'agent thérapeutique comprend un acide nucléique, préférablement dans laquelle l'acide nucléique est sélectionné parmi un ARN antisens et messager.

15. Nanoparticule ou composition lipidique selon la revendication 14, pour l'utilisation dans un procédé de thérapie.
